# EUROPEAN PATENT APPLICATION

(11) **EP 3 517 115 A2**
(43) Date of publication of application: **31.07.2019**
(21) Application number: 19158655.1
(22) Date of filing: 30.11.2012
(51) Int. Cl.: A61K 31/555, A61N 5/06, A61B 5/00

(54) **METHODS FOR TREATING HYPERBILIRUBINEMIA WITH STANNSOPORFIN**

(30) Priority: 01.12.2011 US 201161565842 P
(62) Divisional of application: 12854066.3
(71) Applicant: InfaCare Pharmaceutical Corporation, Trevose, PA 19053 (US)
(72) Inventor: TULLOCH, Simon, J., Trevose, PA 19053 (US); WASIEWSKI, Warren, W., Lancaster, PS 17601-5456 (US)
(74) Representative: Harrison IP Limited

(57) **Abstract**

Some embodiments relate to methods of treating hyperbilirubinemia comprising administrating a therapeutic amount of a metalloporphyrin to an infant. Administration may occur when the infant's measured total serum bilirubin levels are at or below about the level suggested by the AAP nomogram for initiating phototherapy, when the infant's measured total serum bilirubin levels are at about the level suggested for initiating phototherapy in an infant, or when the infant's measured total serum bilirubin levels are at about the level suggested for initiating phototherapy. Administration may occur without regard to the total serum bilirubin level of the infant. In some embodiments, administration of the metalloporphyrin does not cause QT prolongation.

## Description

### B. CROSS REFERENCE

This application claims the benefit of U.S. Provisional Application Serial No. 61/565,842 entitled "Methods For Treating Hyperbilirubinemia With Stannsoporfm" filed December 1, 20 11 which is incorporated herein by reference in its entirety.

### C. BACKGROUND

Raised bilirubin levels may lead to potentially dangerous conditions, particularly in infants. In some cases, elevated bilirubin levels result from conditions that cause an increase in bilirubin production and in others with conditions affecting removal of bilirubin. In some instances, it is a combination. Increased bilirubin levels may lead to hyperbilirubinemia which can be dangerous to a patient. Accordingly, more and different treatments for reducing bilirubin production, increasing bilirubin excretion, or both, are desirable as are other methods of treating hyperbilirubinemia or increased bilirubin production.

### D. SUMMARY

The present disclosure relates to methods of treating hyperbilirubinemia with a metalloporphyrin. More particularly, embodiments disclosed include methods of treating hyperbilirubinemia or the symptoms thereof in an infant.

Some embodiments are directed to methods of treating hyperbilirubinemia or the symptoms thereof in an infant, the method comprising: administering a therapeutic amount of a metalloporphyrin to the infant with hyperbilirubinemia where no exclusion factor is present and at least one of a baseline total bilirubin level is elevated above a predetermined threshold and at least one risk factor is present; wherein the hyperbilirubinemia or symptoms thereof is treated.

Some embodiments further comprise detennining baseline total bilirubin levels in the infant. In some embodiments, baseline total bilirubin levels comprises total serum bilirubin levels, total cutaneous bilirubin or a combination thereof.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, determining baseline total bilirubin levels is performed at a time selected from within 6 hours of birth, 12 hours of birth, within 24 hours of birth, and within 48 hours of birth.

Some embodiments further comprise identifying the presence of at least one risk factor. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, and G6PD deficiency and combinations thereof.

Some embodiments further comprise identifying the absence of at least one exclusion factor. In some embodiments, the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate > 60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is ≥5.0 mg/dL), persistent hypoglycemia (blood glucose <40 mg/dL) despite standard-of-care treatment, liver diseases defined as ALT and/or AST greater than 2 times the upper limit of normal (ULN), abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, the predetermined threshold is the level determined by the AAP nomogram for initiating phototherapy for an infant of known age and known risk level. In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, is about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines and about 1 to about 3 mg/dL below the threshold for administration of phototherapy according to AAP nomogram corresponding to the infants age.

In some embodiments, administering a therapeutic amount of a metalloporphyrin is performed at a time selected from within about 6 hours of birth, within about 12 hours of birth, within about 24 hours of birth, and within about 48 hours of birth.

In some embodiments, the metalloporphyrin is selected from tin mesoporphyrin, zinc mesoporphyrin, chromium mesoporphyrin, tin protoporphyrin, zinc protoporphyrin, chromium protoporphyrin, bisglycol protoporphyrin and ferroporphyrin.

In some embodiments, the metalloporphyrin is tin mesoporphyrin. In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, 1.5 mg/kg, 3.0 mg/kg and 4.5 mg/kg of the infant's weight. In some embodiments, the therapeutic amount of the tin mesoporphyrin is from about 0.75 mg/kg to about 5 mg/kg of the infant's weight. In some embodiments, the therapeutic amount of the tin mesoporphyrin is selected from 0.75 mg/kg, 1.5 mg/kg, 3.0 mg/kg and 4.5 mg/kg of the infant's weight.

In some embodiments, the metalloporphyrin is administered by intramuscular injection.

In some embodiments, administering a therapeutic amount of a metalloporphyrin is performed when the infant's age is less than 20 days of age. In some embodiments, administering a therapeutic amount of a metalloporphyrin is performed when the infant's age is less than 30 days of age.

Some embodiments further comprise administering phototherapy where total bilirubin levels following administration of the metalloporphyrin are above the baseline total bilirubin levels.

Some embodiments further comprise determining post treatment total bilirubin levels following administration of the metalloporphyrin. In some embodiments, determining post treatment total bilirubin levels following administration of the metalloporphyrin is performed from about 6 and to about 72 hours after administering the metallopoφ hyrin to the infant. In some embodiments, post treatment total bilirubin levels are at least 5% below the baseline total bilirubin levels 24 hours after administering a therapeutic amount of a metalloporphyrin to the infant. In some embodiments, post treatment total bilirubin levels are at least 10% below the baseline total bilirubin levels 48 hours after administering a therapeutic amount of a metalloporphyrin to the infant. In some embodiments, post treatment total bilirubin levels are at least 20% below the baseline total bilirubin levels 72 hours after administering a therapeutic amount of a metalloporphyrin to the infant. In some embodiments, post treatment total bilirubin levels are less than 3 mg/dL above the baseline total bilirubin levels 48 hours after administering a therapeutic amount of a metalloporphyrin to the infant.

Some embodiments further comprise conducting on the infant an exam selected from a physical exam, a dermatologic exam, an audiology exam, an ophthalmological exam, a neurological exam, a laboratory test, an electrocardiogram and a combination thereof.

Some embodiments are directed to methods of reducing the likelihood of hyperbilirubinemia and the symptoms thereof in an infant, the method comprising: administering a therapeutic amount of a metalloporphyrin to the infant where the infant's total bilirubin is determined to be increasing in at least one total bilirubin measurement compared with a baseline total bilirubin level wherein the likelihood of hyperbilirubinemia or the symptoms thereof is decreased.

In some embodiments, where the infant's total bilirubin is determined to be increasing in two consecutive total bilirubin measurements.

In some embodiments, the baseline total bilirubin measurement is performed from about 6 to about 96 hours of age. In some embodiments, the baseline total bilirubin measurement is performed at about 6, 12, 24, 48, 72, or 96 hours of age. In some embodiments, the at least one total bilirubin measurement is performed from about 6 to about 72 hours after the baseline total bilirubin measurement.

In some embodiments, administering a therapeutic amount of a metalloporphyrin is performed within about 1 to about 6 hours of when the infant's total bilirubin is determined to be increasing in at least one total bilirubin measurement.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, **1.5** mg/kg, 3.0 mg/kg and 4.5 mg/kg of the infant's weight. In some embodiments, the therapeutic amount of the stannsoporfin is from about 0.75 mg/kg to about 5 mg/kg of the infant's weight. In some embodiments, the therapeutic amount of the stannsoporfin is selected from 0.75 mg/kg, **1.5** mg/kg, 3.0 mg/kg and 4.5 mg/kg of the infant's weight.

Some embodiments are directed to methods of treating hyperbilirubinemia and the symptoms thereof in an infant, the method comprising: administering a therapeutic amount of a metalloporphyrin to the infant; and administering a therapeutic amount of phototherapy to the infant wherein the hyperbilirubinemia or symptoms thereof is treated.

Some embodiments further comprise determining baseline total bilirubin levels. In some embodiments, determining baseline total bilirubin levels is performed within 48 hours of birth.

Some embodiments further comprise identifying the presence of at least one risk factor prior to administering a therapeutic amount of the metalloporphyrin to the infant. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

Some embodiments further comprise identifying the presence of at least one exclusion factor prior to administering a therapeutic amount of the metalloporphyrin to the infant. In some embodiments, the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is ≥5.0 mg/dL), persistent hypoglycemia (blood glucose <40 mg/dL) despite standard-of-care treatment, liver diseases defined as ALT and/or AST greater than 2 times the upper limit of normal (ULN), abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36 °C and/or greater than 37.5 °C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, administering a therapeutic amount of a metalloporphyrin and administering a therapeutic amount of phototherapy is performed where no exclusion factor is present.

In some embodiments, administering a therapeutic amount of a metalloporphyrin and administering a therapeutic amount of phototherapy is performed where at least one of a baseline total bilirubin level elevated above a predetermined threshold and at least one risk factor, or a combination thereof is present.

In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, and about 1 to about 3 mg/dL below the threshold for administration of phototherapy according to AAP nomogram corresponding to the infants age.

In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, 1.5 mg/kg, 3.0 mg/kg and 4.5 mg/kg of the infant's weight. In some embodiments, the therapeutic amount of the stannsoporfin is from about 0.75 mg/kg to about 5 mg/kg of the infant's weight. In some embodiments, the therapeutic amount of the stannsoporfin is selected from 0.75 mg/kg, 1.5 mg/kg, 3.0 mg/kg and 4.5 mg/kg of the infant's weight.

In some embodiments, administering a therapeutic amount of a metalloporphyrin is performed in the infant is performed when the infants age is less than about 48 hours. In some embodiments, administering a therapeutic amount of a metalloporphyrin is performed in the infant is performed when the infants age is less than about 20 days of age. In some embodiments, administering a therapeutic amount of a metalloporphyrin is performed in the infant is performed when the infants age is less than about 30 days of age.

In some embodiments, administering a therapeutic amount of a metalloporphyrin and phototherapy is performed simultaneously. In some embodiments, phototherapy is performed at a time selected from within about 12 hours of administration of therapeutic amount of a metalloporphyrin and within about 24 hours of administration of therapeutic amount of a metalloporphyrin.

Some embodiments further comprise conducting on the infant, a physical exam selected from, a dermatologic exam, an audiology exam, an ophthalmological exam, a neurological exam, a laboratory test, an electrocardiogram and a combination thereof.

Some embodiments are directed to methods of reducing the risk of hyperbilirubinemia and the symptoms thereof in an infant, the method comprising administering a therapeutic amount of a metalloporphyrin to the infant wherein the infant has at least one risk factor associated with hyperbilirubinemia.

In some embodiments, the infant has a total bilirubin level of less than about 3 mg/dL below the threshold for administration of phototherapy according to AAP nomogram corresponding to the infant's age.

In some embodiments, administering a therapeutic amount of a metalloporphyrin to the infant comprises administering a single dose of a metalloporphyrin.

In some embodiments, the least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the risk factor is a total bilirubin level at or above a pre-determined threshold. In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for administration of phototherapy to an infant from about 12 to 48 hours of age per the AAP guidelines.

In some embodiments, administering a therapeutic amount of the metalloporphyrin to the infant results in at least one of a decrease in total bilirubin levels compared with total bilirubin levels prior to administering the metalloporphyrin and no detectable increase in total bilirubin levels compared with total bilirubin levels prior to administering the metalloporphyrin.

In some embodiments, the therapeutic amount of the metalloporphyrin is from about 0.75 mg/kg to about 5 mg/kg of infant's weight. In some embodiments, the therapeutic amount of the metalloporphyrin is selected from 0.75 mg/kg, 1.5 mg/kg, 3.0 mg/kg and 4.5 mg/kg of infant's weight. In some embodiments, the therapeutic amount of the stannsoporfin is from about 0.75 mg/kg to about 5 mg/kg of infant's weight. In some embodiments, the therapeutic amount of the stannsoporfin is selected from 0.75 mg/kg, 1.5 mg/kg, 3.0 mg/kg and 4.5 mg/kg of infant's weight.

Some embodiments are directed to methods of stabilizing bilirubin levels in an infant, the method comprising: obtaining a baseline total bilirubin level measurement; and administering a therapeutic amount of a metalloporphyrin to the infant wherein the infant has at least one of hyperbilirubinemia, bilirubin levels above a pre-determined threshold, rising bilirubin levels, and a combination thereof wherein bilirubin levels in the infant are stabilized.

In some embodiments, administering a therapeutic amount of a metalloporphyrin to the infant comprises administering a single dose of a metalloporphyrin.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the infant is of a gestational age from about 35 to about 43 weeks. In some embodiments, the infant has a minimum birth weight of about 2500 g. In some embodiments, the infant has a birth weight from about 1,700 g to about 4,000 g.

In some embodiments, stabilization of total bilirubin levels is achieved when at least two total bilirubin level measurements taken at pre-determined time points after administration of a single therapeutic amount of a metalloporphyrin indicate a total bilirubin level at or below the baseline total bilirubin level.

In some embodiments, the predetermined threshold is about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for administration of phototherapy to an infant from about 12 to 48 hours of age per the AAP guidelines.

In some embodiments, the therapeutic amount of the metalloporphyrin is from about 0.75 mg/kg to about 5 mg/kg. In some embodiments, the therapeutic amount of the metallopoφ hyrin is selected from 0.75 mg/kg, 1.5 mg/kg, 3.0 mg/kg and 4.5 mg/kg. In some embodiments, the therapeutic amount of the stannsoporfin is from about 0.75 mg/kg to about 5 mg/kg. In some embodiments, the therapeutic amount of the stannsoporfin is selected from 0.75 mg/kg, 1.5 mg/kg, 3.0 mg/kg and 4.5 mg/kg.

Some embodiments are directed to methods for treating rising bilirubin levels comprising: establishing a baseline bilirubin level in a patient at risk for hyperbilirubinemia at a predetermined age; administering to the patient a therapeutic amount of stannsoporfin after the baseline is established. In some embodiments, the predetermined age is about 6 hours, about 12 hours, or about 24 hours from birth. In some embodiments, a baseline reading at the AAP nomogram threshold for administering phototherapy or up to 3.0mg/dL below the AAP nomogram threshold for administering phototherapy indicates treatment is required.

Some embodiments are directed to methods of treating hyperbilirubinemia comprising: administering a therapeutic amount of stannsoporfin to a patient in need thereof to achieve a Cmax of at least 5000ng/mL. In some embodiments, the therapeutic amount of stannsoporfin is 1.5mg/kg and achieves a Cmax of about 6450 ng/mL. In some embodiments, the therapeutic amount of stannsoporfin is 3.0 mg/kg and achieves a Cmax of about 11500 ng/mL. In some embodiments, the therapeutic amount of stannsoporfin is 4.5 mg/kg and achieves a Cmax of about 20400 ng/mL. In some embodiments, Cmax is achieved at a Tmax of about 1.5 hours to about 2.5 hours.

Since embodiments herein are directed at treating hyperbilirubinemia with the idea of minimizing the need for phototherapy, the incidence of or need for exchange transfusion or central nervous system injury may also be reduced.

### E. BRIEF DESCRIPTION OF THE FIGURES

Figure 1 is the AAP Nomogram for initiating phototherapy based on age of infant and total serum bilirubin.
Figure 2 is the AAP Nomogram for initiating exchange therapy based on age of infant and total serum bilirubin.
Figure 3 is a nomogram for administering a metalloporphyrin based on a shift in bilirubin level.
Figure 4 is a nomogram for administering a metalloporphyrin based on a shift in age.
Figure 5 is a nomogram for administering a metalloporphyrin based on a shift with respect to assessed risk level.

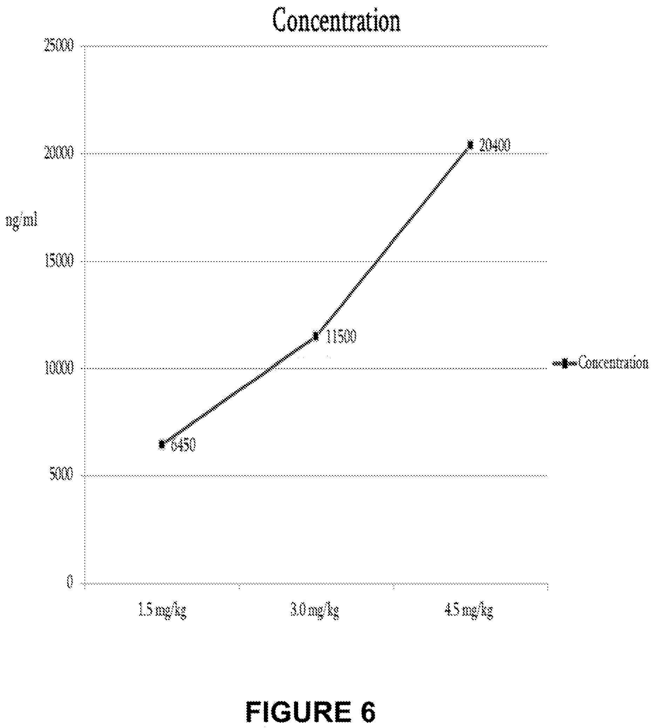

Figure 6 is a graph illustrating the peak serum concentrations of the metalloporphyrin in plasma for 1.5 mg/kg, 3.0 mg/kg and 4.5mg/kg doses.
Figure 7 is a graph detailing total serum bilirubin levels at time points between baseline to 72 hours after treatment with a metalloporphyrin.
Figure 8 is a graph illustrating the bilirubin response curve of 4.5 mg/kg subjects v. placebo subjects.
Figure 9 is a graph illustrating the change from baseline of total serum bilirubin at particular time points for 4.5 mg/kg subjects and placebo subjects.
Figure 10 is a graph indicating total serum bilirubin level for a placebo subject, who was readmitted for phototherapy and who was dosed with stannsoporfin at 39 hours of age, with phototherapy started at 29 hours post dose for a duration of 11 hours and 15 minutes, compared to the phototherapy threshold.
Figure 11 is a graph indicating total serum bilirubin level for a placebo subject, who was readmitted for phototherapy and who was dosed with stannsoporfin at 46 hours of age, with phototherapy started 48 hours of age post dose for 7 hours, compared to the phototherapy threshold.
Figure 12A is a proposed nomogram for high-risk patients based on age and total serum bilirubin.
Figure 12B is a proposed nomogram for medium risk patients based on age and total serum bilirubin.
Figure 12C is a proposed nomogram for low risk patients based on age and total serum bilirubin.
Figure 13 depicts the change in Adjusted TSB (%±SE) LOCF (ITT Population, N=58) (Primary Outcome).
Figure 14 depicts percent Change from Baseline in Unadjusted TSB±SE (ITT Population N=58).

### F. DETAILED DESCRIPTION

Infant hyperbilirubinemia (also known as infant jaundice or neonatal hyperbilirubinemia) occurs in a newborn when the liver is unable to conjugate bilirubin so it can be excreted at a rate commensurate with bilirubin formation. Bilirubin comes from the catabolism of heme as part of the physiological conversion from fetal to adult hemoglobin at birth, or as part of a pathological hemolytic process. The enzyme heme oxygenase oxidizes heme to biliverdin; the enzyme biliverdin reductase then reduces the biliverdin to bilirubin. Bilirubin at high serum levels is a neurotoxic substance. In adult humans, the liver rapidly converts bilirubin into a conjugated, excretable form. In newborn humans, however, the liver is still developing, and uptake and conjugation by the liver is not as efficient as in adults. Additionally, hemolysis may be taking place at a greater relative rate than in adults. All of these factors can lead to excessive bilirubin in the infant. For some infants, high serum levels of bilirubin can have detrimental physiological consequences. Bilirubin is yellow, and infants with excess bilirubin appear jaundiced, having a yellow tinge to their skin and to the whites of their eyes.

Infants who have highly elevated serum levels of bilirubin are at risk of developing kernicterus, a rare but potentially devastating neurological disorder which can result in severe life-long disabilities including cerebral palsy, athetosis, hearing loss, and vision problems. Because early hospital discharge can impair the detection of hyperbilirubinemia in infants, effective means of treating hyperbilirubinemia rapidly are desirable. The unique medical status of a newborn also requires that any means of treatment be as safe as possible, as side effects that are tolerable in adults may be completely unacceptable in neonates.

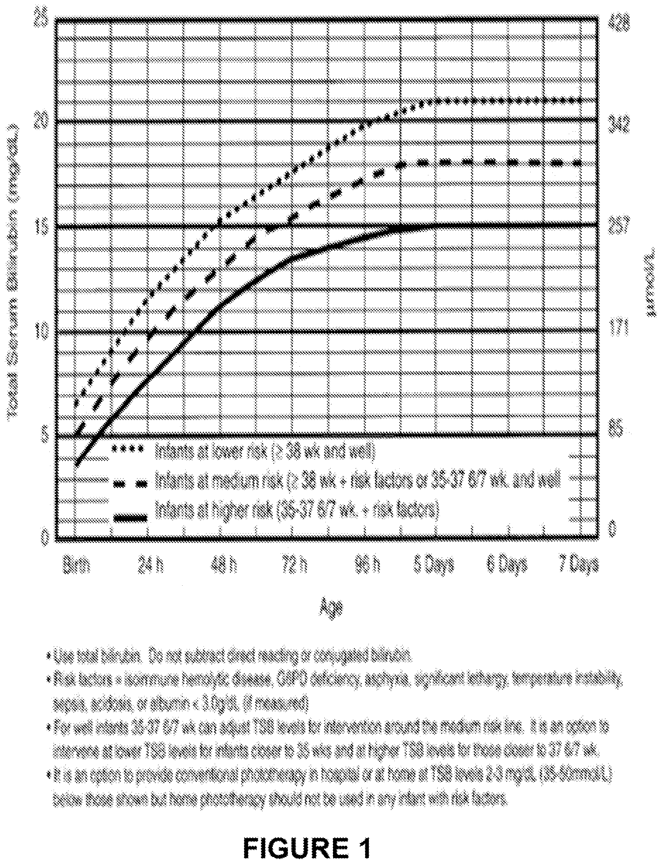

Currently approved and commonly used treatments for hyperbilirubinemia include phototherapy and exchange transfusion. Phototherapy involves irradiating the newborn with light in the 430 to 490 nm range (blue light). The light converts bilirubin into lumirubin and photobilirubin, which are less toxic water-soluble photoisomers that are more readily excreted by the infant, and thus can result in a reduction of bilirubin levels. The decision to initiate phototherapy is based on the newborn's age and total serum bilirubin level, in conjunction with their risk level according to a nomogram approved by the American Academy of Pediatricians (AAP) (see Figure 1). The use of phototherapy requires additional monitoring, patient supervision, and in some cases additional hospital time.

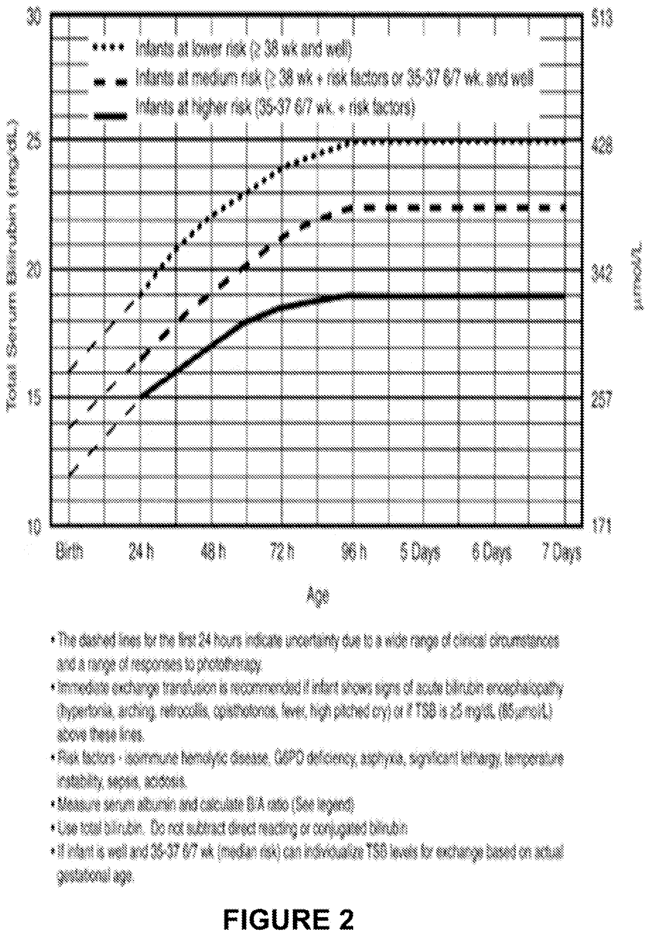

Exchange transfusion should be considered in a newborn with hyperbilirubinemia if intensive phototherapy fails to lower the bilirubin level. This treatment may not be needed when intensive phototherapy is effective. The procedure removes partially hemolyzed and antibody-coated erythrocytes as well as bilirubin and replaces them with uncoated donor red blood cells that lack the sensitizing antigen. Not surprisingly, exchange transfusion may have severe complications and should be avoided, unless necessary. The decision to initiate exchange transfusion is based on the newborn's age and total serum bilirubin level, in conjunction with their risk level according to the nomogram approved by the AAP (see Figure 2).

Infant hyperbilirubinemia constitutes an important medical condition epidemiologically, clinically, and economically. Although its reported incidence varies according to definitions used and populations studied, it is generally accepted that approximately 50% of term and 80% of preterm infants develop jaundice in the first week of life.

The clinical presentation of infant hyperbilirubinemia ranges from mild elevations of bilirubin not requiring any therapeutic intervention to severe hyperbilirubinemia necessitating phototherapy (PT) and/or exchange transfusion (ET). The vast majority of infants affected present with mild-to-moderate hyperbilirubinemia, only requiring laboratory and clinical monitoring. The incidence of severe hyperbilirubinemia (total serum bilirubin [TSB] ≥25 mg/dL) reported for California and the Hospital Corporation of America was 17.9 per 100 000 live births. With 4.13 million births in the United States in 2009, greater than 6% infants underwent PT, and annually, greater than 1,000 are treated with ET. As a result, the AAP issued revised guidelines for the management of neonatal hyperbilirubinemia in 1999 and published a revision in 2004.

Phototherapy for treating infant hyperbilirubinemia and/or jaundice is a well-established technique. There are established guidelines for the timing for initiating phototherapy based upon the age and risk level of the newborn. Based upon these well-established guidelines, including the infant's gestational age and other factors, the infant is assessed a risk level, low, medium, or high. The nomogram for initiating phototherapy approved by the AAP is well known and reproduced as Figure 1. The nomogram establishes when phototherapy should be initiated based upon the infant's measured total serum bilirubin level (mg/dL), the infant's age in hours from birth, and the infant's risk level. For example, Figure 1 indicates that at 36 hours from birth, in a medium risk infant, phototherapy should be initiated if the baby's bilirubin level is about 12 mg/dL or higher. Figure 2 depicts a similar nomogram for initiating an exchange transfusion for extreme cases, particularly where phototherapy has been ineffective or bilirubin levels are exceptionally high.

In some embodiments, administering a metalloporphyrin, e.g. stannsoporfin, prior to attaining the threshold level for initiating phototherapy may significantly reduce bilirubin levels, and dramatically decrease the incidence of or need for phototherapy. In some embodiments, administering a metalloporphyrin, e.g. stannsoporfin, prior to attaining the threshold level may significantly reduce bilirubin levels, and dramatically decrease the incidence of or need for exchange transfusions. Without wishing to be bound by the theory, it is believed that the timing of the administration of the metalloporphyrin, e.g. stannsoporfin, plays a significant role in the reduction of bilirubin levels and reducing the need for phototherapy and/or exchange transfusion. In some embodiments, administering a metalloporphyrin may reduce the duration of phototherapy. In some embodiments, administering a metalloporphyrin may reduce the light intensity of phototherapy.

Stannsoporfin is a synthetic heme analog, which acts as a potent competitive inhibitor of heme oxygenase, the rate-limiting step in the catabolism of heme. Stannsoporfin has been shown to reduce production of bilirubin through heme oxygenases inhibition, creating a rationale for development in clinical situations necessitating the need to reduce bilirubin production. Stannsoporfin has been extensively studied for safety in both in vitro and in vivo studies. Animal studies have demonstrated that stannsoporfin has no biologically significant effects on electrocardiogram (ECG), central nervous system, cardiovascular, pulmonary, and renal functions at doses approximately equivalent to the proposed human dose.

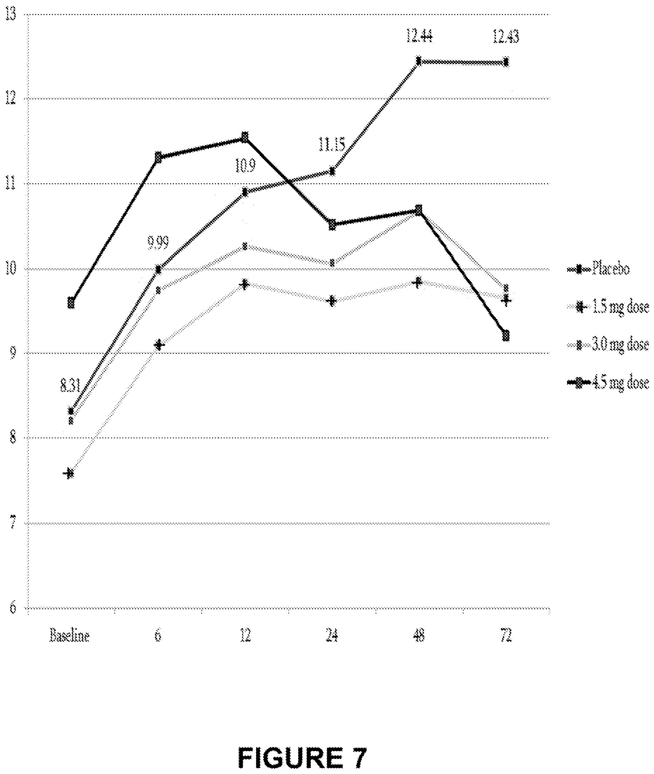

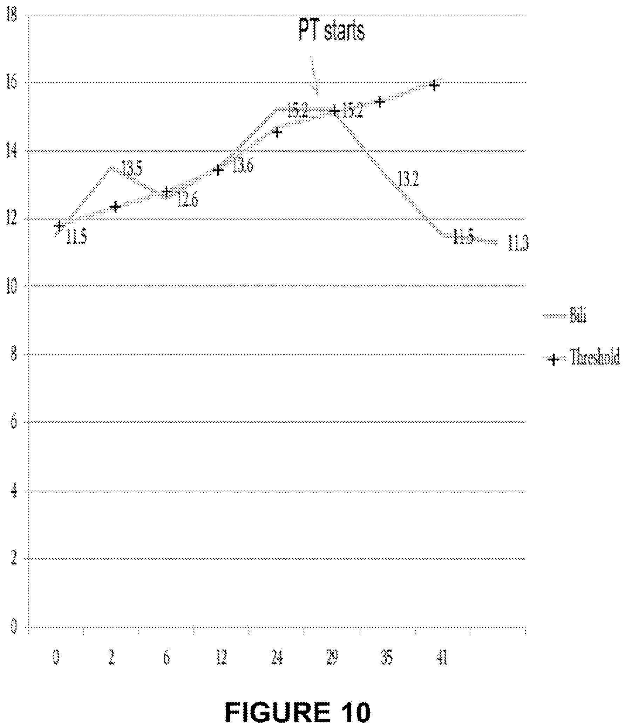

Metalloporphyrins, e.g. stannsoporfin, appear to begin having an effect about 6-12 hours after administration. As shown in Figure 7, after rising for about the first 12 hours after administration of a metalloporphyrin, e.g. stannsoporfin, bilirubin levels plateau and then significantly drop off beyond 48 hours (see 1.5 mg/kg, 3.0 mg/kg and 4.5 mg/kg doses) whereas the placebo continues to rise and rises significantly at 24 hours and beyond. For example, as seen in Figure 10, a single 1.5 mg/kg dose given at 36 hours to medium risk infants having a total serum bilirubin of 3 mg/dL (about 9 mg/dL) less than the phototherapy threshold (about 12 mg/dL). Additionally, as seen in Figure 10, in these treated infants, only 17.6% of those treated with a single 1.5 mg/kg dose required subsequent phototherapy, compared to 53.3% with placebo.

Without wishing to be bound by theory, it is believed that earlier administration allows the drug time to work and therefore results in less need for phototherapy. Administering the dose at about 2 to about 3 mg/dL below the phototherapy threshold may approximate the 6-12 hour delay believed to be required for efficacy of the metalloporphyrin, e.g. stannsoporfin.

Accordingly, some embodiments provide a method of reducing the need for intervention in the treatment of hyperbilirubinemia comprising administering a metalloporphyrin to a subject in need thereof. In some embodiments, the method reduces the need for intervention by phototherapy and/or exchange transfusion. In some embodiments, administration of the metalloporphyrin may occur when the infant's measured total serum bilirubin level is at or below about the level suggested by the AAP nomogram for initiating phototherapy for an infant of the known age and known risk level.

Some embodiments provide a method of treating hyperbilirubinemia, the method comprising administering a therapeutic amount of a metalloporphyrin, such as stannsoporfin, to an infant of a known age and having a known risk level for hyperbilirubinemia; wherein the administration occurs when the infant's measured total serum bilirubin level is at or below about the level suggested by the AAP nomogram for initiating phototherapy for an infant of the known age and known risk level.

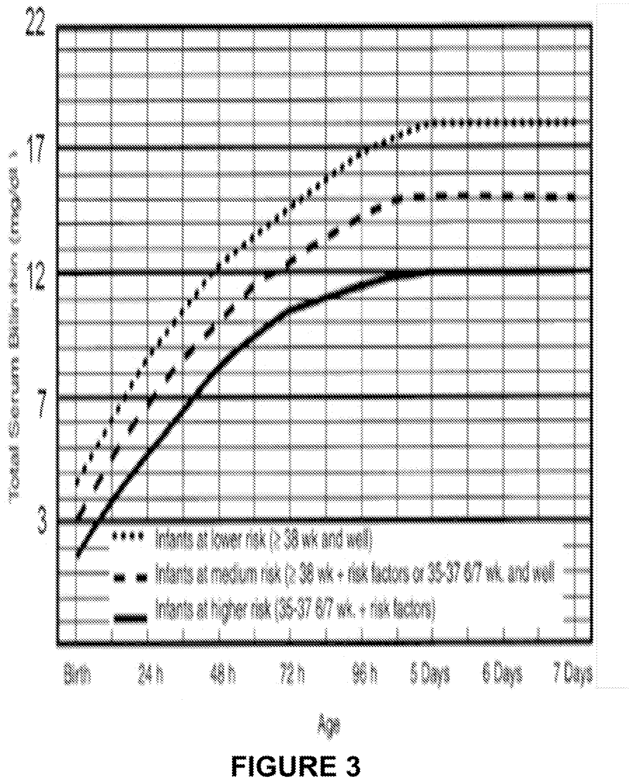

In some embodiments, the metalloporphyrin may be administered when the infant's serum bilirubin level is about 0.5 mg/dL below to about 3 mg/dL below that required to qualify for phototherapy. In some embodiments, the metalloporphyrin may be administered when infant's bilirubin level is about 1 mg/dL to about 3.0 mg/dL below that required to initiate phototherapy. In some embodiments, the metalloporphyrin may be administered when the infant's serum bilirubin level is about 2 mg/dL below to about 3 mg/dL below that required to qualify for phototherapy. In some embodiments, the metalloporphyrin may be administered when total serum bilirubin levels reach the levels indicated by Figure 3 for a known risk level at a known age.

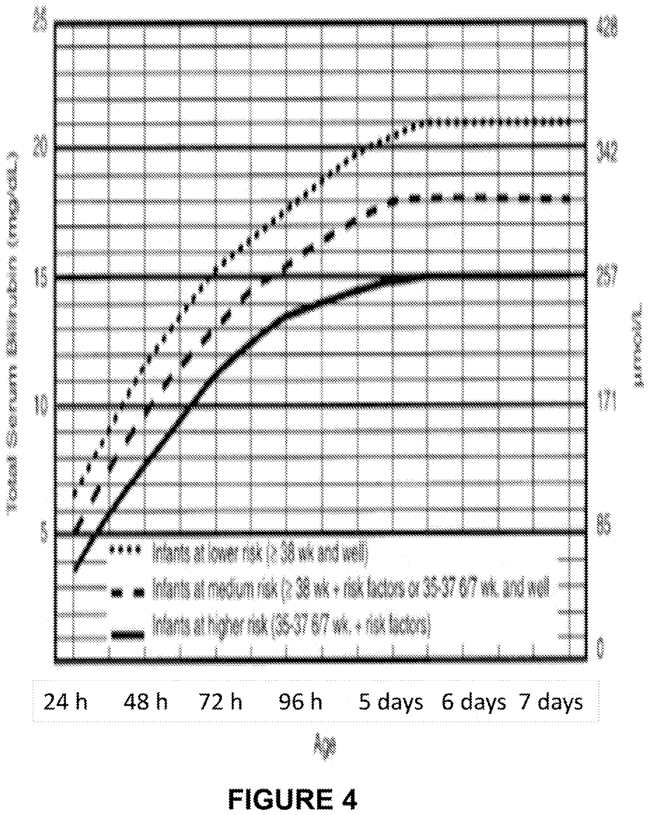

In some embodiments, a method of treating hyperbilirubinemia comprises administering a therapeutic amount of a metalloporphyrin to an infant of a known age and having a known risk level for hyperbilirubinemia; wherein the administration occurs when the infant's measured total serum bilirubin levels are at about the level suggested for initiating phototherapy in an infant of the same known risk level at the known age minus about 12 to about 24 hours. In some embodiments, the metalloporphyrin may be administered when total serum bilirubin levels reach the levels indicated by Figure 4 for a known risk level at a known age.

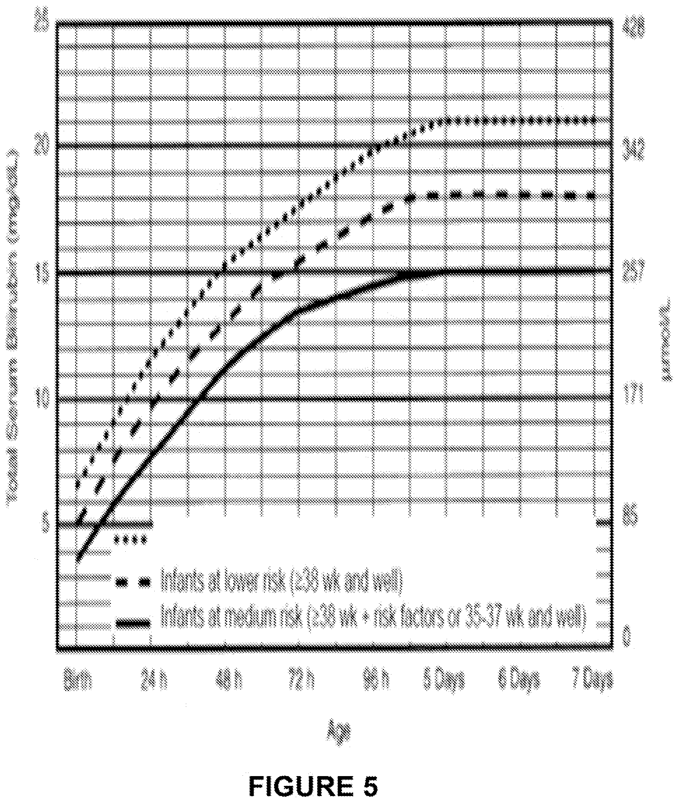

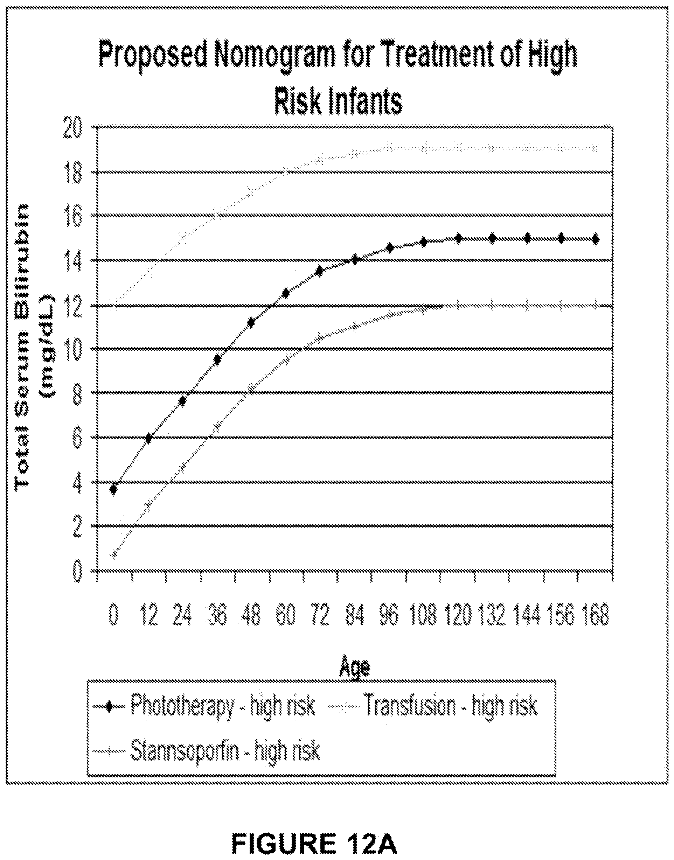

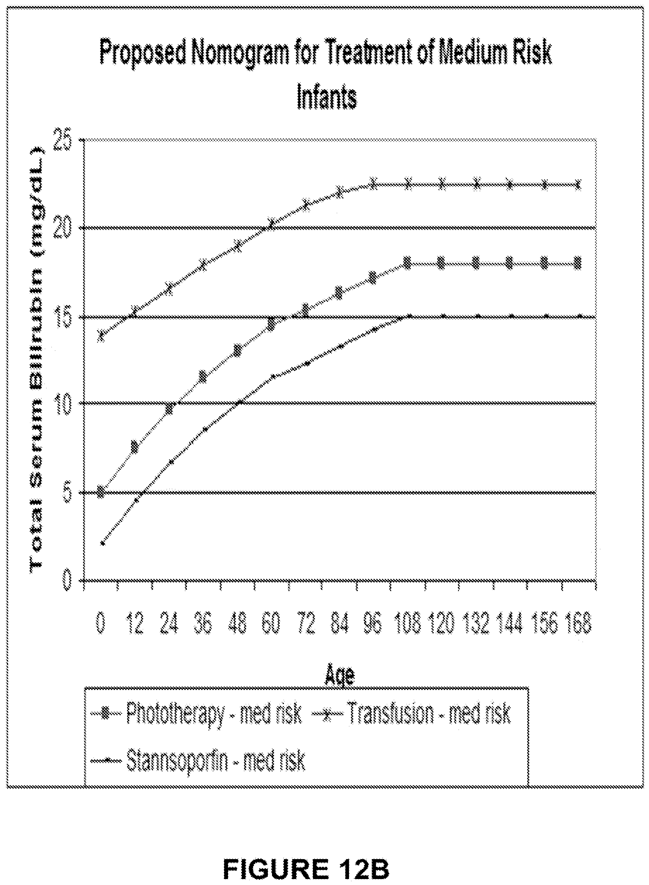

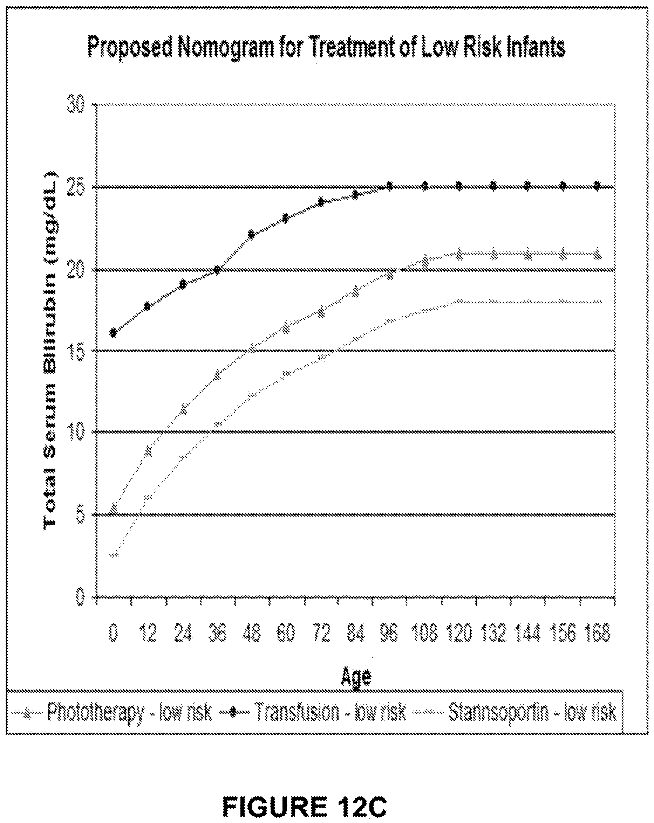

In some embodiments, a method of treating hyperbilirubinemia comprises administering a therapeutic amount of a metalloporphyrin to an infant of a known age and having a known risk level for hyperbilirubinemia; wherein the administration occurs when the infant's measured total serum bilirubin levels are at about the level suggested for initiating phototherapy at the next highest risk level in an infant of the same known age, where the infant to be treated is low or medium risk. In some embodiments, the metalloporphyrin may be administered when total serum bilirubin levels reach the levels indicated by Figure 5 for a known risk level at a known age. In some embodiments, the metalloporphyrin may be administered when total serum bilirubin levels reach the levels indicated by Figures 12A-12C for a known risk level at a known age.

In some embodiments, the level suggested for initiating phototherapy is determined by the use of a modified AAP nomogram. In some embodiments, the subject may be an infant. In some embodiments, the subject may have a gestational age of from about 35 to about 43 weeks. In some embodiments, the subject may have a birth weight of from about 1700 to about 4000 grams. In some embodiments, the subject's age at the time of treatment may be from about birth to about 20 days. In some embodiments, the subject may be at elevated risk for needing intervention. In some embodiments, intervention may include phototherapy, exchange transfusion or a combination thereof. In some embodiments, the method may further comprise administering phototherapy to the subject in accordance with accepted practice.

Some embodiments herein are also directed to a method of reducing the duration of phototherapy needed to lower bilirubin levels comprising administering a metalloporphyrin to a subject in need thereof. In some embodiments, the administration of the metalloporphyrin eliminates the need for phototherapy. In some embodiments, the administration of the metalloporphyrin reduces the duration of phototherapy by from about 0.5 to about 168 hours, 0.5 to about 150 hours, 0.5 to about 125 hours, 0.5 to about 100 hours, 0.5 to about 75 hours, 0.5 to about 50 hours, 0.5 to about 25 hours, 0.5 to about 20 hours, 0.5 to about 15 hours, 0.5 to about 10 hours, 1 to about 168 hours, 1 to about 150 hours, 1 to about 125 hours, 1 to about 100 hours, 1 to about 75 hours, 1 to about 50 hours, from about 1 to about 25 hours, from about 1 to about 20 hours, from about 1 to about 15 hours, from about 1 to about 10 hours, 2 to about 168 hours, 2 to about 150 hours, 2 to about 125 hours, 2 to about 100 hours, 2 to about 75 hours, 2 to about 50 hours, from about 2 to about 25 hours, from about 2 to about 20 hours, from about 2 to about 15 hours, from about 2 to about 10 hours, from about 3 to about 10 hours, from about 4 to about 10 hours, from about 5 to about 10 hours, from about 6 to about 10 hours, from about 1 to about 8 hours, from about 2 to about 8 hours, from about 3 to about 8 hours, from about 4 to about 8 hours, from about 5 to about 8 hours, or from about 6 to about 8 hours. In some embodiments, the administration of the metalloporphyrin reduces the duration of phototherapy by greater than 25 hours.

In some embodiments, administering the metalloporphyrin, e.g. stannsoporfin, about 12 hours before an infant's bilirubin level reaches a phototherapy threshold may significantly reduce the likelihood that phototherapy will be needed. Unfortunately, it is impossible to predict when an infant will reach a certain threshold. It is possible, however, to establish a threshold for administering metalloporphyrin, e.g. stannsoporfin, to an infant that is at risk of needing phototherapy. The threshold may be based upon measured total serum bilirubin in mg/dL, the infant's age, and the infant's risk level. In some embodiments, the twelve-hour advance treatment may be approximated by administering metalloporphyrin, e.g. stannsoporfin, when measured total serum bilirubin levels of the subject are:
a. at least about 0.5 to about 3 mg/dL below the level suggested by AAP nomogram for initiating phototherapy for an infant of a known age and a known risk level; OR
b. at least about the level suggested by the AAP nomogram for initiating phototherapy in an infant of the known risk level at the known age minus about 12 to about 24 hours; OR
c. at least about the level suggested by the AAP nomogram for initiating phototherapy at the next highest risk level in an infant of the known age, where the infant to be treated is low or medium risk.

### Administration based on nomogram shifted with respect to the phototherapy threshold

Some embodiments are directed to a method of treating hyperbilirubinemia or the symptoms thereof in an infant, the method comprising: administering a therapeutic amount of a metalloporphyrin to the infant with hyperbilirubinemia where no exclusion factor is present and at least one of a baseline total bilirubin level is elevated above a predetermined threshold and at least one risk factor is present; wherein the hyperbilirubinemia or symptoms thereof is treated.

Some embodiments further comprise detennining baseline total bilirubin levels in the infant. In some embodiments, baseline total bilirubin levels comprises total serum bilirubin levels, total cutaneous bilirubin or a combination thereof.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, determining baseline total bilirubin levels is performed at a time selected from within 6 hours of birth, 12 hours of birth, within 24 hours of birth, and within 48 hours of birth.

Some embodiments further comprise identifying the presence of at least one risk factor. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, and G6PD deficiency and combinations thereof.

Some embodiments further comprise identifying the absence of at least one exclusion factor. In some embodiments, the at least one exclusion factor is selected from, a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is >5.0 mg/dL), persistent hypoglycemia (blood glucose <40 mg/dL) despite standard-of-care treatment, liver diseases defined as ALT and/or AST greater than 2 times the upper limit of normal [ULN], abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, the predetermined threshold is the level determined by the AAP nomogram for initiating phototherapy for an infant of known age and known risk level. In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, is about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines and about 1 to about 3 mg/dL below the threshold for administration of phototherapy according to AAP nomogram corresponding to the infants age.

In some embodiments, administering a therapeutic amount of a metalloporphyrin is performed at a time selected from within about 6 hours of birth, within about 12 hours of birth, within about 24 hours of birth and within about 48 hours of birth.

In some embodiments, the metalloporphyrin is selected from tin mesoporphyrin, zinc mesoporphyrin, chromium mesoporphyrin, tin protoporphyrin, zinc protoporphyrin, chromium protoporphyrin, bis glycol protoporphyrin and ferroporphyrin. In some embodiments, the metalloporphyrin is tin mesoporphyrin (also referred to as stannsoporfin).

In some embodiments, the therapeutic amount of the metalloporphyrin is from about 0.75 mg/kg to about 5 mg/kg of the infants weight. In some embodiments, the therapeutic amount of the metalloporphyrin is selected from 0.75 mg/kg, 1.5 mg/kg, 3.0 mg/kg and 4.5 mg/kg of the infants weight. In some embodiments, the therapeutic amount of the tin mesoporphyrin is from about 0.75 mg/kg to about 5 mg/kg of the infants weight. In some embodiments, the therapeutic amount of the tin mesoporphyrin is selected from 0.75 mg/kg, 1.5 mg/kg, 3.0 mg/kg and 4.5 mg/kg of the infants weight.

In some embodiments, the metalloporphyrin is administered by intramuscular injection.

In some embodiments, administering a therapeutic amount of a metalloporphyrin is performed when the infants age is less than 20 days of age. In some embodiments, administering a therapeutic amount of a metalloporphyrin is performed when the infants age is less than 30 days of age.

Some embodiments further comprise administering phototherapy where total bilirubin levels following administration of the metalloporphyrin are above the baseline total bilirubin levels.

Some embodiments further comprise determining post treatment total bilirubin levels following administration of the metalloporphyrin. In some embodiments, determining post treatment total bilirubin levels following administration of the metalloporphyrin is performed from about 6 and to about 72 hours after administering the metallopo rphyrin to the infant. In some embodiments, post treatment total bilirubin levels are at least 5% below the baseline total bilirubin levels 24 hours after administering a therapeutic amount of a metalloporphyrin to the infant. In some embodiments, post treatment total bilirubin levels are at least 10% below the baseline total bilirubin levels 48 hours after administering a therapeutic amount of a metalloporphyrin to the infant. In some embodiments, post treatment total bilirubin levels are at least 20% below the baseline total bilirubin levels 72 hours after administering a therapeutic amount of a metalloporphyrin to the infant. In some embodiments, post treatment total bilirubin levels are less than 3mg/dL above the baseline total bilirubin levels 48 hours after administering a therapeutic amount of a ηmetBïIoρorphyrin to the infant.

Some embodiments further comprise conducting on the infant one or more examinations selected from a physical exam, a dermatologic exam, an audiology exam, an ophthalmological exam, a neurological exam, a laboratory test, an electrocardiogram and a combination thereof. The examinations may be administered pre-treatment, post-treatment, and/or post discharge from the hospital. Post treatment examinations may be repeated to evaluate treatment effect as well as any adverse events.

In some embodiments, administering a metalloporphyrin, e.g. stannsoporfin, to an infant when the infant's total serum bilirubin is at least 0.5 mg/dL below, at least about 0.5 mg/dL to about 3 mg/dL below the nomogram threshold level for phototherapy given the infant's age and risk level may allow sufficient time for the drug to exert its effect on bilirubin production. For example, again referring to Figure 1 and the threshold of about 12mg/dL for initiating phototherapy in a medium risk infant at 36 hours of age, under this methodology, metalloporphyrin, e.g. stannsoporfin, should be administered to the infant at about 2 to about 3 mg/dL below that threshold, or at about 9 to about 10 mg/dL. Doing so may significantly reduce the need for phototherapy 12 hours later, that is, fewer infants would require phototherapy at the 48-hour mark.

In some embodiments, this threshold shift may be age related, with older infants benefitting from a larger threshold reduction. For example, in an infant about 12 to about 48 hours old, administration may occur at about 3 mg/dL below the phototherapy threshold at the given age. In an infant less than 12 hours old, administration may occur at about 2 mg/dL below the phototherapy threshold at the given age. Figure 3 shows a proposed nomogram of when metalloporphyrin, e.g. stannsoporfin, may be administered illustrating a 3mg/dL shift from the phototherapy threshold. In some embodiments, the metalloporphyrin may be administered when total serum bilirubin levels reach the levels indicated by Figure 3.

### Administration based on nomogram shifted with respect to age

In some embodiments, metalloporphyrin may be administered to the subject when the bilirubin level is about the same as the threshold level for a subject about 12 to about 24 hours younger but similarly situated subject. For example, a 36-hour-old subject at medium risk may be administered metalloporphyrin, e.g. stannsoporfin, if its bilirubin level (at 36 hours) is about 9.7 mg/dL (the phototherapy threshold for a 24-hour medium risk infant). Essentially, the nomogram for initiating phototherapy is shifted 12 hours to the right for establishing a nomogram for administering metalloporphyrin, e.g. stannsoporfin. Figure 4 shows a proposed nomogram illustrating a 12-hour shift. In some embodiments, the metalloporphyrin may be administered when total serum bilirubin levels reach the levels indicated by Figure 4.

### Administration based on nomogram shifted with respect to risk level

In some embodiments, the metalloporphyrin may be administered when the total serum bilirubin of a subject are at or above the levels indicated by Figures 12A-12C. Figure 5 shows a proposed nomogram in which the patient's risk level is shifted to the next higher risk level. For example, as shown in Figure 5, the proposed nomogram indicates phototherapy should begin at about 12 mg/dL in a low risk subject at 36 hours and in a medium risk infant at about 9.5 mg/dL. This works for both low and medium risk babies, which would begin metalloporphyrin, e.g. stannsoporfin, therapy at the higher risk phototherapy threshold. In some embodiments, high-risk infants may be treated according to the other theories. In some embodiments, the metalloporphyrin may be administered when total serum bilirubin levels reach the levels indicated by Figure 5.

Figures 12A-12C set forth proposed nomograms for high, medium, and low risk infants, respectively, based upon a 3.0 mg/dL shift with respect to total serum bilirubin levels. Thus, once a subject's risk level is assessed, a nomogram such as those shown in Figures 12A-12C may be used to determine what type of treatment should be initiated. For example, Figure 12A is a nomogram for high-risk infants. This single nomogram indicates where, at a given age and TSB level to administer stannsoporfin, phototherapy, or exchange transfusion. In some instances, combined therapies may be advised. In some embodiments, the metalloporphyrin may be administered when total serum bilirubin levels reach the levels indicated by Figures 12A-12C. Figures 12A-12C also disclose embodiments in which phototherapy and/or exchange transfusion may be initiated based on the level of serum bilirubin, age of the subject and risk level of the subject. Similar proposed nomograms can be prepared for shifts for metalloporphyrin treatment based on age related shift and risk level shift as discussed above. These graphs were developed from visual inspection of the AAP nomograms, and may differ slightly from those nomograms since we were not privy to the actual numerical data. Any difference is unintended.

### Administration based on rising bilirubin

Some embodiments are directed to a method of reducing the likelihood of hyperbilirubinemia and the symptoms thereof in an infant, the method comprising: administering a therapeutic amount of a metalloporphyrin to the infant where the infant's total bilirubin is determined to be increasing in at least one total bilirubin measurement compared with a previous total bilirubin level, wherein the likelihood of hyperbilirubinemia or the symptoms thereof is increased.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, where the infant's total bilirubin is determined to be increasing in two consecutive total bilirubin measurements. In some embodiments, the baseline total bilirubin measurement is performed from about 6 to about 96 hours of age. In some embodiments, the baseline total bilirubin measurement is performed at about 6, 12, 24, 48, 72, or 96 hours of age. In some embodiments, the at least one total bilirubin measurement is performed from about 6 to about 72 hours after the baseline total bilirubin measurement.

In some embodiments, administering a therapeutic amount of a metalloporphyrin is performed within about 1 to about 6 hours of when the infant's total bilirubin is determined to be increasing in at least one total bilirubin measurement.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the therapeutic amount of the metalloporphyrin is from about 0.75 mg/kg to about 5 mg/kg of the infants weight. In some embodiments, the therapeutic amount of the metalloporphyrin is selected from 0.75 mg/kg, 1.5 mg/kg, 3.0 mg/kg and 4.5 mg/kg of the infants weight. In some embodiments, the therapeutic amount of the stannsoporfm is from about 0.75 mg/kg to about 5 mg/kg of the infants weight. In yet other embodiments, the therapeutic amount of the stannsoporfin is selected from 0.75 mg/kg, 1.5 mg/kg, 3.0 mg/kg and 4.5 mg/kg of the infants weight.

Some embodiments further comprise conducting on the infant one or more examinations selected from a physical exam, a dermatologic exam, an audiology exam, an ophthalmological exam, a neurological exam, a laboratory test, an electrocardiogram and a combination thereof. The examinations may be administered pre-treatment, post-treatment, and/or post discharge from the hospital. Post treatment examinations may be repeated to evaluate treatment effect as well as any adverse events.

### Administration of a metalloporphyrin to stabilize bilirubin levels

Some embodiments are directed to a method of stabilizing bilirubin levels in an infant, the method comprising: obtaining a baseline total bilirubin level measurement; and administering a therapeutic amount of a metalloporphyrin to the infant wherein the infant has at least one of hyperbilirubinemia, bilirubin levels above a pre-determined threshold, rising bilirubin levels, and a combination thereof wherein bilirubin levels in the infant are stabilized.

In some embodiments, administering a therapeutic amount of a metalloporphyrin to the infant comprises administering a single dose of a metalloporphyrm.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, the infant is of a gestational age from about 35 to about 43 weeks. In some embodiments, the infant has a minimum birth weight of about 2500 g. In some embodiments, the infant has a birth weight from about 1,700 g to about 4,000 g.

In some embodiments, stabilization of total bilirubin levels is achieved when at least two total bilirubin level measurements taken at pre-determined time points after administration of a single therapeutic amount of a metalloporphyrin indicate a total bilirubin level at or below the baseline total bilirubin level.

In some embodiments, the predetermined threshold is about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for administration of phototherapy to an infant from about 12 to 48 hours of age per the AAP guidelines.

In some embodiments, the therapeutic amount of the metalloporphyrin is from about 0.75 mg/kg to about 5 mg/kg. In some embodiments, the therapeutic amount of the metalloporphyrin is selected from 0.75 mg/kg, 1.5 mg/kg, 3.0 mg/kg and 4.5 mg/kg. In some embodiments, the therapeutic amount of the stannsoporfin is from about 0.75 mg/kg to about 5 mg/kg. In some embodiments, the therapeutic amount of the stannsoporfin is selected from 0.75 mg/kg, 1.5 mg/kg, 3.0 mg/kg and 4.5 mg/kg.

### Combined administration of a metalloporphyrin and phototherapy

Some embodiments are directed to a method of treating hyperbilirubinemia and the symptoms thereof in an infant, the method comprising: administering a therapeutic amount of a metalloporphyrin to the infant; and administering a therapeutic amount of phototherapy to the infant wherein the hyperbilirubinemia or symptoms thereof is treated.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

Some embodiments further comprise determining baseline total bilirubin levels.

In some embodiments, determining baseline total bilirubin levels is performed within 48 hours of birth.

In some embodiments, the presence of at least one risk factor is identified prior to administering a therapeutic amount of the metalloporphyrin to the infant. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

Some embodiments further comprise identifying the presence of at least one exclusion factor prior to administering a therapeutic amount of the metalloporphyr n to the infant. In some embodiments, the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is >5.0 mg/dL), persistent hypoglycemia (blood glucose <40 mg/dL) despite standard-of-care treatment, liver diseases defined as ALT and/or AST greater than 2 times the upper limit of normal [ULN], abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, administering a therapeutic amount of a metalloporphyrin and administering a therapeutic amount of phototherapy is performed where no exclusion factor is present.

In some embodiments, administering a therapeutic amount of a metalloporphyrin and administering a therapeutic amount of phototherapy is performed where at least one of a baseline total bilirubin level elevated above a predetermined threshold and at least one risk factor, or a combination thereof is present.

In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, and about 1 to about 3 mg/dL below the threshold for administration of phototherapy according to AAP nomogram corresponding to the infants age.

In some embodiments, the therapeutic amount of the metalloporphyrin is from about 0.75 mg/kg to about 5 mg/kg of the infants weight. In some embodiments, the therapeutic amount of the metalloporphyrin is selected from 0.75 mg/kg, 1.5 mg/kg, 3.0 mg/kg and 4.5 mg/kg of the infants weight. In some embodiments, the therapeutic amount of the stannsoporfin is from about 0.75 mg/kg to about 5 mg/kg of the infants weight. In some embodiments, the therapeutic amount of the stannsoporfin is selected from 0.75 mg/kg, 1.5 mg/kg, 3.0 mg/kg and 4.5 mg/kg of the infants weight.

In some embodiments, administering a therapeutic amount of a metalloporphyrin is performed in the infant is performed when the infants age is less than about 48 hours. In some embodiments, administering a therapeutic amount of a metalloporphyrin is performed in the infant is performed when the infants age is less than about 20 days of age. In some embodiments, administering a therapeutic amount of a metalloporphyrin is performed in the infant is performed when the infants age is less than about 30 days of age.

In some embodiments, administering a therapeutic amount of a metalloporphyrin and phototherapy is performed simultaneously. In some embodiments, phototherapy is performed at a time selected from within about 12 hours of administration of therapeutic amount of a metalloporphyrin and within about 24 hours of administration of therapeutic amount of a metallopoiphyrin.

Some embodiments further comprise conducting on the infant, a physical exam selected from, a dermatologic exam, an audiology exam, an ophthalmological exam, a neurological exam, a laboratory test, an electrocardiogram and a combination thereof.

### Early or prophylactic treatment

One concern in treating infants, and especially newborns and pre-term babies, and especially for prophylactic treatment, is that their developing systems react differently than do adults. An infant's body is often not able to handle such treatments. The FDA, doctors, and parents alike all share the thought that more often than not, it is better NOT to treat an infant until it is proven that such treatment is necessary. There are of course some exceptions. Newborn babies are routinely, and in some cases by statutory mandate, treated with an antibiotic solution in each eye to prevent infections that were once commonplace, and dangerous. Although some of these babies are not at risk of infection, and some may never have been infected, the benefit of treating each baby regardless of risk outweighs the risk associated with the treatment. Treating babies prophylactically with antibiotic eye drops is now commonplace and has dramatically decreased the number of eye infections in newborns. This, potentially, could be the case for infant hyperbilirubinemia and/orjaundice.

Some embodiments are directed to a method of reducing the risk of hyperbilirubinemia and the symptoms thereof in an infant, the method comprising: administering a therapeutic amount of a metalloporphyrin to the infant wherein the infant has at least one risk factor associated with hyperbilirubinemia.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, the infant has a total bilirubin level of less than about 3 mg/dL below the threshold for administration of phototherapy according to AAP nomogram corresponding to the infants age.

In some embodiments, administering a therapeutic amount of a metalloporphyrin to the infant comprises administering a single dose of a metalloporphyrin. In some embodiments, the least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the risk factor is a total bilirubin level at or above a pre-determined threshold. In some embodiments, the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for administration of phototherapy to an infant from about 12 to 48 hours of age per the AAP guidelines.

In some embodiments, administering a therapeutic amount of the metalloporphyrin to the infant results in at least one of a decrease in total bilirubin levels compared with total bilirubin levels prior to administering the metalloporphyrin and no detectable increase in total bilirubin levels compared with total bilirubin levels prior to administering the metalloporphyrin.

In some embodiments, the therapeutic amount of the metalloporphyrin is from about 0.75 mg/kg to about 5 mg/kg of infants weight. In some embodiments, the therapeutic amount of the metalloporphyrin is selected from 0.75 mg/kg, 1.5 mg/kg, 3.0 mg/kg and 4.5 mg/kg of infants weight. In some embodiments, the therapeutic amount of the stannsoporfin is from about 0.75 mg/kg to about 5 mg/kg of infants weight. In some embodiments, the therapeutic amount of the stannsoporfin is selected from 0.75 mg/kg, 1.5 mg/kg, 3.0 mg/kg and 4.5 mg/kg of infants weight.

In some embodiments, the drug may be used in high risk and/or medium risk infants without regard to total serum bilirubin levels in a prophylactic manner or at least with early intervention.

In some embodiments, a method for treating hyperbilirubinemia in an infant comprises administering a therapeutic amount of a metalloporphyrin, such as stannsoporfin, without regard to the total serum bilirubin level of the infant. In some embodiments, the administration occurs without regard to the risk level of the infant. In some embodiments, the risk level is unknown and/or not assessed. In some embodiments, the infant is known or suspected to be a medium risk infant. In some embodiments, the infant is known or suspected to be a high-risk infant.

Regardless of the timing or reasoning for administration, the following, with regard to the infant's age, weight, risk level, as well as the type and formulation of metalloporphyrin to be administered apply to each of the methods disclosed herein.

In some embodiments, the subject may be at medium or high risk for hyperbilirubinemia requiring intervention by phototherapy and/or exchange transfusion according to the 2004 AAP Guidelines (updated October 2009). The risk level may be defined as follows: medium risk: term (>38 weeks gestation) with risk factors (iso-immune hemolytic disease); or near-term infants (>35 to 37 and 6/7 weeks gestation) and well (with no risk factors); and high risk: near-term infants (>35 to 37 and 6/7 weeks gestation) with risk factors (iso-immune hemolytic disease) for an exchange transfusion.

In some embodiments, the subject may be a term or near term infant. In some embodiments, the gestational age of the subject may be from about 35 to about 43 weeks. In some embodiments, the gestational age of the subject may be from about 35 to about 45 weeks, about 35 to about 40 weeks, about 35 to about 39 weeks or about 35 to about 38 weeks.

In some embodiments, the birth weight of the subject may be from about 1700 to about 4000 grams. In some embodiments, the birth weight of the subject may be from about 2000 to about 4000 grams, from about 2000 to about 3700 grams or from about 2300 to about 3000 grams. At the time of treatment, the subject's age may be from birth to about 20 days, from birth to about 15 days, from about 1 day to about 20 days, from about 1 day to about 15 days, or from about 4 to about 13 days. At the time of treatment, the subject's serum bilirubin levels may be greater than about 14 mg/dL, less than about 30 mg/dL, from about 15 to about 30 mg/dL, from about 20 to about 30 mg/dL, or from about 25 to about 29 mg/dL. In some embodiments, the subject's serum bilirubin levels may be about 2 to about 3 mg/dL below that required to qualify for exchange transfusion.

Some embodiments herein include a method of treating hyperbilirubinemia in an infant comprising administering a metalloporphyrin to the infant, wherein the metalloporphyrin does not cause any adverse events. Some embodiments herein include a method of treating hyperbilirubinemia in an infant comprising administering a metalloporphyrin to the infant, wherein the metalloporphyrin does not cause QT prolongation.

Embodiments herein include a method of reducing or preventing jaundice comprising administration of a metalloporphyrin to a subject in need thereof.

In some embodiments, the treatment may decrease the duration of hospitalization by at least about 20 hours, by from about 20 to about 60 hours or by from about 30 to about 50 hours. In some embodiments, the duration of the hospital stay may be from about 130 to about 200 hours, from about 140 to about 170 hours or from about 140 to about 150 hours.

Although the administration of the metalloporphyrins in accordance with the methods herein often remove the need for further intervention altogether, in some embodiments, it is anticipated that such intervention may be accompanied by phototherapy without the need for exchange transfusion. Such phototherapy may be appropriate when the baby's bilirubin level appears to be rising at a fast rate. The use of phototherapy in addition to the administration of the metalloporphyrin in accordance with the methods herein may either reduce the amount of phototherapy needed and/or alleviate the need for an exchange transfusion.

In some embodiments, the method further comprises administering phototherapy to the subject in need thereof. In some embodiments, phototherapy may be administered for a duration of about 6 to about 90 hours, from about 60 to about 90 hours or from about 60 to about 85 hours. In some embodiments, phototherapy may be administered in about 6 hour to about 12 hour aliquots. In some embodiments, reassessment of the infant is performed between aliquots of phototherapy. In some embodiments, the mean duration of phototherapy is about 48 hours to about 72 hours. In some embodiments, phototherapy is administered at a light intensity of from about 10 µw/cm2 to about 40 µw/cm2, from about 20 µw/cm2 to about 40 µw/cm2, from about 25 µw/cm2 to about 40 µw/cm2, from about 30 µw/cm2 to about 40 µw/cm2, or from about 30 to about 35 µw/cnl2. In some embodiments, administration of the metalloporphyrin reduces the amount of phototherapy needed to lower bilirubin levels. In some embodiments, the administration of the metalloporphyrin reduces the duration of phototherapy by from about 0.5 to about 25 hours, 0.5 to about 20 hours, 0.5 to about 15 hours, 0.5 to about 10 hours, from about 1 to about 25 hours, from about 1 to about 20 hours, from about 1 to about 15 hours, from about 1 to about 10 hours, from about 2 to about 25 hours, from about 2 to about 20 hours, from about 2 to about 15 hours, from about 2 to about 10 hours, from about 3 to about 10 hours, from about 4 to about 10 hours, from about 5 to about 10 hours, from about 6 to about 10 hours, from about 1 to about 8 hours, from about 2 to about 8 hours, from about 3 to about 8 hours, from about 4 to about 8 hours, from about 5 to about 8 hours, or from about 6 to about 8 hours. In some embodiments, the subject may receive phototherapy prior to the metalloporphyrin treatment. In some embodiments, the subject may receive phototherapy in conjunction with the metalloporphyrin treatment. In some embodiments, the subject may receive phototherapy after the metalloporphyrin treatment. In some embodiments, the administration of the metalloporphyrin eliminates the need for phototherapy.

In some embodiments, the metalloporphyrin is administered before an exchange transfusion. In some embodiments, the metalloporphyrin is administered instead of performing an exchange transfusion. In some embodiments, the subject may receive an exchange transfusion prior to treatment. In some embodiments, the subject may receive an exchange transfusion after treatment.

Another aspect of this invention is directed towards a method of lowering bilirubin levels in a mammal comprising parenterally administering a metalloporphyrin composition. While the intended recipients of this medication to treat hyperbilirubinemia are humans, particularly infants, the metalloporphyrin solution may also be effective in other mammals.

### Screening and Baseline Assessments

Some embodiments further comprise determination of eligibility and screening assessments. In some embodiments, determination of eligibility and screening assessments include but are not limited to transcutaneous bilirubin (TcB) monitoring, an audiology examination including auditory brainstem response (ABR) (also known as automated auditory brainstem response [A-ABR] or brainstem auditory evoked potential [BAEP]), 12-lead ECGs, review of maternal and subject demographic data, review of subject's medical history, review of inclusion and exclusion factor, review of concomitant medication of subjects, assessment of vital signs, physical examination, including weight, length, head circumference, and eyes, dermatological examination, an Amiel-Tison neurologic examination, blood sampling for the following analyses: clinical chemistry, hematology (including blood smear), pharmacokinetics, and combinations thereof.

Some embodiments further comprise a continued evaluation of the subject before treatment, during treatment, after treatment or a combination thereof. In some embodiments, continued evaluation includes, but is not limited to, transcutaneous bilirubin (TcB) monitoring, an audiology examination including auditory brainstem response (ABR) (also known as automated auditory brainstem response [A-ABR] or brainstem auditory evoked potential [BAEP]), Three 12-lead ECGs, review of maternal and subject demographic data, review of subject's medical history, review of inclusion and exclusion factor, review of concomitant medication of subjects, assessment of vital signs, physical examination, including weight, length, head circumference, and eyes, dermatological examination, an Amiel-Tison neurologic examination, blood sampling for the following analyses: clinical chemistry, hematology (including blood smear), pharmacokinetics, and combinations thereof.

In some embodiments, vital signs comprise measuring temperature (axillary), blood pressure (measured with age- and size-appropriate equipment), pulse rate, respiratory rate and combinations thereof.

In some embodiments, physical examinations comprise an examination of the subjects general appearance, subjects weight, length, head (including head circumference), ears, eyes (including red reflex and pupillary reflex) nose, mouth, throat, neck, respiratory system (pulmonary/chest), cardiovascular system, abdomen, musculoskeletal (spine/reflexes), extremities, skin, lymph nodes, neurological system, genitourinary system and combinations thereof.

In some embodiments, dermatological examinations comprise the identification of photosensitive reactions if any. Photosensitive reaction may occur with metalloporphyrins and broad spectrum light. Photosensitive reactions include skin rashes.

In some embodiments audiology examinations comprise tests to discriminate peripheral (i.e., cochlear) from central (i.e., brainstem) auditory function, ABR (also known as A-ABR or BAEP) allowing for the detection of various failure patterns and information of auditory function in neonates, physiologic tests (tympanometry and acoustic reflex thresholds), behavioral measures (pure-tone and speech audiometry) and combinations thereof.

In some embodiments, ophthalmological examinations comprise monitoring the subject for any signs of lens or retinal phototoxicity, inspection of both the anterior (cornea and lens) and posterior (retina) segments of the eye for abnormalities and combinations thereof.

In some embodiments, neurological examinations comprise neurological and developmental evaluations of the subject, measuring tone, reflexes, and sensory responses, an Amiel-Tison neurologic examination and combinations thereof.

In some embodiments, laboratory tests comprise hematology, clinical chemistry and combination thereof. In some embodiments, clinical and hematological parameters include but are not limited to: complete blood count with differential, electrolytes (na+, k+, and cl-), glucose, protein, albumin, calcium, carbon dioxide, creatinine, blood urea nitrogen, total and direct serum bilirubin, alkaline phosphatase (alp), alanine aminotransferase (alt), aspartate aminotransferase (ast), gamma-glutamyltransferase (ggt) and combinations thereof.

In some embodiments, 12-Lead ECGs measurements comprise clarification of a cardiovascular event, measurement of cardiac intervals and morphological assessment, measurements of the RR, PR, QRS, and QT interval durations and combinations thereof. In some embodiments, Bazett's correction of the QT interval (QTcB), Fridericia's correction of the QT interval (QTcF), and heart rate (HR) can be derived froml2-Lead ECGs measurements.

The compounds can be administered in the conventional manner by any route where they are active. Administration can be systemic, topical, or oral. For example, administration can be, but is not limited to, parenteral, subcutaneous, intravenous, intramuscular, intraperitoneal, transdermal, oral, buccal, or ocular routes, or intravaginally, by inhalation, by depot injections, or by implants. Thus, modes of administration for the compounds of the present invention (either alone or in combination with other pharmaceuticals) can be, but are not limited to, sublingual, injectable (including short-acting, depot, implant and pellet forms injected subcutaneously or intramuscularly), or by use of vaginal creams, suppositories, pessaries, vaginal rings, rectal suppositories, intrauterine devices, and transdermal forms such as patches and creams.

Various metalloporphyrins show potential for the prevention and treatment of hyperbilirubinemia. Suitable metalloporphyrins for use herein are selected from a group consisting of metal mesoporphyrins, metal deuteroporphryins, metal hematoporphyrins, metal bisglycol derivates, metal protoporphyrins or salts thereof. In some embodiments, the metal may be selected from the group consisting of tin, iron, zinc, chromium, manganese, copper, nickel, magnesium, cobalt, platinum, vanadium, titanium, aluminum, gold, silver, arsenic, antimony, cadmium, gallium, germanium, and palladium. In some embodiments, the metalloporphryin may be selected from a group consisting of tin mesoporphyrin, zinc mesopoiphyrin, chromium mesoporphyrin, tin protoporphyrin, zinc protoporphyrin, chromium protoporphyrin, bisglycol protoporphyrin and fenoporphyrin. In some embodiments, the metalloporphyrin is tin IV mesoporphyrin IX dichloride (also called stannsoporfin or SnMP). The structure of tin IV mesoporphyrin IX dichloride is:

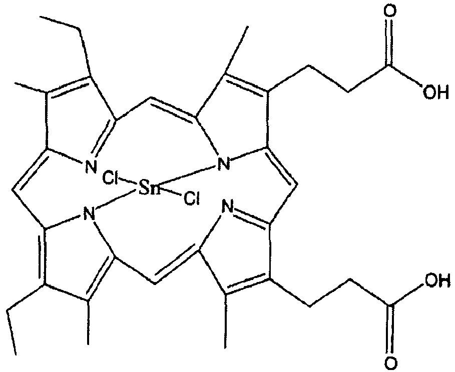

In some embodiments, the metalloporphyrin may be tin (IV) mesoporphyrin IX dichloride. As used herein, tin (IV) mesoporphyrin IX dichloride includes tin 4+ mesoporphyrin IX dichloride and stannsoporfin (SnMP). Tin (IV) mesoporphyrin IX dichloride can be obtained according to a variety of methods, for example, through the methods disclosed in U.S. Pat. No. 6,818,763, U.S. Pat. No. 7,375,216, or co-pending U.S. Application No. 11/867,559 filed on Oct. 4, 2007, which are incorporated herein by reference. However, it should be understood that other methods can be used to produce mesoporphyrin halides such as tin mesoporphyrin IX dichloride, and the present invention is not limited to a particular method of mesoporphyrin production.

In certain embodiments, the metalloporphyrin may be present in a substantially pure form in the pharmaceutical preparation. In some embodiments, the overall purity of the metalloporphyrin in the pharmaceutical preparation may be at least about 85%, at least about 90%, at least about 95%, at least about 97%, at least about 98%, at least about 98.5%, at least about 99%, or at least about 99.5%. In an embodiment, each individual product-related impurity in the pharmaceutical preparation may be in an amount of less than about 1%, less than about 0.5%, less than about 0.3%, or less than about 0.1% of the preparation. In an embodiment, any individual product-related impurity present is present in an amount of less than about 0.5%, less than about 0.3%, less than about 0.2%, less than about 0.15%, less than about 0.1%, less than about 0.09%, less than about 0.08%, or less than about 0.07% of the preparation.

The pharmaceutical preparation may be in unit dosage form. In such form, the preparation is subdivided into unit doses containing appropriate quantities of the active component. The unit dosage form can be a packaged preparation, the package containing discrete quantities of preparation, such as packaged in vials or ampules.

The quantity of active component in a unit dose preparation may be varied or adjusted from about 0.1 to about 50 mg, preferably 0.1 to about 40 mg, and more preferably 0.1 to about 20 mg according to the particular application and the potency of the active component and size of the patient. The composition can, if desired, also contain other compatible therapeutic agents.

In therapeutic use as agents for treating hyperbilirubinemia, the compounds utilized in the pharmaceutical methods of this invention are administered at the initial dosage of about 0.1 mg to about 20 mg per kilogram body weight (IM). In certain embodiments, treatment with the metalloporphyrin is a one-time single dose treatment. In some embodiments, the metalloporphyrin is administered in a dosage of from about 0.5 mg to about 6 mg per kilogram body weight (IM). In some embodiments, the metalloporphyrin is administered in a dosage of from about 0.5 mg/kg to about 4 mg/kg, from about 0.5 mg/kg to about 2 mg/kg, from about 0.75 mg/kg to about 1.5 mg/kg, from about 1.5 mg/kg to about 4.5 mg/kg or from about 3.0 mg/kg to about 4.5 mg/kg, including about 1.5 mg/kg, about 3.0 mg/kg and about 4.5 mg/kg. The dosages, however, may be varied depending upon the requirements of the patient, the severity of the condition being treated and the compound being employed. Determination of the proper dosage for a particular situation is within the skill of the art. In one embodiment, generally, treatment is initiated with smaller dosages, which are less than the optimum dose of the compound. Thereafter, the dosage is increased by small increments until the optimum effect under the circumstance is reached.

In one embodiment, a pharmaceutical composition may comprise the metalloporphyrin in an aqueous solution with a concentration from about 4.5 to about 40 mg/mL, and preferably from about 4.5 mg/mL to about 25 mg/mL. In an embodiment, the pharmaceutical composition may further comprise an acid, a base and a buffering agent mixed in an aqueous solution. The composition may be sterile and may have a physiological osmolality. The compositions or drug products may be packaged in amber glass vials.

The stannsoporfin used may be of pharmaceutically acceptable quality. In some embodiments, ultra high purity stannsoporfin may be used. In some such embodiments, the compound is at least 90% pure stannsoporfin, at least 95% stannsoporfin, at least 97% stannsoporfin, at least 98% stannsoporfin, at least 98.5% stannsoporfin, at least 99% stannsoporfin. Additionally, in some embodiments, any individual impurity is not more than 0.1% by weight of the composition.

In some embodiments, the pharmaceutical composition containing metalloporphyrin may be a component of a drug product, wherein the product is contained in a single dose unit. According to one embodiment, a single dose unit may include at least about 0.5 ml of solution, and more preferably, at least about 1 ml of solution.

The solution may be provided in a drug product form by containing the solution in a suitable container such as an ampule or vial. According to certain embodiments, the solution is stable and has a shelf life of at least about 3 months. In other embodiments, the solution has a shelf life of at least about 6 months.

In some embodiments, the composition may further comprise a buffer. There are numerous buffers, which may be suitable for creating the pharmaceutical composition. Examples of such buffers include: an alkali earth metal buffering agent, a calcium buffering agent, a magnesium buffering agent, an aluminum buffering agent, sodium bicarbonate, potassium bicarbonate, magnesium hydroxide, magnesium lactate, magnesium gluconate, magnesium oxide, magnesium aluminate, magnesium carbonate, magnesium silicate, magnesium citrate, aluminum hydroxide, aluminum hydroxide/magnesium carbonate, aluminum hydroxide/sodium bicarbonate coprecipitate, aluminum glycinate, aluminum magnesium hydroxide, aluminum phosphate, sodium citrate, calcium citrate, sodium tartrate, sodium acetate, sodium carbonate, sodium polyphosphate, sodium dihydrogen phosphate, potassium pyrophosphate, sodium polyphosphate, potassium pyrophosphate, disodium hydrogenphosphate, tribasic sodium phosphate dodecahydrate, dipotassium hydrogen phosphate, trisodium phosphate, tripotassium phosphate, potassium carbonate, potassium metaphosphate, calcium acetate, calcium glycerophosphate, calcium chloride, calcium hydroxide, calcium lactate, calcium carbonate, calcium gluconate, calcium bicarbonate, sodium phosphate, potassium phosphate, calcium phosphate, magnesium phosphate, potassium citrate, trihydroxymethylaminomethane, an amino acid, an acid salt of an amino acid, and an alkali salt of an amino acid, and combinations of the foregoing. The buffer used should be able to be used in a concentration effective to raise the pH of the solution to about 10 or above, when base is added to the solution. In addition, the buffer must be pharmaceutically acceptable.

For example, in some aspects, a pharmaceutical composition suitable for use in the methods described herein comprises a compound, as defined above, and a pharmaceutically acceptable carrier or diluent, or an effective amount of a pharmaceutical composition comprising a compound as defined above.

The compounds may be administered in the conventional manner by any route where they are active. Administration can be systemic, topical, or oral. For example, administration can be, but is not limited to, parenteral, subcutaneous, intravenous, intramuscular, intraperitoneal, transdermal, oral, buccal, or ocular routes, or intravaginally, by inhalation, by depot injections, or by implants. Thus, modes of administration for the compounds of the present invention (either alone or in combination with other pharmaceuticals) can be, but are not limited to, sublingual, injectable (including short-acting, depot, implant and pellet forms injected subcutaneously or intramuscularly), or by use of vaginal creams, suppositories, pessaries, vaginal rings, rectal suppositories, intrauterine devices, and transdermal forms such as patches and creams. In particular, intramuscular (IM) injections have been used with success.

Specific modes of administration will depend on the indication. The selection of the specific route of administration and the dose regimen is to be adjusted or titrated by the clinician according to methods known to the clinician in order to obtain the optimal clinical response. The amount of compound to be administered is that amount which is therapeutic. The dosage to be administered will depend on the characteristics of the subject being treated, e.g., the particular animal treated, age, weight, health, types of concurrent treatment, if any, and frequency of treatments, and can be easily determined by one of skill in the art (e.g., by the clinician).

Pharmaceutical formulations containing the compounds of the present invention and a suitable carrier can be solid dosage forms which include, but are not limited to, tablets, capsules, cachets, pellets, pills, powders and granules; topical dosage forms which include, but are not limited to, solutions, powders, fluid emulsions, fluid suspensions, semi¬ solids, ointments, pastes, creams, gels and jellies, and foams; and parenteral dosage forms which include, but are not limited to, solutions, suspensions, emulsions, and dry powder; comprising an effective amount of a polymer or copolymer of the present invention. It is also known in the art that the active ingredients can be contained in such formulations with pharmaceutically acceptable diluents, fillers, disintegrants, binders, lubricants, surfactants, hydrophobic vehicles, water-soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

The compounds of the present invention can be formulated for parenteral administration by injection, e.g., by bolus injection or continuous infusion. The compounds can be administered by continuous infusion subcutaneously over a period of about 15 minutes to about 24 hours. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, the compounds can be formulated readily by combining these compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include, but are not limited to, push-fit capsules made of gelatin, as well as soft, sealed capsules made of gelatin and a plasticizer, such as glycerol or sorbitol. The push-fit capsules can contain the active ingredients in admixture with filler such as, e.g., lactose, binders such as, e.g., starches, and/or lubricants such as, e.g., talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of, e.g., tablets or lozenges formulated in a conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds of the present invention can also be formulated in rectal compositions such as suppositories or retention enemas, e.g., containing conventional suppository bases such as cocoa butter or other glycerides.

In addition to the formulations described previously, the compounds of the present invention can also be formulated as a depot preparation. Such long acting formulations can be administered by implantation (for example subcutaneously or intramuscularly) or by intramuscular injection.

Depot injections can be administered at about 1 to about 6 months or longer intervals. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

In transdermal administration, the compounds of the present invention, for example, can be applied to a plaster, or can be applied by transdermal, therapeutic systems that are consequently supplied to the organism.

Pharmaceutical compositions of the compounds also can comprise suitable solid or gel phase carriers or excipients. Examples of such carriers or excipients include but are not limited to calcium carbonate, calcium phosphate, various sugars, starches, cellulose derivatives, gelatin, and polymers such as, e.g., polyethylene glycols.

The compounds of the present invention can also be administered in combination with other active ingredients, such as, for example, adjuvants, protease inhibitors, or other compatible drugs or compounds where such combination is seen to be desirable or advantageous in achieving the desired effects of the methods described herein.

### Administration of Phototherapy

In some embodiments, phototherapy (PT) is administered to a subject. In some embodiments, subjects begin PT if total serum bilirubin levels meet the AAP Guidelines for starting PT after administration of a metalloporphyrin. In some embodiments, PT may be stopped after 1 declining total serum bilirubin assessment that is at least 2 mg/dL below the threshold for PT (as determined by the age of the subject when the blood was collected). In some embodiments, neoBLUE lights and neoBLUE cozy (Natus Medical Incorporated, San Carlos, CA) may be used for PT after or in combination with administration of metalloporphyrin.

Some embodiments are directed to the use of a metalloporphyrin in the manufacture of a medicament for the treatment of hyperbilirubinemia or the symptoms thereof in an infant comprising: administering a therapeutic amount of a metalloporphyrin to the infant with hyperbilirubinemia where no exclusion factor is present and at least one of a baseline total bilirubin level is elevated above a predetermined threshold and at least one risk factor is present; wherein the hyperbilirubinemia or symptoms thereof is treated.

Some embodiments further comprise determining baseline total bilirubin levels in the infant. In some embodiments, baseline total bilirubin levels comprises total serum bilirubin levels, total cutaneous bilirubin or a combination thereof.

In some embodiments, the infant is of a gestational age from about 35 to about 43 weeks. In some embodiments, the infant has a minimum birth weight of about 2,500 g. In some embodiments, the infant has a birth weight from about 1,700 g to about 4,000 g.

In some embodiments, the infant is Coombs positive. In some embodiments, the infant is Coombs negative and at least one risk factor is present. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.

In some embodiments, determining baseline total bilirubin levels is performed at a time selected from within 6 hours of birth, 12 hours of birth, within 24 hours of birth, and within 48 hours of birth.

Some embodiments further comprise identifying the presence of at least one risk factor. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, and G6PD deficiency and combinations thereof.

Some embodiments further comprise identifying the absence of at least one exclusion factor. In some embodiments, the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is >5.0 mg/dL), persistent hypoglycemia (blood glucose <40 mg/dL) despite standard-of-care treatment, liver diseases defined as ALT and/or AST greater than 2 times the upper limit of normal [ULN], abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, the predetermined threshold is the level determined by the AAP nomogram for initiating phototherapy for an infant of known age and known risk level. In some embodiments, the predetermined threshold is selected from about 1-3 mg/dl below a threshold for administration of phototherapy to an infant up to about 12 hours of age per the AAP guidelines, about 1 mg/dl below a threshold for administration of phototherapy to an infant up to about 12 hours of age per the AAP guidelines, about 2 mg/dl below a threshold for administration of phototherapy to an infant up to about 12 hours of age per the AAP guidelines, at the threshold for administration of phototherapy to an infant up to about 12 hours of age per the AAP guidelines, about 1-3mg/dl below a threshold for administration of phototherapy to an infant from about 12 to 48 hours of age per the AAP guidelines, is about 2 mg/dl below a threshold for administration of phototherapy to an infant from about 12 to 48 hours of age per the AAP guidelines, about 3 mg/dl below a threshold for administration of phototherapy to an infant from about 12 to 48 hours of age per the AAP guidelines, at the threshold for administration of phototherapy to an infant from about 12 to 48 hours of age per the AAP guidelines and about 1 to about 3 mg/dl below the threshold for administration of phototherapy according to AAP nomogram corresponding to the infants age.

In some embodiments, administering a therapeutic amount of a metalloporphyrin is performed at a time selected from within about 6 hours of birth, within about 12 hours of birth, within about 24 hours of birth and within about 48 hours of birth.

In some embodiments, the metalloporphyrin is selected from tin mesoporphyrin, zinc mesoporphyrin, chromium mesoporphyrin, tin protoporphyrin, zinc protoporphyrin, chromium protoporphyrin, bisglycol protoporphyrin and ferroporphyrin.

In some embodiments, the metalloporphyrin is tin mesoporphyrin. In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, 1.5 mg/kg, 3.0 mg/kg and 4.5 mg/kg of the infant's weight. In some embodiments, the therapeutic amount of the tin mesoporphyrin is from about 0.75 mg/kg to about 5 mg/kg of the infant's weight. In some embodiments, the therapeutic amount of the tin mesoporphyrin is selected from 0.75 mg/kg, 1.5 mg/kg, 3.0 mg/kg and 4.5 mg/kg of the infant's weight.

In some embodiments, the metalloporphyrin is administered by intramuscular injection.

In some embodiments, administering a therapeutic amount of a metalloporphyrin is performed when the infant's age is less than 20 days of age. In some embodiments, administering a therapeutic amount of a metalloporphyrin is performed when the infant's age is less than 30 days of age.

Some embodiments further comprise administering phototherapy where total bilirubin levels following administration of the metalloporphyrin are above the baseline total bilirubin levels.

Some embodiments further comprise determining post treatment total bilirubin levels following administration of the metalloporphyrin. In some embodiments, determining post treatment total bilirubin levels following administration of the metalloporphyrin is performed from about 6 and to about 72 hours after administering the metalloporphyrin to the infant. In some embodiments, post treatment total bilirubin levels are at least 5% below the baseline total bilirubin levels 24 hours after administering a therapeutic amount of a metalloporphyrin to the infant. In some embodiments, post treatment total bilirubin levels are at least 10% below the baseline total bilirubin levels 48 hours after administering a therapeutic amount of a metalloporphyrin to the infant. In some embodiments, post treatment total bilirubin levels are at least 20% below the baseline total bilirubin levels 72 hours after administering a therapeutic amount of a metalloporphyrin to the infant. In some embodiments, post treatment total bilirubin levels are less than 3mg/dl above the baseline total bilirubin levels 48 hours after administering a therapeutic amount of a metalloporphyrin to the infant.

Some embodiments further comprise conducting on the infant an exam selected from a physical exam, a dermatologic exam, an audiology exam, an ophthalmological exam, a neurological exam, a laboratory test, an electrocardiogram and a combination thereof.

Some embodiments are directed to the use of a metalloporphyrin in the manufacture of a medicament to reduce the likelihood of hyperbilirubinemia and the symptoms thereof in an infant, comprising: administering a therapeutic amount of a metalloporphyrin to the infant where the infant's total bilirubin is determined to be increasing in at least one total bilirubin measurement compared with a baseline total bilirubin level wherein the likelihood of hyperbilirubinemia or the symptoms thereof is decreased.

In some embodiments, where the infant's total bilirubin is determined to be increasing in two consecutive total bilirubin measurements.

In some embodiments, the baseline total bilirubin measurement is performed from about 6 to about 96 hours of age. In some embodiments, the baseline total bilirubin measurement is performed at about 6, 12, 24, 48, 72, or 96 hours of age. In some embodiments, the at least one total bilirubin measurement is performed from about 6 to about 72 hours after the baseline total bilirubin measurement.

In some embodiments, administering a therapeutic amount of a metalloporphyrin is performed within about 1 to about 6 hours of when the infant's total bilirubin is determined to be increasing in at least one total bilirubin measurement.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, 1.5 mg/kg, 3.0 mg/kg and 4.5 mg/kg of the infant's weight. In some embodiments, the therapeutic amount of the stannsoporfin is from about 0.75 mg/kg to about 5 mg/kg of the infant's weight. In some embodiments, the therapeutic amount of the stannsoporfin is selected from 0.75 mg/kg, 1.5 mg/kg, 3.0 mg/kg and 4.5 mg/kg of the infant's weight.

Some embodiments are directed to the use of a metalloporphyrin in the manufacture of a medicament for the treatment of hyperbilirubinemia and the symptoms thereof in an infant, comprising: administering a therapeutic amount of a metalloporphyrin to the infant; and administering a therapeutic amount of phototherapy to the infant wherein the hyperbilirubinemia or symptoms thereof is treated.

Some embodiments further comprise determining baseline total bilirubin levels. In some embodiments, determining baseline total bilirubin levels is performed within 48 hours of birth.

Some embodiments further comprise identifying the presence of at least one risk factor prior to administering a therapeutic amount of the metalloporphyrin to the infant. In some embodiments, the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

Some embodiments further comprise identifying the presence of at least one exclusion factor prior to administering a therapeutic amount of the metalloporphyrin to the infant. In some embodiments, the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is ≥5.0 mg/dL), persistent hypoglycemia (blood glucose <40 mg/dL) despite standard-of-care treatment, liver diseases defined as ALT and/or AST greater than 2 times the upper limit of normal [ULN], abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.

In some embodiments, administering a therapeutic amount of a metalloporphyrin and administering a therapeutic amount of phototherapy is performed where no exclusion factor is present.

In some embodiments, administering a therapeutic amount of a metalloporphyrin and administering a therapeutic amount of phototherapy is performed where at least one of a baseline total bilirubin level elevated above a predetermined threshold and at least one risk factor, or a combination thereof is present.

In some embodiments, the predetermined threshold is selected from about 1-3 mg/dl below a threshold for administration of phototherapy to an infant up to about 12 hours of age per the AAP guidelines, about 1 mg/dl below a threshold for administration of phototherapy to an infant up to about 12 hours of age per the AAP guidelines, about 2 mg/dl below a threshold for administration of phototherapy to an infant up to about 12 hours of age per the AAP guidelines, at the threshold for administration of phototherapy to an infant up to about 12 hours of age per the AAP guidelines, about 1-3mg/dl below a threshold for administration of phototherapy to an infant from about 12 to 48 hours of age per the AAP guidelines, about 2 mg/dl below a threshold for administration of phototherapy to an infant from about 12 to 48 hours of age per the AAP guidelines, about 3 mg/dl below a threshold for administration of phototherapy to an infant from about 12 to 48 hours of age per the AAP guidelines, at the threshold for administration of phototherapy to an infant from about 12 to 48 hours of age per the AAP guidelines, and about 1 to about 3 mg/dl below the threshold for administration of phototherapy according to AAP nomogram corresponding to the infants age.

In some embodiments, the therapeutic amount of the metalloporphyrin is from about 0.75 mg/kg to about 5 mg/kg of the infant's weight. In some embodiments, the therapeutic amount of the metalloporphyrin is selected from 0.75 mg/kg, 1.5 mg/kg, 3.0 mg/kg and 4.5 mg/kg of the infant's weight. In some embodiments, the therapeutic amount of the stannsoporfin is from about 0.75 mg/kg to about 5 mg/kg of the infant's weight. In some embodiments, the therapeutic amount of the stannsoporfin is selected from 0.75 mg/kg, 1.5 mg/kg, 3.0 mg/kg and 4.5 mg/kg of the infant's weight.

In some embodiments, administering a therapeutic amount of a metalloporphyrin is performed in the infant is performed when the infants age is less than about 48 hours. In some embodiments, administering a therapeutic amount of a metalloporphyrin is performed in the infant is performed when the infants age is less than about 20 days of age. In some embodiments, administering a therapeutic amount of a metalloporphyrin is performed in the infant is performed when the infants age is less than about 30 days of age.

In some embodiments, administering a therapeutic amount of a metalloporphyrin and phototherapy is performed simultaneously. In some embodiments, phototherapy is performed at a time selected from within about 12 hours of administration of therapeutic amount of a metalloporphyrin and within about 24 hours of administration of therapeutic amount of a metalloporphyrin.

Some embodiments further comprise conducting on the infant, a physical exam selected from, a dermatologic exam, an audiology exam, an ophthalmological exam, a neurological exam, a laboratory test, an electrocardiogram and a combination thereof.

Some embodiments are directed to the use of a metalloporphyrin in the manufacture of a medicament for reducing the risk of hyperbilirubinemia and the symptoms thereof in an infant, comprising administering a therapeutic amount of a metalloporphyrin to the infant wherein the infant has at least one risk factor associated with hyperbilirubinemia.

In some embodiments, the infant has a total bilirubin level of less than about 3 mg/dl below the threshold for administration of phototherapy according to AAP nomogram corresponding to the infant's age.

In some embodiments, administering a therapeutic amount of a metalloporphyrin to the infant comprises administering a single dose of a metalloporphyrin.

In some embodiments, the least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the risk factor is a total bilirubin level at or above a pre-determined threshold. In some embodiments, the predetermined threshold is selected from about 1-3 mg/dl below a threshold for administration of phototherapy to an infant up to about 12 hours of age per the AAP guidelines, about 1 mg/dl below a threshold for administration of phototherapy to an infant up to about 12 hours of age per the AAP guidelines, about 2 mg/dl below a threshold for administration of phototherapy to an infant up to about 12 hours of age per the AAP guidelines, is at the threshold for administration of phototherapy to an infant up to about 12 hours of age per the AAP guidelines, about 1-3mg/dl below a threshold for administration of phototherapy to an infant from about 12 to 48 hours of age per the AAP guidelines, about 1-3mg/dl below a threshold for administration of phototherapy to an infant from about 12 to 48 hours of age per the AAP guidelines, about 2 mg/dl below a threshold for administration of phototherapy to an infant from about 12 to 48 hours of age per the AAP guidelines, about 3 mg/dl below a threshold for ailministration of phototherapy to an infant from about 12 to 48 hours of age per the AAP guidelines, at the threshold for administration of phototherapy to an infant from about 12 to 48 hours of age per the AAP guidelines, about 1 to about 3 mg/dl below the threshold for administration of phototherapy according to AAP nomogram corresponding to the infants age and at the threshold for administration of phototherapy to an infant from about 12 to 48 hours of age per the AAP guidelines.

In some embodiments, administering a therapeutic amount of the metalloporphyrin to the infant results in at least one of a decrease in total bilirubin levels compared with total bilirubin levels prior to administering the metalloporphyrin and no detectable increase in total bilirubin levels compared with total bilirubin levels prior to administering the metalloporphyrin.

In some embodiments, the therapeutic amount of the metalloporphyrin is from about 0.75 mg/kg to about 5 mg/kg of infant's weight. In some embodiments, the therapeutic amount of the metalloporphyrin is selected from 0.75 mg/kg, 1.5 mg/kg, 3.0 mg/kg and 4.5 mg/kg of infant's weight. In some embodiments, the therapeutic amount of the stannsoporfin is from about 0.75 mg/kg to about 5 mg/kg of infant's weight. In some embodiments, the therapeutic amount of the stannsoporfin is selected from 0.75 mg/kg, 1.5 mg/kg, 3.0 mg/kg and 4.5 mg/kg of infant's weight.

Some embodiments are directed to the use of a metalloporphyrin in the manufacture of a medicament for stabilizing bilirubin levels in an infant, comprising: obtaining a baseline total bilirubin level measurement; and administering a therapeutic amount of a metalloporphyrin to the infant wherein the infant has at least one of hyperbilirubinemia, bilirubin levels above a pre-determined threshold, rising bilirubin levels, and a combination thereof wherein bilirubin levels in the infant are stabilized.

In some embodiments, administering a therapeutic amount of a metalloporphyrin to the infant comprises administering a single dose of a metalloporphyrin.

In some embodiments, the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.

In some embodiments, the infant is of a gestational age from about 35 to about 43 weeks. In some embodiments, the infant has a minimum birth weight of about 2500 g. In some embodiments, the infant has a birth weight from about 1,700 g to about 4,000 g.

In some embodiments, stabilization of total bilirubin levels is achieved when at least two total bilirubin level measurements taken at pre-determined time points after administration of a single therapeutic amount of a metalloporphyrin indicate a total bilirubin level at or below the baseline total bilirubin level.

In some embodiments, the predetermined threshold is about 1-3 mg/dl below a threshold for administration of phototherapy to an infant up to about 12 hours of age per the AAP guidelines, about 1 mg/dl below a threshold for administration of phototherapy to an infant up to about 12 hours of age per the AAP guidelines, about 2 mg/dl below a threshold for administration of phototherapy to an infant up to about 12 hours of age per the AAP guidelines, is at the threshold for administration of phototherapy to an infant up to about 12 hours of age per the AAP guidelines, about 1-3mg/dl below a threshold for administration of phototherapy to an infant from about 12 to 48 hours of age per the AAP guidelines, about 1-3mg/dl below a threshold for administration of phototherapy to an infant from about 12 to 48 hours of age per the AAP guidelines, about 2 mg/dl below a threshold for administration of phototherapy to an infant from about 12 to 48 hours of age per the AAP guidelines, about 3 mg/dl below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dl below the threshold for administration of phototherapy according to AAP nomogram corresponding to the infants age and at the threshold for administration of phototherapy to an infant from about 12 to 48 hours of age per the AAP guidelines.

In some embodiments, the therapeutic amount of the metalloporphyrin is from about 0.75 mg/kg to about 5 mg/kg. In some embodiments, the therapeutic amount of the metalloporphyrin is selected from 0.75 mg/kg, 1.5 mg/kg, 3.0 mg/kg and 4.5 mg/kg. In some embodiments, the therapeutic amount of the stannsoporfin is from about 0.75 mg/kg to about 5 mg/kg. In some embodiments, the therapeutic amount of the stannsoporfin is selected from 0.75 mg/kg, 1.5 mg/kg, 3.0 mg/kg and 4.5 mg/kg.

Some embodiments are directed to the use of a metalloporphyrin in the manufacture of a medicament for the treatment of rising bilirubin levels comprising: establishing a baseline bilirubin level in a patient at risk for hyperbilirubinemia at a predetermined age; administering to the patient a therapeutic amount of stannsoporfin after the baseline is established. In some embodiments, the predetermined age is about 6 hours, about 12 hours, or about 24 hours from birth. In some embodiments, a baseline reading at the AAP nomogram threshold for administering phototherapy or up to 3.0mg/dL below the AAP nomogram threshold for ao!ministering phototherapy indicates treatment is required.

Some embodiments are directed to the use of a metalloporphyrin in the manufacture of a medicament for the treatment hyperbilirubinemia comprising: administering a therapeutic amount of stannsoporfin to a patient in need thereof to achieve a Cmax of at least 5000ng/mL. In some embodiments, the therapeutic amount of stannsoporfin is 1.5mg/kg and achieves a Cmax of about 6450 ng/mL. In some embodiments, the therapeutic amount of stannsoporfin is 3.0 mg/kg and achieves a Cmax of about 11500 ng/mL. In some embodiments, the therapeutic amount of stannsoporfin is 4.5 mg/kg and achieves a Cmax of about 20400 ng/mL. In some embodiments, Cmax is achieved at a Tmax of about 1.5 hours to about 2.5 hours.

This invention and embodiments illustrating the method and materials used may be further understood by reference to the following non-limiting examples.

### G. EXAMPLES

Phase 2b, Multicenter, Single-dose, Blinded, Randomized, Placebo-controlled, Dose-escalation, Safety and Efficacy Trial of Stannsoporfin in Neonates with Hyperbilirubinemia

### Study Design and Duration of Treatment

This was a Phase 2b, multicenter, single-dose, blinded, randomized, placebo controlled, dose-escalation, safety and efficacy study of stannsoporfin in term and late preterm subjects with hyperbilirubinemia.

The clinical study consisted of 4 phases: determination of eligibility, screening procedures, treatment, and post-treatment assessments, including 2 follow-up visits (Visits 3 and 4) at 14 and 30 days after treatment.

Three cohorts of subjects were recruited. This clinical study was designed to evaluate safety and efficacy parameters in each cohort. The first cohort of subjects received stannsoporfin at a single dose of 1.5 mg/kg by intramuscular (IM) injection or a placebo IM injection of saline solution. Treatment in Cohort 2 did not begin until a review of safety data from Cohort 1 was conducted by the Data Safety Monitoring Board (DSMB). Since the safety profile of Cohort 1 was acceptable, Cohort 2 received a single injection of stannsoporfin at a dose of 3.0 mg/kg IM or a placebo injection of saline solution. Once the DSMB determined the safety profile from Cohort 2 was acceptable, subjects in Cohort 3 received a single injection of stannsoporfin at a dose of 4.5 mg/kg IM or a placebo injection of saline solution.

Twenty-three study centers participated in this study: 9 in the US and 14 in Europe (5 in Ukraine, 6 in Spain, and 3 in Poland). The duration of the study included both in-hospital (24 to 48 hours) and discharge/follow-up (30-day) periods. A subsequent protocol allowed infants to be enrolled into a long-term follow up study (Study 64,185-203).

The primary objective of the study was to determine the safety of 3 ascending doses of stannsoporfin in subjects with hyperbilirubinemia. Secondary objectives were to determine the efficacy of 3 ascending doses of stannsoporfin in subjects with hyperbilirubinemia, determine the pharmacokinetics (PK) of 3 ascending doses of stannsoporfin in subjects with hyperbilirubinemia and an exploratory pharmacodynamic analyses could also be performed.

### Number of Subjects

Approximately 72 subjects were to be enrolled into 3 cohorts (1.5, 3.0, or 4.5 mg/kg stannsoporfin) at 20 to 30 clinical sites, globally, to achieve 24 subjects in each cohort. Each cohort was to include 6 subjects randomly assigned to a control group receiving placebo and 18 subjects randomly assigned to a treatment group receiving stannsoporfin. Enrollment of 72 subjects was expected to yield 64 evaluable subjects. The number of subjects actually enrolled was 63 subjects rather than 72 because the study was discontinued before enrollment of the full 4.5 mg/kg cohort. Seventeen subjects received 1.5 mg/kg stannsoporfm, 18 subjects received 3.0 mg/kg stannsoporfm, 8 subjects received 4.5 mg/kg stannsoporfm, 15 subjects received placebo, and 5 subjects were randomized but not treated.

### Diagnosis and Main entry Criteria

Term and late preterm subjects (≥35 weeks and <43 weeks gestational age) up to 48 hours of age with hyperbilirubinemia and risk factors for hemolytic disease, including subjects with Coombs positive ABO blood type incompatibility, Rhesus (Rh) incompatibility (anti-C, c, D, E, or e), or G6PD deficiency, with a minimum birth weight of 2500 g (5.5 lbs.). After consent was obtained, subjects were to be followed until their serum bilirubin level was within a window of 1 mg/dL below the threshold for phototherapy (PT) per the American Academy of Pediatrics (AAP) Guidelines at up to 12 hours of age or within 2 mg/dL below the threshold for PT at ≥12 to 48 hours of age (inclusive), at which time subjects were eligible for randomization, provided screening criteria were met. This inclusion criterion window was amended to 2 mg/dL below the threshold at up to 12 hours of age and 3 mg/dL below from 12 to 48 hours of age during the first cohort. A new amendment reverted to the original criteria of 1 mg/dL below the threshold for PT per the AAP Guidelines at up to 12 hours of age or within 2 mg/dL below the threshold for PT at ≥I2 to 48 hours of age (inclusive) midway through Cohort 2. Treatment with the investigational medicinal product (IMP) was not to be initiated until the total serum bilirubin (TSB) level was within the window described and all other entry criteria were met.

Subjects with a positive direct Coombs test were categorized as high or medium risk according to the AAP Guidelines for PT. Per a protocol amendment, ABO- or Rh-incompatible subjects with a negative direct Coombs test could be entered into the clinical study if they had at least 1 additional risk factor as defined by the AAP Guidelines.

### Exclusion factor

Key exclusion factor selected in this study also serve to homogenize the study population and assure patient safety by excluding clinically important comorbidities, exposure to photosensitizing medications or those known or suspected to displace bilirubin from albumin, and anyone at risk of exposure to surgical lights. In addition, subjects whose mothers used phenobarbital 30 days before or after delivery, if breastfeeding, were excluded since phenobarbital increases the conjugation and excretion of bilirubin.

### Dose and Mode of Administration

The Test Treatment was a single dose of stannsoporfin via IM injection of 1.5 mg/kg (Cohort 1), 3.0 mg/kg (Cohort 2), or 4.5 mg/kg (Cohort 3). The Placebo Control Treatment was a single saline solution IM injection matching the volume of the stannsoporfin dose.

### Schedule of Assessments

Parents/guardians of neonates who had suspected isoimmune hemolytic disease were approached for consent into the clinical study. Once consent was obtained, subjects were observed for clinical signs of jaundice and had transcutaneous bilirubin (TcB) or TSB levels completed. TcB measurements could be used to help track subjects' bilirubin levels. If subjects met inclusion criteria, screening procedures were completed, and subjects could be enrolled into the clinical study. If subjects did not yet meet the criteria, TcB or TSB levels could be repeated, until up to 48 hours of age, as clinically indicated.

Scheduled assessments (including TSB) occurred before treatment and at 0.75 and 2 hours (or 1.5 and 3 hours, depending on PK blood sampling assignment); 6, 12, 24, 48, and 72 hours; and 14 and 30 (except TSB) days after treatment or early termination.

PT was standardized for this study. TSB levels were initially assessed at 6 hours after IM injection to determine if PT was necessary. If the subject met the AAP Guidelines for starting PT, PT was initiated, and if the criteria were not met at the time, the subjects continued in the study until the next TSB level assessment (at 12, 24, and 48 hours following IMP injection). If, at any of these time points, the subject met the criteria for initiation of PT per AAP Guidelines, then PT was started.

In subjects receiving PT, determinations to assess whether or not PT was still required were done every 6 hours (±15 min); if the TSB was declining over 2 of these 6 hour assessments, TSB assessments could be done every 12 hours (±15 min). PT was stopped after 1 declining TSB assessment had been reviewed and the TSB level had declined to at least 2 mg/dL below the threshold for PT (as determined by the age of the subject when the blood was collected). As part of the clinical management of the subject, once PT was stopped, a TSB assessment was performed at approximately 6 hours later to assure there was no rebound in the bilirubin level. If the TSB rebounded above the threshold for PT, further clinical management was at the discretion of the principal investigator. After completion of Visit 4 (Day 30), parents/guardians were asked to enroll the completed subjects into a long term safety follow-up study.

### Efficacy Measurements

The primary efficacy endpoint was the change in adjusted TSB from baseline to 48 hours after treatment. The adjusted TSB was a calculation of the percentage variance of the TSB level from the age specific threshold for PT initiation per the AAP Guidelines, i.e, an indication of the distance below the PT threshold by time.

The Secondary Efficacy Endpoints were the change from baseline in unadjusted TSB at 48 hours after treatment, change from baseline in adjusted and unadjusted TSB at various other time points after treatment, percent change from baseline in unadjusted TSB, proportion of subjects who require PT/exchange transfusion (ET), time to PT/ET, duration of PT and time to hospital discharge.

Blood samples were taken at prespecified time points following treatment to evaluate the PK of stannsoporfin at the 3 doses being studied.

### Safety Measurements

Safety measurements included adverse events (AEs), hematology and chemistry laboratory tests, vital sign measurements, physical examinations, dermatological assessments, hearing assessments, ophthalmological assessments, neurological assessments, and quantitative electrocardiograms (ECGs). The relationship between the plasma concentration of stannsoporfin and the changes in the QTc interval over time was analyzed.

### Statistical Methods

Several Analysis Populations in the study were defined:
Intent-to-treat population (ITT): Defined as all subjects who were randomly assigned treatment in the clinical study, had received IMP, and had at least 1 post baseline TSB measurement during the first 48 hours after treatment. Subjects were summarized based on randomized treatment.

Per-protocol (PP) population: Defined as all subjects who were in the ITT population, completed the clinical study, and did not have any major protocol violations during the clinical study.

Safety population: Defined as all subjects who were enrolled in the clinical study and received IMP. Subjects were summarized according to the treatment received.

The primary efficacy analyses were conducted on the ITT population. Additional supportive efficacy analyses were conducted on the PP population.

Descriptive statistics were used to summarize all efficacy and safety outcomes. Unless otherwise specified, all statistical analyses were 2 tailed with alpha=0.05.

Analysis of Efficacy Parameters: The primary efficacy analysis applied the analysis of covariance (ANCOVA) method to determine if there was a significant difference between treatment groups in change in adjusted TSB levels from baseline to 48 hours post baseline after controlling for the effects of gestational age and adjusted baseline TSB levels. If assumptions of ANCOVA appeared to be violated, a Wilcoxon rank-sum test could be performed instead. Time-to-event variables were analyzed using the Kaplan- Meier method, and Kaplan-Meier survival curves were presented.

Pharmacokinetic Assessments: Subjects were stratified into 2 groups having different sampling times to better characterize the PK curves. Modeling from the samples taken from all subjects allowed calculations of PK parameters, including time to reach maximum concentration, maximum concentration (Cmax), terminal half-life, and area under the plasma concentration versus time curve (AUC) in all 3 active treatment groups.

Analysis of Safety Parameters: Descriptive statistical methods were used to summarize safety parameters based on the safety population. All treatment-emergent adverse events (TEAEs) were summarized. Tabular summaries were also presented for all TEAEs considered potentially related to the IMP, TEAEs by intensity, serious adverse events (SAEs), and AEs leading to discontinuation.

All other safety endpoints (vital sign measurements, hematology and chemistry laboratory tests, physical examination results, ECG results, etc.) were summarized by treatment group and visit. In addition, shift tables were generated for laboratory results comparing postbaseline results with baseline. Similar shift tables were generated for results of vital sign measurements.

### Preliminary Data

The preliminary data suggested that administration of a metalloporphyrin, such as stannsoporfin, to an infant from birth to 20 days old, effectively reduces total serum bilirubin levels and has no significant side effects.

**Table 1 - Table detailing the disposition of infant subjects in a clinical study**

| **Total Number of Subjects** | **Stannsoporfin 1.5 mg/kg (N=17)** | **Stannsoporfin 3.0 mg/kg (N=18)** | **Stannsoporfin 4.5mg/kg (N=8)** | **Placebo (N=15)** |
|---|---|---|---|---|
| **Safety** | 17 | 18 | 8 | 15 |
| **Intent-to-treat** | 17 | 18 | 8 | 15 |
| **Received PT** | 3 (17.6%) | 6 (33.3%) | 2 (25%) | 8 (53.3%) |
| **Per Protocol** | 10 (58.8%) | 13 (72.2%) | 5 (62.5%) | 13 (86.7%) |
| **Completed** | 16 (94.1%) | 18 (100%) | 8 (100%) | 14 (93.3%) |
| **Discontinued** | 1 (5.9%) | 0 | 0 | 1 (6.7%) |

**Table 2 - Table detailing the disposition of infant subjects in a clinical study**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Gestational age (Mean±SD) | 39.1±0.91 | 39.5±0.99 | 39.0±1.33 | 38.9±1.44 |
| Gender M:F | 7:10 | 12:6 | 2:6 | 8:7 |
| Birth Weight (Mean±SD) | 3393±503 | 3582±415 | 3337±539 | 3356±514 |
| Coombs +/- | 16/17 | 18/18 | 5/3 | 14/1 |
| Rh + | 16 | 18 | 7 | 15 |

**Table 3 - Table detailing the disposition of infant subjects in a clinical study**

| | **Stannsoporfin 15 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Risk Category | | | | |
| Low | 1 | 0 | 0 | 1 |
| Medium | 14 | 18 | 7 | 11 |
| High | 2 | 0 | 1 | 3 |

| Blood type | | | | |
|---|---|---|---|---|
| A | | 11 | 5 | 7 |
| B | | 5 | 1 | 7 |
| AB | 0 | 0 | 0 | 0 |
| O | 2 | 2 | 2 | 0 |

**Table 4 - Table detailing the maternal disposition in the clinical study**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Age ±SD | 27.1+6.1 | 28.2±6.3 | 27.6±6.7 | 27.6±5.3 |

| Blood type | | | | |
|---|---|---|---|---|
| A | 1 | 1 | 0 | 0 |
| B | 0 | 1 | 1 | 0 |
| AB | 0 | 0 | 0 | 0 |
| O | 16 | 16 | 7 | 15 |
| Rh +/- | 13/4 | 17/1 | 6/2 | 15/0 |

**Table 5 - Table detailing the maternal disposition in the clinical study**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Gestational Diabetes | 2 | 3 | 0 | 1 |
| Pre-Eclampsia | 1 | 1 | 0 | 0 |
| Pregnancy HTN | 0 | 1 | 0 | 0 |
| Multiple Births | 1 | 0 | 0 | 0 |
| Previous infant with jaundice | 5 | 5 | 3 | 3 |
| Required PT | 3 | 3 | 0 | 3 |

**Table 6 - Pharmacokinetic data for 1.5 mg/kg, 3.0 mg/kg and 4.5mg/kg doses.**

| **Dose** | **Cₘₐₓ ng/ml** | **Tₘₐₓ hr** | **T_{½} hr** |
|---|---|---|---|
| 1.5 mg/kg | 6450 | 1.87 | 5.46 |
| 3.0 mg/kg | 11,500 | 1.52 | 10.7 |
| 4.5 mg/kg | 20,400 | 2.3 | 9.86 |
| Mean | | 1.89 | 8.6 |

There is no difference in the type or severity of adverse events between groups. One infant had a self-limited rash (1.5mg/kg) considered related to study drug and phototherapy. There were four serious adverse events, anemia, meningitis and two cases of hyperbilirubinemia requiring hospitalization for phototherapy. Chemistries, liver function tests and renal function tests were normal. With the exception of platelet counts, all other hematologic parameters were normal. Platelet counts decreased from baseline to 48 hours after the administration of stannsoporfin, whereas the platelet count for subjects in the placebo remained stable. See Tables 13 and 14. However, as can be seen in Table 13, by day 14 following administration of stannsoporfin, average platelet counts had returned to the normal range in all test groups. Table 14 reports the baseline and 48 hours post dose platelet counts for certain subjects in the 4.5 mg/kg dose group.

Additionally, there was no effect on heart rate, PR interval, RR interval, QRS complexes and QT interval. This was the first clinical study in humans regarding QT data. Importantly, the study showed no QT prolongation, which is used as an indicator of cardiac effects. All physical exams were normal. All neurologic exams were normal. Hearing tests were essentially normal.

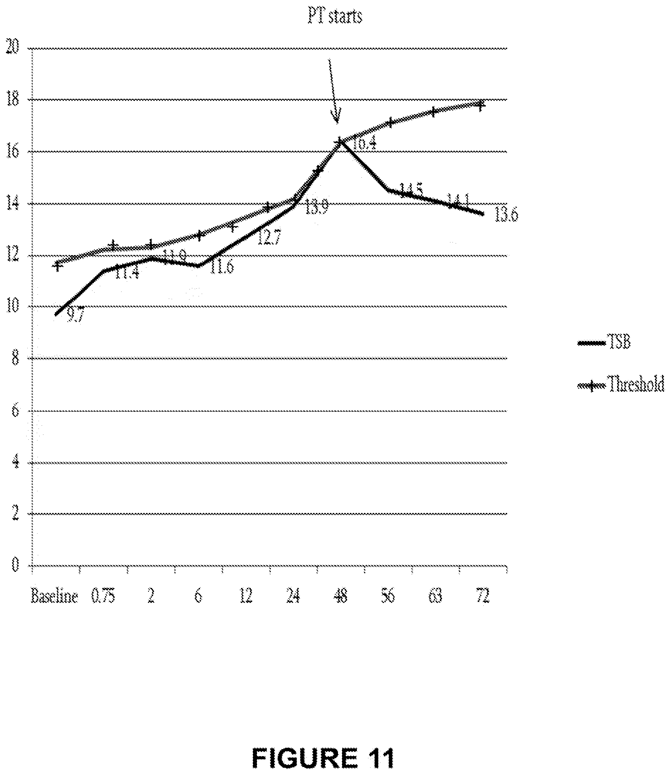

Tables 7, 8 and 9 report adverse events. Importantly, due to reporting anomalies, hyperbilirubinemia and jaundice were reported as adverse events in a portion of the study. These should not have been reported, since these conditions are, in fact, the conditions being treated. None of the reported events appears to be serious and/or drug related. Table 10 reports adverse events in which a single patient reported the adverse event. Figure 11 reports serious adverse events and includes hyperbilirubinemia in the placebo group, which was subsequently resolved.

**Table 7 - Table detailing the adverse events of the subjects in the clinical study.**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| **Total AEs** | 17 | 16 | 8 | 8 |
| **# of subjects one AE** | 8 (47.1%) | 10 (55.6%) | 3 (37.5%) | 5 (33.3%) |

**Table 8 - Table detailing the adverse events of the subjects in the clinical study.**

| **Preferred Term** | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| **Anemia** | 0 | 1(5.6%) | 1(12.5%) | 0 |
| **Hyperbilirubinemia** | 2 (11.8%) | 1 (5.6%) | 0 | 2 (13.3%) |
| **Jaundice** | 5 (29.4%) | 0 | 0 | 1 (6.7%) |
| **Meningitis** | 0 | 0 | 1 (12.5%) | 0 |
| **Oral Candidiasis** | 0 | 0 | 0 | 2 (13.3%) |
| **Diaper dermatitis** | 2 (11.8%) | 1 (5.6%) | 0 | 1 (6.7%) |
| **Erythema** | 0 | 1 (5.6%) | 1 (12.5%) | 0 |
| **Erythema toxicum** | 0 | 3 (16.7%) | 0 | 0 |
| **Rash** | 1 (5.9%) | 0 | 0 | 0 |
| **Rash neonatal** | 0 | 1 (5.6%) | 0 | 0 |
| **Rash papular** | 0 | 0 | 0 | 1 (6.7%) |
| **Seborrhoeic dermatitis** | 0 | 1 (5.6%) | 0 | 0 |
| **Skin exfoliation** | 1 (5.9%) | 0 | 0 | 0 |
| **Flushing** | 0 | 1 (5.6%) | 0 | 0 |

**Table 9 - table detailing adverse events experienced by single patients in the clinical study.**

| **Preferred Term** | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| **Leukocytosis** | 0 | 0 | 1 (12.5%) | 0 |
| **Thrombocytopenia** | 0 | 0 | 1(12.5%) | 0 |
| **Bradycardia** | 0 | 0 | 1 (12.5%) | 0 |
| **Umbilical Hernia** | 0 | 1 (5.6%) | 0 | 0 |
| **Vomiting** | 0 | 0 | 0 | 1 (6.7%) |
| **Anal Abscess** | 0 | 1 (5.6%) | 0 | 0 |
| **Contusion** | 1 (5.9%) | 0 | 0 | 0 |
| **Serum Glucose decreased** | 0 | 1 (5.6%) | 0 | 0 |
| **Serum sodium increased** | 0 | 1 (5.6%) | 0 | 0 |
| **CRP increased** | 1 (5.9%) | 0 | 0 | 0 |
| **CO₂ decreased** | 0 | 1 (5.6%) | 0 | 0 |
| **Hemoglobin increased** | 0 | 0 | 1 (12.5%) | 0 |
| **Hemangioma** | 1 (5.9%) | 0 | 0 | 0 |
| **Depressed level consciousness** | 0 | 0 | 1 (12.5%) | 0 |

**Table 10 - Table of serious adverse events in the clinical study**

| **Preferred Term** | **Dose Group** | **History** | **Outcome** |
|---|---|---|---|
| Hyperbilirubinemia (2) | Placebo | Readmit for PT | Resolved |
| Anemia | 3.0 mg/kg | Rh incompatibility Required transfusion | Resolved |
| Meningitis | 4.5 mg/kg | Negative cultures | Resolved |

Administration of the stannsoporfin doses had no effect on vital signs, such as, without limitation, blood pressure, pulse, respiratory rate, and temperature. Furthermore, there was no effect present when a general physical and neurological examination was conducted. There was also no effect on the weight gain, linear growth and head growth in the subjects. Laboratory data showed no effect on chemistry, including without limitation, sodium, potassium and chlorine levels, liver function tests, albumin/total protein, renal function, hemoglobin/hematocrit, white cell count, lymphocytes, eosinophils, basophils and reticulocytes.

A dermatological assessment, shown in Tables 11 and 12, indicated that three (17.6%) subjects dosed with 1.5 mg/kg of stannsoporfin had a rash and out of those with a rash, two subjects (66.7%) were administered phototherapy. Additionally, eight (44.4%) subjects dosed with 3.0 mg/kg stannsoporfin had a rash and out of those with a rash, three (37.5%) were administered phototherapy. In the 4.5 mg/kg group, one (12.5%) subject had a rash and was also administered phototherapy. In the placebo group, two (13.3%) subjects had a rash and of those with a rash, one (50%) was administered phototherapy. Table 12 reports the types of rashes that were observed.

**Table 11- Dermatology assessments of the subjects in the clinical study.**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Subjects with any rash | 3(17.6%) | 8(44.4%) | 1(13.3%) | 2(13.3%) |
| Rash and PT | 2(66.7%) | 3(37.5%) | 1(12.5%) | 1 (50%) |

**Table 12 - Dermatology assessments of the subjects in the clinical study.**

| **Stannsoporfin 1.5 mg/kg (N=17)** | **Stannsoporfin 3.0mg/kg (N=18)** | **Stannsoporfin 4.5 mg/kg (N=8)** | **Placebo (N=15)** |
|---|---|---|---|
| PT Induced rash* Desquamation Contact Dermatitis Contusion Diaper Dermatitis | Diaper rash Erythema Flushing Neonatal Acne Erythema Toxicum(4) Seborrhea | Erythema** | Yeast Diaper rash Papular Rash |

**Table 13 - Laboratory data of platelet counts in the clinical study.**

| **Platelet counts over time (mean)** | | | | |
|---|---|---|---|---|
| **Time** | **Stannsoporfin 1.5 mg/kg** | **Stannsoporfin 3.0 mg/kg** | **Stannsoporfin 4.5 mg/kg** | **Placebo** |
| Baseline | 262,000 | 263,000 | 265,000 | 265,000 |
| 48 hours | 234.000 | 220,000 | 180,000 | 282,000 |
| Delta | *-31,000* | *-43,000* | *-85,000* | *+17,000* |
| Day 14 | 477,000 | 453,000 | 441,000 | 471,000 |

**Table 14 - laboratory data of platelet counts in the clinical study.**

| ▲ **Baseline in Platelet counts - 4.5mg dose** | | | |
|---|---|---|---|
| **Baseline (n=8)** | **48 hours post dose (n=6)** | **Delta** | **%Change** |
| 379,000 | 368,000 | -11,000 | 2.9 |
| 263,000 | 240,000 | -22,000 | 8.4 |
| 312,000 | 184,000^{†} | -128,000 | 41.0 |
| 223,000 | 70,000* | -153,000 | 68.6 |
| 240,000 | 127,000^{†} | -113,000 | 47.1 |
| 232,000 | 91,000** | -141,000 | 60.8 |

| | | | |
|---|---|---|---|
| **† Not CS and No adverse events reported meningitis/**Infection** | | | |

Accordingly, the drug may be administered with little fear of side effects. This indicates that the drug may be administered earlier and in a broader array of patients, perhaps eliminating the need for phototherapy and/or exchange transfusion.

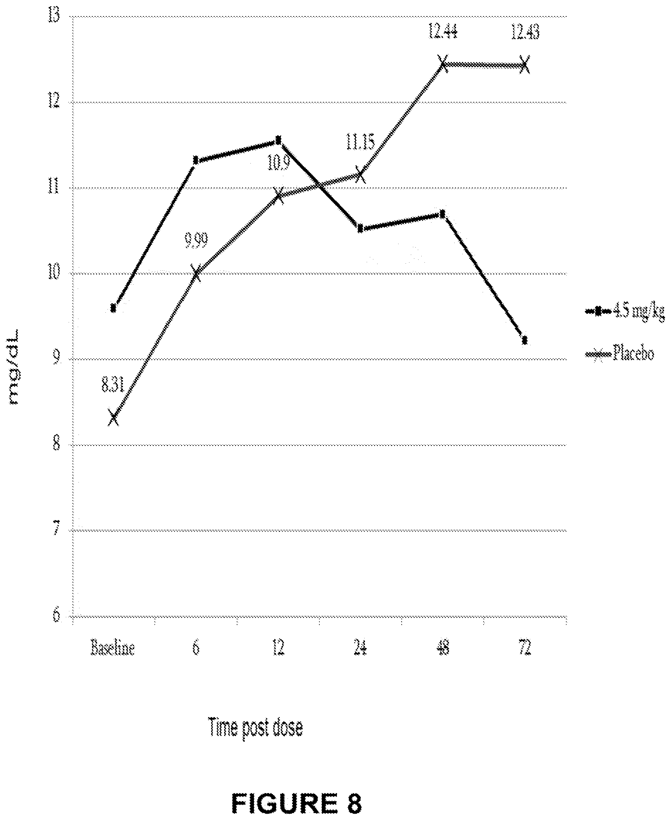

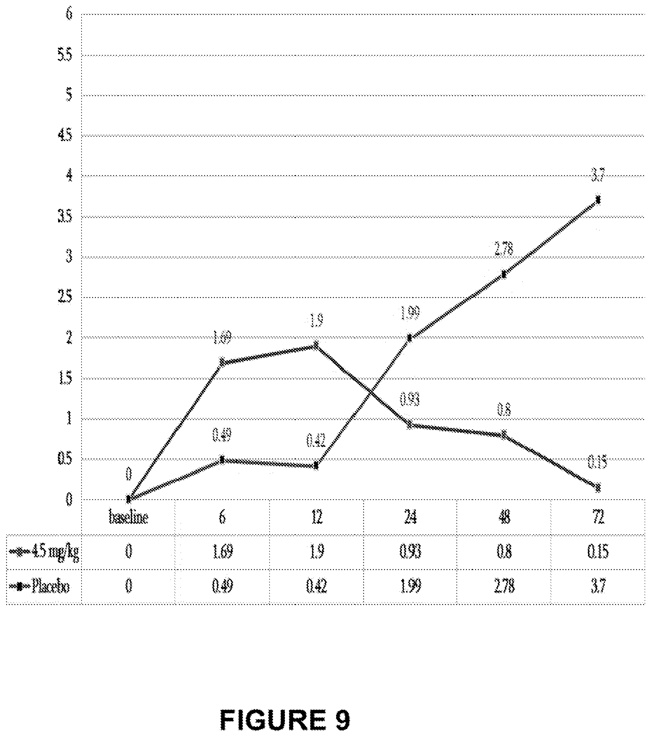

Efficacy data indicates that the total serum bilirubin levels for those in the intent to treat group increased far less than the placebo group. See Table 15. Intention to treat analysis uses data from all subjects who received study drug and had at least one efficacy assessment. Per protocol, analysis includes only subjects who have no protocol violations that could impact the assessment of efficacy. Eliminating those that dropped out or were not treated per protocol, the treated per protocol group shows statistically significant change from baseline of total serum bilirubin in the 3.0 mg/kg and 4.5 mg/kg groups. See Table 16. As seen in Table 17, in viewing the change from baseline of total serum bilirubin of subjects treated with 4.5 mg/kg stannsoporfin v. placebo at different time points, it is seen that after about 24 hours, a statistically significant effect can be seen in patients treated with stannsoporfin. In the placebo group, on the other hand, the total serum bilirubin continues to rise at an accelerated level. See Table 17 and Figures 8-9. Figure 7 indicates that the total serum bilirubin levels for all groups treated with stannsoporfin increased at a lower rate after 12 hours and decreased between 12-24 and 48-72 hours. On the other hand, the total serum bilirubin levels for the placebo group continued to increase and appeared to plateau after 48 hours.

**Table 15 - Change from baseline of total serum bilirubin in the intent to treat group.**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Baseline | 7.59±1.8 | 8.21±1.6 | 9.59±1.9 | 8.31±2.2 |
| 48 hours | 9.91±3.1 | 10.68±3.0 | 10.39±2.7 | 12.26±2.17 |
| *Delta* | *232±2.5* | *2.47±2.7* | *0.80±2.8* | *3.95±3.7* |
| | p=0.061 | p=0.163 | p=0.028 | |

**Table 16 - Change from baseline of total serum bilirubin in the treated per protocol group.**

| | **Stannsoporfin 1.5 mg/kg (N=10)** | **Stannsoporfin 3.0 mg/kg (N=13)** | **Stannsoporfin 4.5 mg/kg (N=5)** | **Placebo (N=13)** |
|---|---|---|---|---|
| Baseline | 7.52±2.0 | 8.33±1.6 | 9.47±2.3 | 8.31±2.2 |
| 48 hours | 10.96±1.8 | 10.53±2.9 | 9.88±2.8 | 12.24±2.1 |
| *Delta* | *3.44±1.5* | *2,20±2.9* | *0.04±3.1* | *3.93±2.6* |
| | p=0.338 | p=0.078 | p=0.030 | |

**Table 17 - Change from baseline of total serum bilirubin at particular time points.**

| **Time** | **Stannsoporfin 4.5 mg/kg (N=5)** | **Placebo (N=13)** | **P Value** |
|---|---|---|---|
| **6 hours** | 1.69±0.50 | 1.67±0.65 | p=0.940 |
| **12 Hours** | 1.89±.0.42 | 2.59±.0.80 | p=0.215 |
| **24 hours** | 0.93±0.20 | 2.84±.1.02 | p=0.030 |
| **48 hours** | 0.80±2.79 | 3.95±2.71 | p=0.028 |
| **72 hours** | 0.15±.3.7 | 4.26±.3.8 | p=0.020 |

Table 18 reports the incidence of subjects needing phototherapy. As shown in Table 18, in the 1.5 mg/kg stannsoporfin group, three subjects (17.6%) needed phototherapy. Six subjects (33.3%) from the 3.0 mg/kg dose stannsoporfin group and two subjects (25%) from the 4.5 mg/kg dose stannsoporfin group required phototherapy whereas 8 subjects (53.3%) from the placebo group required phototherapy. There were no exchange transfusions. Accordingly, administration of stannsoporfin in all groups reduced the need for phototherapy. Table 19 is a table detailing the range of time (in hours) that it took after administration of stannsoporfin for the subject to reach the phototherapy threshold. The 1.5 mg/kg dose stannsoporfin group which needed phototherapy reached the phototherapy threshold at about 5.2 to about 17.8 hours. The 3.0 mg/kg dose stannsoporfin group which needed phototherapy reached the phototherapy threshold at about 7.2 to about 15.2 hours. The 4.5 mg/kg dose stannsoporfin group which needed phototherapy reached the phototherapy threshold at about 7.3 to about 8.8 hours. In the placebo group, a wide range was seen with those needing phototherapy reaching the phototherapy threshold anywhere between about 1 hour to about 45.8 hours.

**Table 18 - Incidence of phototherapy in the clinical study post dose (there were no exchange transfusions).**

| **Incidence of PT** | | | |
|---|---|---|---|
| **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
| 3(17.6%) | 6 (33.3%) | 2 (25%) | 8 (53.3%) |
| p=0.062 | p=0.467 | p=0.379 | |

**Table 19 - Time required to reach a serum bilirubin level which required acirninistration of phototherapy.**

| **Time to PT (hours, range)** | | | |
|---|---|---|---|
| **Stannsoporfin 15 mg/kg (N=17)** | **Stannsoporfin 3.0 mg/kg (N=18)** | **Stannsoporfin 45 mg/kg (N=8)** | **Placebo (N=15)** |
| 5.2-17.8 | 7.2-15.2 | 7.3-8.8 | 1.0-45.8 |
| p=0.032 | p=0.342 | p=0.177 | |

The duration of phototherapy, as shown in Table 20, was about 20 hours for the 1.5 mg/kg group, about 14 hours for the 3.0 mg/kg group, about 14 hours for the 4.5 mg/kg group, and about 16 hours for the placebo group. Table 21 is a table detailing the average and range of time from treatment to discharge of the subjects. As shown in Figure 24, average discharge time for the 1.5 mg/kg group was 38 hours, 42.8 hours for the 3.0 mg/kg group, 48.3 hours for the 4.5 mg/kg group and 28.1 hours for the placebo group. No subjects who received stannsoporfin were readmitted. However, two of the 15 placebo subjects (13.3%) were readmitted. Figure 10 shows the bilirubin levels of a placebo patient who was dosed at 39 hours of age with the placebo dose and started phototherapy at 29 hours post dose. Figure 11 shows the total serum bilirubin levels of a placebo subject dosed at 46 hours of age, phototherapy started 48 hours post dose for 7 hours.

**Table 20 - Duration of phototherapy.**

| **Duration of PT (minutes)** | | | |
|---|---|---|---|
| **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
| 1202±200 20 hours | 847±207 14 hours | 847±201 14 hours | 973±489 16 hours |
| ns | ns | ns | |

**Table 21 - Time to Discharge**

| **Time to discharge (hours)** | | | | |
|---|---|---|---|---|
| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
| Mean | 38 | 42.8 | 48.3 | 28.1 |
| Range | 14.6-143.3 | 11.5-77.0 | 15-405.3 | 12.0-48.7 |

### Final Data

The study has been completed and data analyzed. The findings are consistent with the preliminary findings, and are reported in further detail below.

### Summary of Efficacy Results

In the analysis of the primary efficacy endpoint, a decrease in adjusted TSB levels from baseline to 48 hours after treatment in the ITT population was observed in each treatment group, with greater numerical decreases seen as the dose of stannsoporfin increased. The reduction from baseline in least square mean (LSM) adjusted TSB was statistically significantly greater in the stannsoporfin 1.5 mg/kg group than in the placebo group at 48 hours post-treatment (p=0.040, ANCOVA). No statistical differences were seen in the PP population. The mixed model repeated measures (MMRM) analysis showed a statistically significant greater reduction in LSM in the stannsoporfin 4.5 mg/kg group.

In the analysis of the secondary efficacy endpoints, the mean unadjusted TSB levels increased less in the starmsoporfin-treated groups than in the placebo group from 6 to 12 hours post-treatment onward, with the smallest increase in TSB (i.e, the maximum effect) seen at the highest dose of stannsoporfin (4.5 mg/kg) and the largest increase seen in the placebo group. The ANCOVA analysis showed a statistically significant smaller increase in LSM in the stannsoporfin 4.5 mg/kg group than in the placebo group.

Across the assessed time points (6, 12, 24, 48, and 72 hours), the reduction from baseline in LSM adjusted TSB was statistically significantly greater in the stannsoporfin 1.5 mg/kg group than in the placebo group at 48 and 72 hours post-treatment. For unadjusted TSB across those same time points, the ANCOVA analysis showed a statistically significant smaller increase in LSM in the stannsoporfin 4.5 mg/kg group than in the placebo group at 24, 48, and 72 hours post-treatment, and in the stannsoporfin, 1.5 mg/kg group than in the placebo group at 72 hours post-treatment.

At 14 days after treatment, mean TSB levels were decreasing to adult levels (3.06 mg/dL in the stannsoporfin 1.5 mg/kg group, 5.23 mg/dL in the stannsoporfin 3.0 mg/kg group, 2.94 mg/dL in the stannsoporfin 4.5 mg/kg group, and 5.70 mg/dL in the placebo group), with no apparent dose effects.

There were no subjects requiring ET. Nineteen subjects required PT; 53.3% of placebo subjects required PT (including 2 infants who were readmitted to the hospital for PT), whereas across all stannsoporfin treatment groups, approximately 26% required PT. This difference did not test as statistically significant due to the sample size limitations. In the stannsoporfin 1.5 and 3.0 mg/kg groups, the likelihood of requiring PT was significantly related to adjusted TSB levels at baseline (i.e, the proximity of the subject's TSB to the TSB threshold at study entry).

The time to PT, data were variable between subjects and difficult to interpret in terms of a clear drug effect; it should be noted that the study was not designed to measure an effect on time to PT. In all treatment groups, time to PT was significantly related to adjusted TSB levels at baseline and also to age and treatment in the stannsoporfin 4.5 mg/kg group. Importantly, there were 2 subjects in the placebo group that were readmitted to the hospital for PT after discharge, and this did not occur in stannsoporfin-treated subjects. The mean duration of PT across all subjects reflected the lower numbers of stannsoporfin subjects receiving PT, and ranged from approximately 212 to 280 minutes in the stannsoporfin treatment groups and was approximately 520 minutes in the placebo group. The maximum duration of PT was 1426 minutes in the 1.5 mg/kg stannsoporfin group, 1140 minutes in the 3 mg/kg stannsoporfin group, 990 minutes in the 4.5 mg/kg stannsoporfin group, and 1840 minutes in the placebo group.

Time to hospital discharge was significantly greater in the stannsoporfin 4.5 mg/kg group compared to the placebo group, largely due to 1 subject who required a prolonged hospital stay for the treatment of meningitis. When one patient with prolonged hospitalization due to meningitis was removed from the analyses, time to hospital discharge was similar in the stannsoporfin 4.5 mg/kg group compared to the placebo group.

Analysis of exposure data showed that stannsoporfin was rapidly and well absorbed from an IM injection, with peak plasma concentrations observed within 1 hour post-treatment. The elimination of stannsoporfin from plasma follows linear kinetics, and the elimination half-life was approximately 10 hours. Plasma stannsoporfin concentrations were measurable for at least 48 hours post treatment in all subjects at doses of 3 mg/kg and above. The intersubject variability in Cmax and AUCs was generally <30%, which is considered relatively small for neonates. The small intersubject variability could be related to good absorption from the IM injection site, as well as a relatively small contribution of metabolism in the elimination of stannsoporfin. There was a dose-proportional increase in Cmax over the 1.5 to 4.5 mg/kg dose range, suggesting that the absorption of stannsoporfin from the injection site follows first-order linear kinetics. There was slightly more than a dose-proportional increase in area under the plasma concentration versus time curve from time 0 to infinity (AUCO-inf) of stannsoporfin, especially from the 1.5 to 3.0 mg/kg dose range, which could be partly due to the low plasma stannsoporfin concentrations in the 1.5 mg/kg group that fell below the limit of quantitation. As the dose increased from 3.0 to 4.5 mg/kg, there was only a 20% to 25% more than dose proportional increase in the AUCO-inf of stannsoporfin, which may not be clinically meaningful.

### Detailed description of Efficacy Endpoint Results

The primary endpoint was the change in adjusted TSB from baseline to 48 hours after treatment. A decrease in adjusted TSB levels from baseline to 48 hours after treatment was observed in each treatment group, with greater numerical decreases seen as the dose of stannsoporfin increased. The LSM change from baseline was 15.0% (1.5 mg/kg stannsoporfin), 11.6% (3.0 mg/kg stannsoporfin), and 16.5% (4.5 mg/kg stannsoporfin) compared to 1.6 in the placebo group. The difference in reduction of LSM between the stannsoporfin 1.5 mg/kg group and the placebo group was statistically significant (p=0.040, ANCOVA) (Table 22).

The baseline adjusted TSB values showed some differences between treatment groups, in that the stannsoporfin 4.5 mg/kg group had the TSBs nearest the PT threshold ( 9%), the stannsoporfin 1.5 mg/kg group had the TSBs furthest below the threshold (20%), and the placebo group was 13% below the threshold. These findings are consistent with the unadjusted levels, which were highest in the stannsoporfin 4.5 mg/kg group.

**Table 22 - Change from Baseline in Adjusted TSB at 48 Hours (ITT Population)**

| | **Stannsoporfin 1.5 mg/kg (N=17)** | **Stannsoporfin 3.0 mg/kg (N=18)** | **Stannsoporfin 4.5 mg/kg (N=8)** | **Placebo (N=15)** |
|---|---|---|---|---|
| Baseline adjusted **TSB (%)** | | | | |
| n | 17 | 18 | 8 | 15 |
| Mean (SD) | -20.24 (7.956) | -15.92 (12.002) | -9.19 (8.027) | -13.41 (8.519) |
| Median | -19.60 | -18.00 | -7.65 | -14.60 |
| Min, Max | -38.1, -4.0 | -32.0, 0.0 | -22.9, -0.8 | -28. 1, 1.7 |
| 48 Hours/Early termination adjusted TSB (%) | | | | |
| n | 17 | 18 | 8 | 15 |
| Mean (SD) | -35.09 (19.198) | -31.80 (18.214) | -34.14 (14.733) | -19.43 (14.922) |
| Median | -27.10 | -24.75 | -32.00 | -15.30 |
| Min, Max | -79.2, -17.2 | -72.2, -6.6 | -64.6, -17.8 | -40.5, 9.3 |
| 48 Hours/Early termination change from baseline in adjusted TSB (%) | | | | |
| n | 17 | 18 | 8 | 15 |
| Mean (SD) | -14.85 (15.442) | -15.88 (21.406) | -24.95 (19.261) | -6.03 (18.659) |
| Median | -12.30 | -9.05 | -19.95 | -5.70 |
| Min, Max | -55.7, 0.3 | -54.8, 12.0 | -59.0, -5.0 | -42.1, 22.2 |
| LS mean (SEM) | -15.03 (5.273) | -11.60 (5.612) | -16.51 (6.925) | - 1.58 (5.050) |
| LS mean difference *³* | -13.45 | -10.02 | -14.93 | |
| 95% confidence interval **^{a}** | (-26.27, -0.62) | (-22.61, 2.58) | (-30.31, 0.44) | |
| P-value **^{a}** | 0.040 | 0.117 | 0.057 | |

| | | | | |
|---|---|---|---|---|
| "Pairwise comparison for each stannsoporfin treatment group versus placebo. Note: LOCF is used to impute missing postbaseline TSB. Least-squares means are from an ANCOVA model for adjusted TSB with treatment and gestational age as fixed effects and baseline adjusted TSB as a covariate. TSB is calculated as [(TSB - PT threshold/ PT threshold] x 100%. ANCOVA = analysis of covariance; ITT = intent-to-treat; LOCF = last observation carried forward; Max = maximum; Min = minimum; PT = phototherapy; SD = standard deviation; SEM = standard error of mean; TSB = total serum bilirubin. | | | | |

**In the sensitivity analysis, the primary analysis was repeated using (1) the PP population and (2) an MMRM analysis. As with the primary analysis, the analysis on the PP population showed a decrease in adjusted TSB levels from baseline to 48 hours after treatment, with greater decreases seen as the dose of stannsoporfin increased; however, the ANCOVA analysis did not show any statistically significant difference in LSM between the placebo group and any of the treatment groups. The MMRM analysis showed a statistically significant greater reduction in LSM between the placebo and stannsoporfin 4.5 mg/kg groups at the 24 and 48 hour post treatment time points (Table 23).**

**Table 23 - Change from Baseline in Adjusted TSB - MMRM Analysis (ITT Population)**

| **Time point Statistics** | **Stannsoporfin 1.5 mg/kg (N=17)** | **Stannsoporfin** 3.0 **mg/kg (N=18)** | **Stannsoporfin 4.5 mg/kg (N=8)** | **Placebo (N=15)** |
|---|---|---|---|---|
| 6 Hours | | | | |
| **LS** means **(SEM)** | 2.94 (2.098) | 2.68 (2.252) | 2.00 (2.825) | 4.37 (1.993) |
| **LS** mean difference | -1.44 | -1.69 | -2.38 | |
| 95% confidence interval | (-6.45, 3.58) | (-6.61, 3.23) | (-8.56, 3.80) | |
| P-value | 0.567 | 0.493 | 0.444 | |

| 12 Hours | | | | |
|---|---|---|---|---|
| **LS** means **(SEM)** | 3.29 (2.731) | 0.83 (2.821) | -2.41 (3.856) | 5.97 (2.728) |
| **LS** mean difference | -2.67 | -5.14 | -8.38 | |
| **95%** confidence interval | **(-9.84, 4.49)** | **(-12.19, 1.91)** | **(-17.30, 0.55)** | |
| P-value | **0.457** | **0.150** | **0.065** | |
| **24** Hours | | | | |
| **LS** means **(SEM)** | **-5.33 (3.948)** | **-8.57 (3.894)** | **-16.26 (5.495)** | **-1.56 (4.011)** |
| **LS** mean difference | **-3.77** | **-7.01** | **-14.70** | |
| **95%** confidence interval | **(-14.70,7.16)** | **(-17.70, 3.68)** | **(-28.02, -1.39)** | |
| P-value | **0.491** | **0.193** | **0.031** | |
| **48** Hours | | | | |
| **LS** means **(SEM)** | **-14.94 (5.019)** | **-15.92 (4.919)** | **-27.34 (7.287)** | **-6.68 (5.255)** |
| **LS** mean difference**^{a}** | **-8.26** | **-9.24** | **-20.66** | |
| **95%** confidence interval" | **(-22.55, 6.03)** | **(-23.26, 4.79)** | **(-38.41, -2.91)** | |
| P-value**^{a}** | **0.251** | **0.192** | **0.023** | |

| | | | | |
|---|---|---|---|---|
| **^{a}** Pairwise comparison for each Stannsoporfin treatment group versus the placebo group. Note: MMRM analysis is conducted for observed case adjusted TSB with treatment, gestational age group (**35-37** weeks **6** days or ≥ **38** weeks), time point, treatment-by-time point as fixed effects, and observed case adjusted baseline TSB levels as a covariate. Time point is a repeated factor and an unstructured covariance matrix pattern is applied. TSB is observed case and is calculated as [(TSB - PT threshold)/ PT threshold] X **100%.** ITT = intent-to-treat, LS = least squares, MMRM = Mixed Model Repeated Measures; PT = phototherapy, SEM = standard error of mean, TSB = total serum bilirubin. | | | | |

### Summary of Safety Results

**Of the 58 subjects in the safety population (who received study drug), 17 subjects received stannsoporfin 1.5 mg/kg, 18 subjects received 3.0 mg/kg, 8 subjects received 4.5 mg/kg, and 15 subjects were administered a single dose of placebo.**

**There were no statistically significant differences between the 3 stannsoporfin treatment groups and the placebo group in the incidence of TEAEs. Greater than 30% of subjects in each treatment group experienced at least 1 TEAE, and all except 1 TEAE were considered mild or moderate in severity. There was 1 case of a severe contusion reported, which was considered unlikely related to study drug by the investigator. There were 4 SAEs** reported (anemia, meningitis, and 2 cases of hyperbilirubinemia), all of which resolved, and were considered not related to the study drug and were either mild or moderate in severity.

In the clinical laboratory evaluation, the majority of hematological and clinical chemistry parameters showed no dose-related trends or marked differences in mean values between the stannsoporfin-treated groups and the placebo group; all mean values for all parameters and treatment groups were well within normal ranges, and most returned to baseline levels at Day 14.

Some shifts from normal to high neutrophil levels and from normal to low platelet counts were observed after treatment with stannsoporfin; all subjects in the stannsoporfin 4.5 mg/kg group showed moderate drops in platelets at 48 hours, which had normalized by Day 14. The evaluation of individual shifts from baseline in clinical laboratory results showed no dose related trends or marked differences between the stannsoporfin treatment groups compared to the placebo group for any parameter except platelets.

There was a brief and self-limiting decrease in platelets that was evident at 48 hours and returned to normal by Day 14. There were no bleeding abnormalities associated with this decrease in platelets.

There were a number of clinical laboratory changes reported as AEs during the study, all of which were mild or moderate in severity, and only a hemoglobin increased and a thrombocytopenia were considered possibly related to the study drug.

The evaluation of vital signs showed a decrease in mean pulse observed 45 minutes after treatment with stannsoporfin that did not occur in the placebo group. The effect was no longer observed at 1.5 hours post treatment, and all measured pulse rates were within normal limits, during the study, except for 1 measurement of 87 bpm that occurred in a placebo subject 72 hours post-treatment. There were no other dose-related trends or marked differences observed in vital signs between the treatment groups.

There were no differences among the treatment groups in physical examination results or change from baseline in weight, length, or head circumference.

There was no significant difference between the proportion of subjects who experienced rashes and received PT in the stannsoporfin treatment groups versus the placebo group. Skin and subcutaneous tissue disorders reported as AEs were mild or moderate, and most were considered not related to the study drug. One case of erythema, 1 case of erythema toxicum neonatorum, and 1 case of rash in the stannsoporfin treated groups were considered probably or possibly related to the study drug.

All subjects had normal audiology examinations at screening, 48 hours after treatment/hospital discharge, or early termination.

Ophthalmological examinations showed very few abnormalities. Four subjects had retinal pigmentation. None of the abnormalities were reported as AEs, and there were no dose-related trends or marked differences between the stannsoporfin-treated groups and the placebo group in number of abnormalities.

There were few neurological abnormalities reported among the treatment groups, and they were regarded as not being of clinical significance by the investigators. There were no dose-related trends or marked differences between the stannsoporfin-treated groups and the placebo group in results. There was an AE of depressed level of consciousness reported for a subject in the stannsoporfin 4.5 mg/kg group (Subject 038-0012) that started 28 hours after receiving treatment and resolved within 3 days. The event was considered moderate in severity and unlikely related to the study drug.

The ECG results showed no dose-related trends or marked differences between the stannsoporfin treated groups compared to the placebo group. There were a few QTc outliers observed in every treatment group and at every time point.

### Detailed description of Efficacy Endpoint Results

The change from baseline in unadjusted TSB at 48 hours after treatment was analyzed using the ITT (Table 25) and PP (Table 28) populations. The placebo group demonstrated an increase in the TSB from baseline, representing the natural course of the condition. All 3 doses of stannsoporfin reduced the increase in TSB at 48 hours with a numerical dose-response, and the ANCOVA analysis showed a statistically significant difference (smaller increase) in LSM between the stannsoporfin 4.5 mg/kg and placebo groups for both ITT and PP populations.

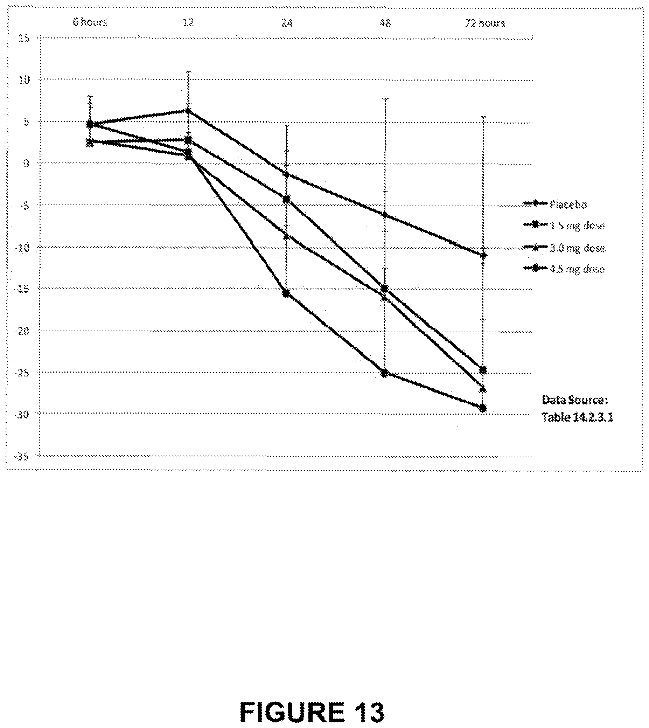

The change from baseline in adjusted TSB at the 6, 12, 24, 48, and 72 hour post treatment time points is shown in Table 29. The mean adjusted TSB levels for each treatment group from baseline to 72 hours after treatment are shown in Figure 13 and table 24.

**Table 24 - Change from Baseline in Adjusted Total Serum Bilirubin (TSB) by Time Point - LOCF (ITT Population).**

| | Stannsoporfin 1.5 mg/kg (N-17) | Stannsoporfin 3.0 mg/kg (N=18) | Stannsoporfin 4.5 mg/kg (N=8) | Placebo (N-1 5) |
|---|---|---|---|---|
| Baseline Adj.TSB (%) | | | | |
| n | 17 | 18 | 8 | 15 |
| Mean (SD) | -20.24 (7.956) | -15.92 (12.002) | -9.19 (8.027) | -13.41 (8.519) |
| Min, Max | -38.1, -4.0 | -32.0, 0.0 | -22.9, -0.8 | -28.1, 1.7 |

| 6 Hours Adj.TSB (%) | | | | |
|---|---|---|---|---|
| n | 17 | 18 | 8 | 15 |
| Mean (SD) | -17.77 (13.326) | -13.20 (13.1 18) | -4.49 (11.322) | -8.73 (10.283) |
| Min, Max | -43.8, 2.9 | -32.7, 6.0 | -24.8, 7.8 | -28.8, 10.3 |

| 6 Hours Change from Baseline in Adj.TSB (%) | | | | |
|---|---|---|---|---|
| n | 17 | 18 | 8 | 15 |
| Mean (SD) | 2.47 (7.928) | 2.72 (7.274) | 4.70 (4.583) | 4.67 (4.941) |
| Min, Max | -14.6, 12.8 | -13.4, 20.7 | -1.9, 13.4 | -1.9, 15.5 |
| LS Mean (SEM) | 2.72 (2.053) | 2.57 (2.186) | 4.03 (2.697) | 4.38 (1.967) |
| LS Mean Difference [1] | -1.67 | -1.82 | -0.35 | |
| 95% Confidence Interval [1] | (-6.66, 3.33) | (-6.72, 3.09) | (-6.34, 5.64) | |
| P-value [1] | 0.506 | 0.461 | 0.907 | |

| 12 Hours Adj.TSB (%) | | | | |
|---|---|---|---|---|
| n | 17 | 18 | 8 | 15 |
| Mean (SD) | -17.41 (17.294) | -15.06 (13.787) | -7.80 (9.906) | -7.14 (12.454) |
| Min, Max | -47.8, 13.6 | -37.3, 5.6 | -25.2, 7.8 | -25.0, 11.2 |

| 12 Hours Change from Baseline in Adj.TSB (%) | | | | |
|---|---|---|---|---|
| n | 17 | 18 | 8 | 15 |
| Mean (SD) | 2.83 (12.949) | 0.87 (9.503) | 1.39 (6.394) | 6.27 (6.934) |
| Min, Max | -21.7, 33.2 | -22.6, 18.5 | -4.9, 13.4 | -3.6, 23.7 |
| LS Mean (SEM) | 4.54 (3.015) | 2.64 (3.209) | 2.17 (3.960) | 7.13 (2.888) |
| LS Mean Difference [1] | -2.59 | -4.50 | -4.96 | |
| 95% Confidence Interval [1] | (-9.93, 4.74) | (-11.70, 2.70) | (-13.75, 3.83) | |
| P-value [1] | 0.481 | 0.216 | 0.263 | |

| 24 Hours Adj.TSB (%) | | | | |
|---|---|---|---|---|
| n | 17 | 18 | 8 | 15 |
| Mean (SD) | -24.44 (18.927) | -24.46 (11.302) | -24.66 (15.145) | -14.67 (10.384) |
| Min, Max | -62.0, 13.6 | -46.4, -8.8 | -42.9, -0.7 | -30.5, 3.4 |

| 24 Hours Change from Baseline in Adj.TSB (%) | | | | |
|---|---|---|---|---|
| n | 17 | 18 | 8 | 15 |
| Mean (SD) | -4.20 (16.064) | -8.53 (16.045) | -15.48 (18.694) | -1.26 (10.804) |
| Min, Max | -38.5, 33.2 | -38.0, 15.9 | -39.3, 12.1 | -19.0, 15.9 |
| LS Mean (SEM) | -7.62 (4.320) | -9.21 (4.598) | -11.59 (5.674) | -0.09 (4.137) |
| LS Mean Difference [1] | -7.53 | -9.12 | -11.50 | |
| 95% Confidence Interval [1] | (-18.04, 2.98) | (-19.44, 1.20) | (-24.10, 1.10) | |
| P-value [1] | 0.157 | 0.082 | 0.073 | |

| 48 Hours Adj.TSB (%) | | | | |
|---|---|---|---|---|
| n | 17 | 18 | 8 | 15 |
| Mean (SD) | -35.09 (19.198) | -31.80 (18.214) | -34.14 (14.733) | -19.43 (14.922) |
| Min, Max | -79.2, -17.2 | -72.2, -6.6 | -64.6, -17.8 | -40.5, 9.3 |

| 48 Hours Change from Baseline in Adj.TSB (%) | | | | |
|---|---|---|---|---|
| n | 17 | 18 | 8 | 15 |
| Mean (SD) | -14.85 (15.442) | -15.88 (21.406) | -24.95 (19.261) | -6.03 (18.659) |
| Min, Max | -55.7, 0.3 | -54.8, 12.0 | -59.0, -5.0 | -42.1, 22.2 |
| LS Mean (SEM) | -15.03 (5.273) | -11.60 (5.612) | -16.51 (6.925) | -1.58 (5.050) |
| LS Mean Difference [1] | -13.45 | -10.02 | -14.93 | |
| 95% Confidence Interval [1] | (-26.27, -0.62) | (-22.61, 2.58) | (-30.31, 0.44) | |
| P-value [1] | 0.040 | 0.117 | 0.057 | |

| 72 Hours/Early Termination Adj.TSB (%) | | | | |
|---|---|---|---|---|
| n | 17 | 18 | 8 | 15 |
| Mean(SD) | -44.82 (19.254) | -42.64 (21.640) | -38.38 (16.637) | -24.27 (23. 134) |
| Min, Max | -79.2, -17.9 | -83.3, -16.9 | -75.9, -21.6 | -73.9, 9.7 |

| 72 Hours/Early Termination Change from Baseline in Adj.TSB (%) | | | | |
|---|---|---|---|---|
| n | 17 | 18 | 8 | 15 |
| Mean (SD) | -24.58 (15.743) | -26.72 (24.245) | -29.19 (19.225) | -10.87 (25. 123) |
| Min, Max | -55.7, -1.2 | -70.4, 6.4 | -70.3, -11.9 | -75.6, 17.8 |
| LS Mean (SEM) | -21.94 (6.232) | - 19.28 (6.633) | - 19.00 (8. 184) | -4.75 (5.968) |
| LS Mean Difference [1] | -17.19 | -14.53 | -14.25 | |
| 95% Confidence Interval [1] | (-32.35, -2.03) | (-29.42, 0.35) | (-32.42, 3.92) | |
| P-value [1] | 0.027 | 0.055 | 0.122 | |
| Note: Last Observation Carry Forward (LOCF) is used to impute missing post-baseline TSB. Analysis of covariance (ANCOVA) is conducted | | | | |
| for adjusted TSB including treatment and gestational age as fixed effects and baseline adjusted TSB as a covariate. Least-squares means | | | | |
| (LS means) and standard errors (SEM) are estimated for each treatment group and placebo. LS mean difference, 95% Confidence Interval, | | | | |
| and p-value are estimated based on LS mean difference between each stannsoporfin group and placebo. Adjusted (adj.) TSB is calculated | | | | |
| as [(TSB - Phototherapy (PT) threshold)/ PT threshold] X 100%. | | | | |
| [1] Pairwise comparison for each Stannsoporfin treatment group versus placebo. | | | | |

**The ANCOVA analysis showed a statistically significant greater reduction in LSM adjusted TSB levels in the stannsoporfin 1.5 mg/kg group at 48 and 72 hours post-treatment than in the placebo group. The results from the other treatment groups did not reach statistical significance.**

**Table 25 - Change from Baseline in Adjusted TSB Time point (ITT Population)**

| | **Stannsoporfin 1.5 mg/kg (N=10)** | **Stannsoporfin 3.0 mg/kg (N=13)** | **Stannsoporfin 4.5 mg/kg (N=8)** | **Placebo (N=13)** |
|---|---|---|---|---|
| Baseline adjusted TSB (%) | | | | |
| n | 17 | 18. | 8 | 15 |
| Mean (SD) | -20.24 (7.956) | -15.92 (12.002) | -9.19 (8.027) | -13.41 (8.519) |
| Median | -19.60 | -18.00 | -7.65 | -14.60 |
| Min, Max | -38.1, -4.0 | -32.0, 0.0 | -22.9, -0.8 | -28.1, 1.7 |

| 6 Hours change from baseline in adjusted TSB (%) | | | | |
|---|---|---|---|---|
| n | 17 | 18 | 8 | 15 |
| Mean (SD) | 2.47 (7.928) | 2.72 (7.274) | 4.70 (4.583) | 4.67 (4.941) |
| Median | 2.80 | 2.10 | 4.10 | 3.80 |
| Min, Max | -14.6, 12.8 | -13.4, 20.7 | -1.9, 13.4 | -1.9, 15.5 |
| LS mean (SEM) | 2.72 (2.053) | 2.57 (2.186) | 4.03 (2.697) | 4.38 (1.967) |
| LS mean difference **^{a}** | -1.67 | -1.82 | -0.35 | |
| 95% confidence interval **^{a}** | (-6.66, 3.33) | (-6.72, 3.09) | (-6.34, 5.64) | |
| P-value **^{a}** | 0.506 | 0.461 | 0.907 | |

| 12 Hours change from baseline in adjusted TSB (%) | | | | |
|---|---|---|---|---|
| n | 17 | 18 | 8 | 15 |
| Mean (SD) | 2.83 (12.949) | 0.87 (9.503) | 1.39 (6.394) | 6.27 (6.934) |
| Median | 5.70 | 1.55 | -0.70 | 4.20 |

| | **Stannsoporfin 1.5 mg/kg (N=10)** | **Stannsoporfin 3.0 mg/kg (N=13)** | **Stannsoporfin 4.5 mg/kg (N=8)** | **Placebo (N=13)** |
|---|---|---|---|---|
| Min, Max | -21.7, 33.2 | -22.6, 18.5 | -4.9, 13.4 | -3.6, 23.7 |
| LS mean (SEM) | 4.54 (3.015) | 2.64 (3.209) | 2.17 (3.960) | 7.13 (2.888) |
| LS mean difference **^{a}** | -2.59 | -4.50 | -4.96 | |
| 95% confidence interval **^{a}** | (-9.93, 4.74) | (-11.70, 2.70) | (-13.75, 3.83) | |
| P-value **^{a}** | 0.481 | 0.216 | 0.263 | |

| 24 Hours change from baseline in adjusted TSB (%) | | | | |
|---|---|---|---|---|
| n | 17 | 18 | 8 | 15 |
| Mean (SD) | -4.20 (16.064) | -8.53 (16.045) | -15.48 (18.694) | -1.26 (10.804) |
| Median | -4.30 | -3.10 | - 11.15 | 1.30 |
| Min, Max | -38.5, 33.2 | -38.0, 15.9 | -39.3, 12.1 | -19.0, 15.9 |
| LS mean (SEM) | -7.62 (4.320) | -9.21 (4.598) | -11.59 (5.674) | -0.09 (4.137) |
| LS mean difference **^{a}** | -7.53 | -9.12 | -11.50 | |
| 95% confidence interval **^{a}** | (-18.04, 2.98) | (-19.44, 1.20) | (-24. 10, 1.10) | |
| P-value **^{a}** | 0.157 | 0.082 | 0.073 | |

| 48 Hours change from baseline in adjusted TSB (%) | | | | |
|---|---|---|---|---|
| n | 17 | 18 | 8 | 15 |
| Mean (SD) | -14.85 (15.442) | -15.88 (21.406) | -24.95 (19.261) | -6.03 (18.659) |
| Median | -12.30 | -9.05 | -19.95 | -5.70 |
| Min, Max | -55.7, 0.3 | -54.8, 12.0 | -59.0, -5.0 | -42.1, 22.2 |
| LS mean (SEM) | -15.03 (5.273) | -11.60 (5.612) | -16.51 (6.925) | -1.58 (5.050) |
| LS mean difference **^{a}** | -13.45 | -10.02 | -14.93 | |
| 95% confidence interval **^{a}** | (-26.27, -0.62) | (-22.61, 2.58) | (-30.31, 0.44) | |
| P-value **^{a}** | 0.040 | 0.117 | 0.057 | |

| 72 Hours change from baseline in adjusted TSB (%) | | | | |
|---|---|---|---|---|
| n | 17 | 18 | 8 | 15 |
| Mean (SD) | -24.58 (15.743) | -26.72 (24.245) | -29.19 (19.225) | -10.87 (25.123) |
| Median | -22.10 | -17.30 | -21.45 | -5.20 |
| Min, Max | -55.7, -1.2 | -70.4, 6.4 | -70.3, -11.9 | -75.6, 17.8 |
| LS mean (SEM) | -21.94 (6.232) | -19.28 (6.633) | -19.00 (8.184) | -4.75 (5.968) |
| LS mean difference **^{a}** | -17.19 | -14.53 | -14.25 | |
| 95% Confidence interval **^{a}** | (-32.35, -2.03) | (-29.42, 0.35) | (-32.42, 3.92) | |
| P-value **^{a}** | 0.027 | 0.055 | 0.122 | |

| | | | | |
|---|---|---|---|---|
| **^{a}** Pairwise comparison for each Stannsoporfin treatment group versus the placebo group. Note: LOCF is used to impute missing post-baseline TSB. ANCOVA is conducted for TSB including treatment and gestational age as fixed effects and baseline TSB as a covanate. LS means and standard errors (SEM) are estimated for each treatment group and the placebo group. LS mean difference, 95% Confidence Interval, and p-value are estimated based on LS mean difference between each stannsoporfin group and the placebo group. ANCOVA = analysis of covariance; ITT = intent-to-treat; LOCF = last observation carried forward; LS = least squares; Max = maximum; Min = minimum; PT = phototherapy; SD = standard deviation; SEM = standard error of mean; TSB = total serum bilirubin. | | | | |

**The change from baseline in unadjusted TSB at the 6, 12, 24, 48, and 72 hour and 14 day post treatment time points is shown in Table 26 below.**

**Table 26 - Change from Baseline in Unadjusted Total Serum Bilirubin (TSB) by Time Point - LOCF(ITT Population)**

| | Stannsoporfin 1.5 mg/kg (N=17) | Stannsoporfin 3.0 mg/kg (N=18) | Stannsoporfin 4.5 mg/kg (N=8) | Placebo (N=15) |
|---|---|---|---|---|
| Baseline Unadj.TSB (mg/dL) | | | | |
| n | 17 | 18 | 8 | 15 |
| Mean (SD) | 7.59 (1.816) | 8.21 (1.577) | 9.59 (1.911) | 8.31 (2.154) |
| Median | 7.80 | 8.45 | 9.35 | 8.00 |
| Min, Max | 4.3, 12.0 | 5.5, 10.4 | 6.4, 12.8 | 4.6, 11.9 |

| 6 hrs Unadj.TSB (mg/dL) | | | | |
|---|---|---|---|---|
| n | 17 | 18 | 8 | 15 |
| Mean (SD) | 9.09 (2.175) | 9.74 (1.730) | 11.27 (2.074) | 9.99 (2.000) |
| Median | 9.60 | 9.90 | 11.25 | 10.20 |
| Min, Max | 5.1, 14.1 | 6.8, 12.3 | 7.6,14.8 | 5.7, 13.0 |

| 6 hrs Change from Baseline in Unadj.TSB (mg/dL) | | | | |
|---|---|---|---|---|
| n | 17 | 18 | 8 | 15 |
| Mean (SD) | 1.50 (0.861) | 1.53 (0.762) | 1.69 (0.493) | 1.67 (0.647) |
| Median | 1.50 | 1.35 | 1.61 | 1.50 |
| Min, Max | -0.1, 2.9 | -0.2, 3.6 | 1.2,2.6 | 0.9, 3.2 |
| LS Mean (SEM) | 1.38 (0.242) | 1.39 (0.254) | 1.62 (0.293) | 1.59 (0.221) |
| LS Mean Difference [1] | -0.21 | -0.20 | 0.03 | |
| 95% Confidence Interval [1] | (-0.76, 0.33) | (-0.75, 0.34) | (-0.65, 0.70) | |
| P-value [1] | 0.433 | 0.464 | 0.940 | |

| 12 hrs Unadj.TSB (mg/dL) | | | | |
|---|---|---|---|---|
| n | 17 | 18 | 8 | 15 |
| Mean (SD) | 9.82 (2.599) | 10.26 (1.748) | 11.48 (1.924) | 10.90 (1.989) |
| Median | 10.20 | 10.90 | 11.27 | 10.90 |
| Min, Max | 5.6, 14.3 | 6.9, 12.2 | 8.0, 14.8 | 6.6, 13.8 |

| 12 hrs Change from Baseline in Unadj.TSB (mg/dL) | | | | |
|---|---|---|---|---|
| n | 17 | 18 | 8 | 15 |
| Mean (SD) | 2.22 (1.624) | 2.05 (1.004) | 1.89 (0.421) | 2.59 (0.800) |
| Median | 2.50 | 1.90 | 1.92 | 2.40 |
| Min, Max | -1.0, 6.0 | 0.0, 4.5 | 1.1, 2.5 | 1.5, 4.1 |
| LS Mean (SEM) | 2.21 (0.374) | 2.06 (0.393) | 1.95 (0.453) | 2.60 (0.342) |
| LS Mean Difference [1] | -0.39 | -0.54 | -0.65 | |
| 95% Confidence Interval [1] | (-1.23, 0.46) | (-1.38, 0.31) | (-1.69, 0.39) | |
| P-value [1] | 0.363 | 0.206 | 0.215 | |

| 24 hrs Unadj.TSB (mg/dL) | | | | |
|---|---|---|---|---|
| n | 17 | 18 | 8 | 15 |
| Mean (SD) | 9.84 (2.656) | 10.06 (1.757) | 10.52 (2.668) | 11.15 (2.160) |
| Median | 10.80 | 10.45 | 9.66 | 10.60 |
| Min, Max | 5.2,14.2 | 6.7, 11.9 | 7.7, 14.0 | 7.7, 15.2 |

| 24 hrs Change from Baseline in Unadj.TSB (mg/dL) | | | | |
|---|---|---|---|---|
| n | 17 | 18 | 8 | 15 |
| Mean (SD) | 2.24 (2.084) | 1.85 (1.596) | 0.93 (1.990) | 2.84 (1.016) |
| Median | 2.70 | 2.08 | 1.20 | 3.10 |
| Min, Max | -2.6, 6.0 | -0.9, 5.3 | -1.4, 3.8 | 1.0, 5.0 |
| LS Mean (SEM) | 1.98 (0.549) | 1.67 (0.578) | 1.05 (0.665) | 2.75 (0.502) |
| LS Mean Difference [1] | -0.76 | -1.08 | -1.70 | |
| 95% Confidence Interval [1] | (-2.00, 0.48) | (-2.32, 0.16) | (-3.22, -0.18) | |
| P-value [1] | 0.222 | 0.087 | 0.030 | |

| 48 hrs Unadj.TSB (mg/dL) | | | | |
|---|---|---|---|---|
| n | 17 | 18 | 8 | 15 |
| Mean (SD) | 9.91 (3.116) | 10.68 (2.956) | 10.39 (2.650) | 12.26 (2. 169) |
| Median | 10.90 | 11.65 | 10.35 | 11.90 |
| Min, Max | 3.3, 13.5 | 4.2, 14.1 | 5.6, 13.4 | 8.1, 15.8 |

| 48 hrs Change from Baseline in Unadj.TSB (mg/dL) | | | | |
|---|---|---|---|---|
| n | 17 | 18 | 8 | 15 |
| Mean (SD) | 2.32 (2.560) | 2.47 (2.703) | 0.80 (2.788) | 3.95 (2.71 1) |
| Median | 2.70 | 2.94 | 1.45 | 3.70 |
| Min, Max | -4.5, 5.2 | -3.4, 6.2 | -3.4, 3.6 | -0.2, 8.7 |
| LS Mean (SEM) | 2.43 (0.842) | 2.90 (0.886) | 1.60 (1.020) | 4.24 (0.770) |
| LS Mean Difference [1] | -1.81 | -1.34 | -2.63 | |
| 95% Confidence Interval [1] | (-3.71, 0.09) | (-3.24, 0.56) | (-4.97, -0.30) | |
| P-value [1] | 0.061 | 0.163 | 0.028 | |

| 72 hrs Unadj.TSB (mg/dL) | | | | |
|---|---|---|---|---|
| n | 17 | 18 | 8 | 15 |
| Mean (SD) | 9.35 (3.409) | 9.87 (3.681) | 9.73 (3.672) | 12.57 (3.192) |
| Median | 10.60 | 10.70 | 11.15 | 13.30 |
| Min, Max | 3.3, 14.7 | 2.9, 14.1 | 3.7, 13.4 | 4.6, 16.3 |

| 72 hrs Change from Baseline in Unadj.TSB (mg/dL) | | | | |
|---|---|---|---|---|
| n | 17 | 18 | 8 | 15 |
| Mean (SD) | 1.76 (2.790) | 1.66 (3.496) | 0.15 (3.740) | 4.26 (3.805) |
| Median | 2.40 | 2.45 | 1.90 | 4.60 |
| Min, Max | -4.5, 6.0 | -4.7, 6.2 | -6.0, 3.7 | -3.6, 9.6 |
| LS Mean (SEM) | 2.43 (1.077) | 2.77 (1.133) | 1.38 (1.305) | 4.96 (0.984) |
| LS Mean Difference [1] | -2.52 | -2.18 | -3.57 | |
| 95% Confidence Interval [1] | (-4.95, -0.10) | (-4.61, 0.25) | (-6.56, -0.58) | |
| P-value [1] | 0.042 | 0.077 | 0.020 | |

| 14 days/Early Termination Unadj.TSB (mg/dL) | | | | |
|---|---|---|---|---|
| N | 17 | 18 | 8 | 15 |
| Mean (SD) | 3.52 (2.751) | 5.23 (5.123) | 5.31 (4.596) | 5.63 (2.567) |
| Median | 2.80 | 3.40 | 2.98 | 5.00 |
| Min, Max | 0.4, 10.9 | 0.6, 18.8 | 1.7, 13.4 | 2.0, 9.7 |

| 14 days/Early Termiantion Change from Baseline in Unadj.TSB (mg/dL) | | | | |
|---|---|---|---|---|
| n | 17 | 18 | 8 | 15 |
| Mean (SD) | -4.08 (2.322) | -2.98 (5.389) | -4.28 (3.828) | -2.69 (3.767) |
| Median | -4.00 | -4.80 | -5.73 | -1.70 |
| Min, Max | -7.4, 1.9 | -9.1, 12.2 | -8.0, 2.2 | -9.9, 2.8 |
| | | | | |
| | | | | |
| LS Mean (SEM) | -4.04 (1.258) | -2.42 (1.324) | -3.02 (1.524) | -2.30 (1.150) |
| LS Mean Difference [1] | -1.74 | -0.12 | -0.72 | |
| 95% Confidence Interval [1] | (-4.58, 1.10) | (-2.96, 2.72) | (-4.21, 2.77) | |
| P-value [1] | 0.224 | 0.931 | 0.682 | |
| Note: Last Observation Carry Forward (LOCF) is used to impute missing post-baseline TSB. Analysis of covariance (ANCOVA) is conducted forTSB including treatment and gestational age as fixed effects and baseline TSB as a covariate. Least-squares means (LS means) and standard errors (SEM) are estimated for each treatment group and placebo. LS mean difference, 95% Confidence Interval, and p-value are estimated based on LS mean difference between each stannsoporfin group and placebo. | | | | |
| [1] Pairwise comparison for each Stannsoporfin treatment group versus placebo. | | | | |

**Table 27 - Percent Change from Baseline in Unadjusted Total Serum Bilirubin (TSB) By Time Point (ITT Population)**

| | Stannsoporfin 1.5 mg/kg (N=I7) | Stannsoporfin 3.0 mg/kg (N=18) | Stannsoporfin 4.5 mg/kg (N=8) | Placebo (N=15) |
|---|---|---|---|---|
| Baseline TSB (mg/dL) | | | | |
| n | 17 | 18 | 8 | 15 |
| Mean (SD) | 7.59 (1.816) | 8.21 (1.577) | 9.59 (1.91 1) | 8.31 (2.154) |
| Min, Max | 4.3, 12.0 | 5.5, 10.4 | 6.4, 12.8 | 4.6, 11.9 |

| 6 hrs TSB (mg/dL) | | | | |
|---|---|---|---|---|
| n | 17 | 18 | 7 | 15 |
| Mean (SD) | 9.09 (2.175) | 9.74 (1.730) | 11.3 1 (2.237) | 9.99 (2.000) |
| Min, Max | 5.1, 14.1 | 6.8, 12.3 | 7.6, 14.8 | 5.7, 13.0 |

| 6 hrs Percent Change from Baseline in TSB (%) | | | | |
|---|---|---|---|---|
| n | 17 | 18 | 7 | 15 |
| Mean (SD) | 20.13 (12.268) | 19.54 (12.055) | 17.33 (5.373) | 22.20 (12.265) |
| Min, Max | -1.7, 42.0 | -2.6, 59.0 | 10.7, 26.4 | 9.2, 46.3 |

| 12 hrs TSB (mg/dL) | | | | |
|---|---|---|---|---|
| n | 17 | 18 | 7 | 15 |
| Mean (SD) | 9.82 (2.599) | 10.26 (1.748) | 11.54 (2.067) | 10.90 (1.989) |
| Min, Max | 5.6, 14.3 | 6.9, 12.2 | 8.0, 14.8 | 6.6, 13.8 |

| 12 hrs Percent Change from Baseline in TSB (%) | | | | |
|---|---|---|---|---|
| n | 17 | 18 | 7 | 15 |
| Mean (SD) | 29.91 (21.467) | 26.17 (15.650) | 20.16 (6.326) | 34.23 (16.979) |
| Min, Max | -12.8, 73.2 | 0.0, 73.8 | 9.8, 29.2 | 16.0, 70.4 |

| 24 hrs TSB (mg/dL) | | | | |
|---|---|---|---|---|
| n | 15 | 18 | 8 | 15 |
| Mean (SD) | 9.61 (2.566) | 10.06 (1.757) | 10.52 (2.668) | 11.15 (2.160) |
| Min, Max | 5.2, 12.2 | 6.7, 11.9 | 7.7, 14.0 | 7.7, 15.2 |

| 24 hrs Percent Change from Baseline in TSB (%) | | | | |
|---|---|---|---|---|
| n | 15 | 18 | 8 | 15 |
| Mean (SD) | 30.23 (27.126) | 24.84 (23.900) | 10.75 (23.016) | 37.72 (21.024) |
| Median | 38.20 | 23.00 | 12.15 | 34.20 |
| Min, Max | -33.3, 66.1 | -11.8, 86.9 | -14.8, 43.8 | 11.5,92.6 |

| 48 hrs TSB (mg/dL) | | | | |
|---|---|---|---|---|
| n | 16 | 18 | 7 | 14 |
| Mean (SD) | 9.85 (3.208) | 10.68 (2.956) | 10.69 (2.708) | 12.44 (2.127) |
| Min, Max | 3.3, 13.5 | 4.2, 14.1 | 5.6, 13.4 | 8.1, 15.8 |
| | | | | |

| 48 hrs Percent Change from Baseline in TSB (%) | | | | |
|---|---|---|---|---|
| n | 16 | 18 | 7 | 14 |
| Mean (SD) | 32.71 (37.298) | 31.84 (36.062) | 14.87 (33.167) | 59.62 (48.163) |
| Min, Max | -57.7, 82.3 | -44.7, 88.5 | -37.7, 56.3 | -1.7, 161. 1 |

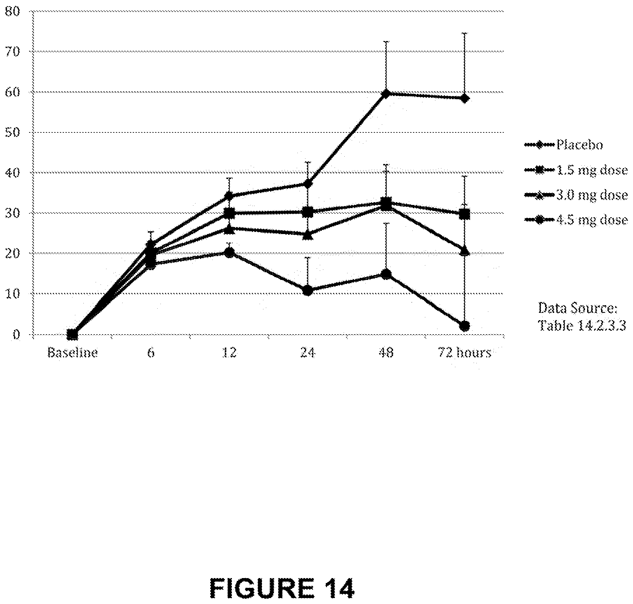

**The mean unadjusted TSB levels for each treatment group from baseline to 14 days after treatment are shown in** **Figure 14** **and table 27. In all treatment groups, the mean unadjusted TSB levels increased from baseline to 72 hours post treatment. Decreases in mean unadjusted TSB levels were seen at the 14-day time point. The ANCOVA analysis showed a statistically significant greater difference (smaller increase) in LSM between the stannsoporfin 4.5 mg/kg and placebo groups at 24, 48, and 72 hours post-treatment and between the stannsoporfin 1.5 mg/kg and placebo groups at 72 hours post-treatment.**

**Table 28 - Change from Baseline in Unadjusted TSB at 48 Hours (ITT Population)**

| | **Stannsoporfin 1.5 mg/kg (N=17)** | **Stannsoporfin** 3.0 **mg/kg (N=18)** | **Stannsoporfin 4.5 mg/kg (N=8)** | **Placebo (N=15)** |
|---|---|---|---|---|
| Baseline unadjusted TSB (mg/dL) | | | | |
| n | 17 | 18 | 8 | 15 |
| Mean (SD) | 7.59 (1.816) | 8.21 (1.577) | 9.59 (1.91 1) | 8.3 1(2.154) |
| Median | 7.80 | 8.45 | 9.35 | 8.00 |
| Min, Max | 4.3, 12.0 | 5.5, 10.4 | 6.4, 12.8 | 4.6, 11.9 |

| 48 Hours/Early termination unadjusted TSB (mg/dL) | | | | |
|---|---|---|---|---|
| n | 17 | 18 | 8 | 15 |
| Mean (SD) | 9.91 (3.116) | 10.68 (2.956) | 10.39 (2.650) | 12.26 (2.169) |
| Median | 10.90 | 11.65 | 10.35 | 11.90 |
| Min, max | 3.3, 13.5 | 4.2, 14.1 | 5.6, 13.4 | 8.1, 15.8 |

| 48 Hours/Early termination change from baseline in unadjusted TSB (mg/dL) | | | | |
|---|---|---|---|---|
| n | 17 | 18 | 8 | 15 |
| Mean (SD) | 2.32 (2.560) | 2.47 (2.703) | 0.80 (2.788) | 3.95 (2.71 1) |
| Median | 2.70 | 2.94 | 1.45 | 3.70 |
| Min, Max | -4.5, 5.2 | -3.4, 6.2 | -3.4, 3.6 | -0.2, 8.7 |
| LS mean (SEM) | 2.43 (0.842) | 2.90 (0.886) | 1.60 (1.020) | 4.24 (0.770) |
| LS mean difference **^{a}** | -1.81 | -1.34 | -2.63 | |
| 95% confidence interval **^{a}** | (-3.71, 0.09) | (-3.24, 0.56) | (-4.97, -0.30) | |
| P-value **^{a}** | 0.061 | 0.163 | 0.028 | |

| | | | | |
|---|---|---|---|---|
| **^{a}** Pairwise comparison for each Stannsoporfin treatment group versus the placebo group. Note: LOCF is used to impute missing post-baseline TSB. ANCOVA is conducted for TSB including treatment and gestational age as fixed effects and baseline TSB as a covariate. LS means and standard errors (SEM) are estimated for each treatment group and the placebo group. LS mean difference, 95% Confidence Interval, and p-value are estimated based on LS mean difference between each stannsoporfin group and the placebo group. ANCOVA = analysis of covariance, ITT = intent-to-treat, LOCF = last observation carried forward, LS = least squares; Max = maximum; Min = minimum; PT = phototherapy, SD = standard deviation, SEM = standard error of mean, TSB = total serum bilirubin | | | | |

**Table 29 - Change from Baseline in Unadjusted TSB at 48 Hours (PP Population)**

| | **Stannsoporfin 1.5 mg/kg (N=10)** | **Stannsoporfin 3.0 mg/kg (N=13)** | **Stannsoporfin 4.5 mg/kg (N=5)** | **Placebo (N=13)** |
|---|---|---|---|---|
| Baseline unadjusted TSB (mg/dL) | | | | |
| n | 10 | 13 | 5 | 13 |
| Mean (SD) | 7.52 (2.030) | 8.33 (1.585) | 9.47 (2.319) | 8.31 (2.322) |
| Median | 7.35 | 8.90 | 9.00 | 7.60 |
| Min, Max | 4.3, 12.0 | 6.0, 10.4 | 6.4, 12.8 | 4.6, 11.9 |

| 48 Hours/Early termination unadjusted TSB (mg/dL) | | | | |
|---|---|---|---|---|
| n | 10 | 13 | 5 | 13 |
| Mean (SD) | 10.96 (1.842) | 10.53 (2.91 1) | 9.88 (2.807) | 12.24 (2.066) |
| Median | 11.35 | 11.60 | 10.00 | 11.90 |
| Min, Max | 6.8, 12.8 | 4.2, 13.8 | 5.6, 13.4 | 8.1, 15.8 |

| 48 Hours/Early termination change from baseline in unadjusted TSB (mg/dL) | | | | |
|---|---|---|---|---|
| n | 10 | 13 | 5 | 13 |
| Mean (SD) | 3.44 (1.458) | 2.20 (2.928) | 0.40 (3.111) | 3.93 (2.650) |
| Median | 3.40 | 2.87 | 0.70 | 3.70 |
| Min, Max | 0.7, 5.2 | -3.4, 5.4 | -3.4, 3.6 | -0.2, 8.7 |
| LS mean (SEM) | 2.96 (0.868) | 2.20 (0.881) | 1.11 (1.095) | 3.93 (0.722) |
| LS mean difference **^{a}** | -0.97 | -1.74 | -2.82 | |

| | Stannsoporfin 1.5 mg/kg (N=10) | Stannsoporfin 3.0 mg/kg (N=13) | Stannsoporfin 4.5 mg/kg (N=5) | Placebo (N=13) |
|---|---|---|---|---|
| 95% confidence interval^{a} | (-3.00, 1.06) | (-3.68, 0.20) | (-5.36, -0.29) | |
| P-value ^{a} | 0.338 | 0.078 | 0.030 | |

| | | | | |
|---|---|---|---|---|
| **" Pairwise comparison for each Stannsoporfin treatment group versus the placebo group.** **Note: LOCF is used to impute missing post-baseline TSB. ANCOVA is conducted for TSB including treatment and gestational age as fixed effects and baseline TSB as a covariate. LS means and standard errors (SEM) are estimated for each treatment group and the placebo group. LS mean difference, 95% Confidence Interval, and p-value are estimated based on LS mean difference between each stannsoporfin group and the placebo group.** **ANCOVA = analysis of covariance; ITT = intent-to-treat; LOCF = last observation carried forward; LS = least squares; Max = maximum; Min = minimum; PT = phototherapy; SD = standard deviation; SEM = standard error of mean; TSB = total serum bilirubin.** | | | | |

### Safety Summary

Of the 58 subjects in the safety population, 17 subjects received stannsoporfin 1.5 mg/kg, 18 subjects received 3.0 mg/kg, 8 subjects received 4.5 mg/kg, and 15 subjects were administered a single dose of placebo.

There were no statistically significant differences between the 3 stannsoporfin treatment groups and placebo in the incidence of TEAEs. Greater than 30% of subjects in each treatment group experienced at least 1 TEAE, and all except 1 TEAE were considered mild or moderate in severity. There was 1 case of a severe contusion reported, which was considered unlikely related to study drug by the investigator. There were 4 SAEs reported (anemia, meningitis, and 2 cases of hyperbilirubinemia), all of which resolved, and were considered not related to the study drug and were either mild or moderate in severity.

In the clinical laboratory evaluation, the majority of hematological and clinical chemistry parameters showed no dose-related trends or marked differences in mean values between the stannsoporfin treated groups and the placebo group, all mean values for all parameters and treatment groups were well within normal ranges, and most returned to baseline levels at Day 14.

Some shifts from normal to high neutrophil levels and from normal to low platelet counts were observed after treatment with stannsoporfin; all subjects in the stannsoporfin 4.5 mg/kg group showed moderate drops in platelets at 48 hours, which had normalized by Day 14. The evaluation of individual shifts from baseline in clinical laboratory results showed no dose related trends or marked differences between the stannsoporfin treatment groups compared to the placebo group for any parameter except platelets.

There was a brief and self-limiting decrease in platelets that was evident at 48 hours and returned to normal by Day 14. There were no bleeding abnormalities associated with this decrease in platelets.

There were a number of clinical laboratory changes reported as AEs during the study, all of which were mild or moderate in severity, and only a hemoglobin increased and a thrombocytopenia were considered possibly related to the study drug.

The evaluation of vital signs showed a decrease in mean pulse observed 45 minutes after treatment with stannsoporfin that did not occur in the placebo group. The effect was no longer observed at 1.5 hours post-treatment, and all measured pulse rates were within normal limits during the study, except for 1 measurement of 87 bpm that occurred in a placebo subject 72 hours post-treatment. There were no other dose-related trends or marked differences observed in vital signs between the treatment groups.

There were no differences among the treatment groups in physical examination results or change from baseline in weight, length, or head circumference.

One subject treated with stannsoporfin and 1 subject treated with placebo had a rash after PT. There was no significant difference between the proportion of subjects who experienced rashes and received PT in the stannsoporfin treatment groups versus the placebo group. Skin and subcutaneous tissue disorders reported as AEs were mild or moderate, and most were considered not related to the study drug. One case of erythema, 1 case of erythema toxicum neonatorum, and 1 case of rash in the stannsoporfin-treated groups were considered probably or possibly related to the study drug.

All subjects had normal audiology examinations at screening, 48 hours after treatment/hospital discharge, or early termination.

Ophthalmological examinations showed very few abnormalities. Four subjects had retinal pigmentation. None of the abnormalities were reported as AEs, and there were no dose-related trends or marked differences between the stannsoporfin treated groups and the placebo group in number of abnormalities.

There were few neurological abnormalities reported among the treatment groups, and they were regarded as not being of clinical significance by the investigators. There were no dose-related trends or marked differences between the stannsoporfin-treated groups and the placebo group in results. There was an AE of depressed level of consciousness reported for a subject in the stannsoporfin 4.5 mg/kg group (Subject 038-0012) that started 28 hours after receiving treatment and resolved within 3 days. The event was considered moderate in severity and unlikely related to the study drug.

The ECG results showed no dose-related trends or marked differences between the stannsoporfin treated groups compared to the placebo group. There were a few QTc outliers observed in every treatment group and at every time point.

### Conclusions

This blinded, randomized study of neonates with hyperbilirubinemia included **58** subjects who each received a single dose of either stannsoporfin (**1.5** mg/kg, **17** subjects; **3.0** mg/kg, **18** subjects; **4.5** mg/kg, **8** subjects) or placebo (**15** subjects) across **23** study sites in the US and Europe. The study was stopped early and therefore the stannsoporfin **4.5** mg/kg group enrolled only **8** subjects. Demographic characteristics were well balanced across treatment groups, with some differences in race and gender distribution. The mean gestational age of subjects in each treatment group was approximately **39** weeks. Birth weights ranged between **2614** and **4490** g, with mean birth weights among treatment groups ranging from approximately **3,337** to **3,582** g.

In the analysis of the primary efficacy endpoint, a decrease in adjusted TSB levels from baseline to **48** hours after treatment in the ITT population was observed in each treatment group, with greater numerical decreases seen as the dose of stannsoporfin increased. The difference in reduction of LSM between the stannsoporfin **1.5** mg/kg group and the placebo group was statistically significant. MMRM analysis showed a statistically significant greater reduction in LSM TSB levels in the stannsoporfin **4.5** mg/kg group. In the secondary analysis of the unadjusted TSB levels, a statistically significant smaller increase was observed in LSM TSB levels in the stannsoporfin **4.5** mg/kg group than in the placebo group. Analysis at various time points showed statistically significant differences up to the **72** hour post treatment time point. The data indicate a dose-related effect on the rise in TSB from approximately **6** hours onward, with all **3** stannsoporfin groups reducing the TSB, maximally at the **4.5** mg/kg dose.

At **14** days after treatment, mean TSB levels were decreasing to adult levels (**3.06** mg/dL in the stannsoporfin **1.5** mg/kg group, **5.23** mg/dL in the stannsoporfin **3.0** mg/kg group, **2.94** mg/dL in the stannsoporfin **4.5** mg/kg group, and **5.70** mg/dL in the placebo group), with no apparent dose effects.

Approximately **53%** of placebo subjects went on to receive PT, compared to **26%** of stannsoporfin subjects. There were **2** subjects in the placebo group that were readmitted to the hospital for PT after discharge, and this did not occur in stannsoporfin-treated subjects.

Analysis of exposure data showed that stannsoporfin was rapidly and well absorbed from an IM injection, and the absorption of stannsoporfin followed first-order linear kinetics, with peak plasma concentrations observed within 1 hour post-treatment. The elimination half-life was approximately 10 hours.

Overall, exposure to stannsoporfin was well tolerated, with no statistically significant differences between the 3 stannsoporfin treatment groups and the placebo group in the incidence of TEAEs. All TEAEs except one (a contusion) were considered mild or moderate in severity. There were 4 SAEs reported, an anemia, a meningitis, and 2 subjects with hyperbilirubinemia, all of which were considered not related to the study drug and were either mild or moderate in severity.

In the clinical laboratory evaluation, the majority of hematological and clinical chemistry parameters showed no dose-related trends or marked differences in mean values between the stannsoporfin-treated groups and the placebo group, all mean values for all parameters and treatment groups were well within normal ranges, and most returned to baseline levels at Day 14. For clinical laboratory changes reported as AEs during the study, only a hemoglobin increased and a thrombocytopenia were considered possibly related to the study drug.

There were no concerning dose-related trends or marked differences between treatment groups observed in vital signs, physical examinations, dermatological evaluations, audiology examinations, ophthalmological evaluations, or neurological evaluations. In the ECG evaluations, there were no marked dose-related trends in either changes from baseline or absolute values.

In conclusion, this multicenter, randomized, blinded study in 58 neonates with hyperbilirubinemia demonstrated similar safety across 3 dose levels up to 4.5 mg/kg, with predictable linear PK for stannsoporfin, and efficacy as a dose-related reduction in adjusted and unadjusted TSB levels from 12 hours onward after a single dose.

It is to be understood that the embodiments disclosed herein are not limited to the particular processes, compositions, or methodologies described, as these may vary. It is also to be understood that the terminology used in the description is for the purpose of describing the particular versions or embodiments only, and is not intended to limit the scope of the present invention. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. All publications mentioned herein are incorporated by reference in their entirety. Nothing herein is to be construed as an admission that the invention is not entitled to antedate such disclosure by virtue of prior invention.

Optical Isomers-Diastereomers-Geometric Isomers-Tautomers. Compounds described herein may contain an asymmetric center and may thus exist as enantiomers. Where the compounds according to the invention possess two or more asymmetric centers, they may additionally exist as diastereomers. The present invention includes all such possible stereoisomers as substantially pure resolved enantiomers, racemic mixtures thereof, as well as mixtures of diastereomers. The formulas are shown without a definitive stereochemistry at certain positions. The present invention includes all stereoisomers of such formulas and pharmaceutically acceptable salts thereof. Diastereoisomeric pairs of enantiomers may be separated by, for example, fractional crystallization from a suitable solvent, and the pair of enantiomers thus obtained may be separated into individual stereoisomers by conventional means, for example by the use of an optically active acid or base as a resolving agent or on a chiral HPLC column. Further, any enantiomer or diastereomer of a compound of the general formula may be obtained by stereospecific synthesis using optically pure starting materials or reagents of known configuration.

It must also be noted that as used herein and in the appended claims, the singular forms "a", "an", and "the" include plural reference unless the context clearly dictates otherwise. Thus, for example, reference to "a compound" is a reference to one or more compounds and equivalents thereof known to those skilled in the art, and so forth.

As used herein, the term "about" means plus or minus 10% of the numerical value of the number with which it is being used. Therefore, about 50% means in the range of 45%-55%.

"Administering" when used in conjunction with a therapeutic means to administer a therapeutic directly into or onto a target tissue or to administer a therapeutic to a patient whereby the therapeutic positively impacts the tissue to which it is targeted. Thus, as used herein, the term "administering", when used in conjunction with a metalloporphyrin, can include, but is not limited to, providing the metalloporphyrin into or onto the target tissue; providing the metalloporphyrin systemically to a patient by, e.g., intravenous injection whereby the therapeutic reaches the target tissue. "Administering" a composition may be accomplished by injection, topical administration, or by either method in combination with other known techniques.

The term "animal," "subject" or "patient" as used herein includes, but is not limited to, humans and non-human vertebrates such as wild, domestic and farm animals. Most preferably, "animal," "subject," or "patient" refers to humans, particularly infants.

The term "improves" is used to convey that the present invention changes either the appearance, form, characteristics and/or the physical attributes of the tissue to which it is being provided, applied or administered. The change in form may be demonstrated by any of the following alone or in combination: enhanced appearance of the skin; reduced need for exchange transfusion, reduced need for phototherapy, decrease in bilirubin levels, decrease in jaundice, prevention or reduction of zone 5 jaundice, and/or reduction in the length of hospital stay.

The term "inhibiting" includes the administration of a compound of the present invention to prevent the onset of the symptoms, alleviating the symptoms, or eliminating the disease, condition or disorder.

By "pharmaceutically acceptable", it is meant the carrier, diluent or excipient must be compatible with the other ingredients of the formulation and not deleterious to the recipient thereof.

As used herein, the phrase "physiological osmolality" means the drug product or composition, when administered to a patient does not cause irritation or an adverse reaction. A suitable range for the osmolality according to certain embodiments may be from about 270 to about 328 mOsmol/L, and more preferably from about 280 to about 300 mOsmol/L osmolality.

As used herein, the term "therapeutic" means an agent utilized to treat, combat, ameliorate, prevent or improve an unwanted condition or disease of a patient. In part, embodiments of the present invention are directed to the treatment of hyperbilirubinemia or the reduction in total serum bilirubin.

A "therapeutic amount" or "effective amount" of a composition is a predetermined amount calculated to achieve the desired effect, i.e., to treat, prevent or reduce jaundice or hyperbilirubinemia, to reduce bilirubin production, to increase bilirubin excretion, or combination thereof, or to reduce total serum bilirubin and/or total cutaneous bilirubin, or to otherwise delay, inhibit, or slow the progression of hyperbilirubinemia. The activity contemplated by the present methods includes both medical therapeutic and/or prophylactic treatment, as appropriate. The specific dose of a compound administered according to this invention to obtain therapeutic and/or prophylactic effects will, of course, be determined by the particular circumstances surrounding the case, including, for example, the compound administered, the route of administration, and the condition being treated. The compounds are effective over a wide dosage range. However, it will be understood that the effective amount administered will be determined by the physician in the light of the relevant circumstances including the condition to be treated, the choice of compound to be administered, and the chosen route of administration, and therefore the above dosage ranges are not intended to limit the scope of the invention in any way. A therapeutic amount of compound of this invention is typically an amount such that when it is administered in a physiologically tolerable excipient composition, it is sufficient to achieve an effective systemic concentration or local concentration in the tissue.

The terms "treat," "treated," or "treating" as used herein refers to both therapeutic treatment and prophylactic or preventative measures, wherein the object is to prevent or slow down (lessen) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

As used herein, the term "baseline" is refers to an infant's serum bilirubin levels prior to administration of therapeutic treatment and prophylactic or preventative measures. In some embodiments, an infant's baseline serum bilirubin levels serves to measure changes in the an infant's serum bilirubin levels.

Although the present invention has been described in considerable detail with reference to certain preferred embodiments thereof, other versions are possible. Therefore, the spirit and scope of the appended claims should not be limited to the description and the preferred versions contained within this specification.
1. A method of treating hyperbilirubinemia or the symptoms thereof in an infant, the method comprising:
   administering a therapeutic amount of a metalloporphyrin to the infant with hyperbilirubinemia where no exclusion factor is present and at least one of a baseline total bilirubin level is elevated above a predetermined threshold and at least one risk factor is present;
   wherein the hyperbilirubinemia or symptoms thereof is treated.
2. The method of paragraph 1, farther comprising determining baseline total bilirubin levels in the infant.
3. The method of paragraph 1, wherein baseline total bilirubin levels comprises total serum bilirubin levels, total cutaneous bilirubin or a combination thereof.
4. The method of paragraph 1, wherein the infant is of a gestational age from about 35 to about 43 weeks.
5. The method of paragraph 1, wherein the infant has a minimum birth weight of about 2,500 g-
6. The method of paragraph 1, wherein the infant has a birth weight from about 1,700 g to about 4,000 g.
7. The method of paragraph 1, wherein the infant is Coombs positive.
8. The method of paragraph 1, wherein the infant is Coombs negative and at least one risk factor is present.
9. The method of paragraph 8, wherein the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and combinations thereof.
10. The method of paragraph 1, wherein determining baseline total bilirubin levels is performed at a time selected from within 6 hours of birth, 12 hours of birth, within 24 hours of birth, and within 48 hours of birth.
11. The method of paragraph 1, further comprising identifying the presence of at least one risk factor.
12. The method of paragraph 11, wherein the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, and G6PD deficiency and combinations thereof.
13. The method of paragraph 1, further comprising identifying the absence of at least one exclusion factor.
14. The method of paragraph 16, wherein the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is >5.0 mg/dL), persistent hypoglycemia (blood glucose <40 mg/dL) despite standard- of-care treatment, liver diseases defined as ALT and/or AST greater than 2 times the upper limit of normal [ULN], abnormal renal function defined as creatinine and or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.
15. The method of paragraph 1, wherein the predetermined threshold is the level determined by the AAP nomogram for initiating phototherapy for an infant of known age and known risk level.
16. The method of paragraph 1, wherein the predetermined threshold is selected from about 1- 3 mg/dL below a threshold for administration of phototherapy to an infant up to about 12 hours of age per the AAP guidelines, about I mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, is about 2 mg/dL below a threshold for administration of phototherapy to an infant from about 12 to 48 hours of age per the AAP guidelines, about 3 mg dL below a threshold for administration of phototherapy to an infant from about 12 to 48 hours of age per the AAP guidelines, at the threshold for administration of phototherapy to an infant from about 12 to 48 hours of age per the AAP guidelines and about 1 to about 3 mg/dL below the threshold for administration of phototherapy according to AAP nomogram corresponding to the infants age.
17. The method of paragraph 1, wherein administering a therapeutic amount of a metalloporphyrin is performed at a time selected from within about 6 hours of birth, within about 12 hours of birth, within about 24 hours of birth and within about 48 hours of birth.
18. The method of paragraph 1, wherein the metalloporphyrin is selected from tin mesoporphyrin, zinc mesoporphyrin, chromium mesoporphyrin, tin protoporphyrin, zinc protoporphyrin, chromium protoporphyrin, bisglycol protoporphyrin and ferroporphyrin.
19. The method of paragraph 1, wherein the metalloporphyrin is tin mesopo hyrin.
20. The method of paragraph 1, wherein the therapeutic amount of the metalloporphyrin is from about 0.75 mg/kg to about 5 mg kg of the infant's weight.
21. The method of paragraph 1, wherein the therapeutic amount of the metalloporphyrin is selected from 0.75 mg/kg, 1.5 mg/kg, 3.0 mg/kg and 4.5 mg/kg of the infant's weight.
22. The method of paragraph 1, wherein the therapeutic amount of the tin mesoporphyrin is from about 0.75 mg/kg to about 5 mg/kg of the infants weight.
23. The method of paragraph m 1, wherein the therapeutic amount of the tin mesoporphyrin is selected from 0.75 mg/kg, 1.5 mg kg, 3.0 mg/kg and 4.5 mg/kg of the infants weight.
24. The method of paragraph 1, wherein the metalloporphyrin is administered by intramuscular injection.
25. The method of paragraph 1, wherein administering a therapeutic amount of a metalloporphyrin is performed when the infants age is less than 20 days of age.
26. The method of paragraph 1, wherein administering a therapeutic amount of a metalloporphyrin is performed when the infants age is less than 30 days of age.
27. The method of paragraph 1, further comprising administering phototherapy where total bilirubin levels following administration of the metalloporphyrin are above the baseline total bilirubin levels.
28. The method of paragraph 1, further comprising determining post treatment total bilirubin levels following administration of the metalloporphyrin.
29. The method of paragraph 28, wherein determining post treatment total bilirubin levels following administration of the metalloporphyrin is performed from about 6 and to about 72 hours after administering the metalloporphyrin to the infant.
30. The method of paragraph 28, wherein post treatment total bilirubin levels are at least 5% below the baseline total bilirubin levels 24 hours after administering a therapeutic amount of a metalloporphyrin to the infant.
31. The method of paragraph 28, wherein post treatment total bilirubin levels are at least 10% below the baseline total bilirubin levels 48 hours after administering a therapeutic amount of a metalloporphyrin to the infant.
32. The method of paragraph 28, wherein post treatment total bilirubin levels are at least 20% below the baseline total bilirubin levels 72 hours after administering a therapeutic amount of a metallopo hyrin to the infant.
33. The method of paragraph 28, wherein post treatment total bilirubin levels are less than 3mg/dL above the baseline total bilirubin levels 48 hours after administering a therapeutic amount of a metalloporphyrin to the infant.
34. The method of paragraph 1, further comprising conducting on the infant an exam selected from a physical exam, a dermatologic exam, an audiology exam, an ophthalmological exam, a neurological exam, a laboratory test, an electrocardiogram and a combination thereof.
35. A method of reducing the likelihood of hyperbilirubinemia and the symptoms thereof in an infant, the method comprising:
   administering a therapeutic amount of a metalloporphyrin to the infant where the infant's total bilirubin is determined to be increasing in at least one total bilirubin measurement compared with a baseline total bilirubin level
   wherein the likelihood of hyperbilirubinemia or the symptoms thereof is decreased.
36. The method of paragraph 35, wherein the infant's total bilirubin is determined to be increasing in two consecutive total bilirubin measurements.
37. The method of paragraph 35, wherein the baseline total bilirubin measurement is performed from about 6 to about 96 hours of age.
38. The method of paragraph 35, wherein the baseline total bilirubin measurement is performed at about 6, 12, 24, 48, 72, or 96 hours of age.
39. The method of paragraph 35, wherein the at least one total bilirubin measurement is performed from about 6 to about 72 hours after the baseline total bilirubin measurement.
40. The method of paragraph 35, wherein administering a therapeutic amount of a metalloporphyrin is performed within about 1 to about 6 hours of when the infant's total bilirubin is determined to be increasing in at least one total bilirubin measurement.
41. The method of paragraph 35, wherein the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.
42. The method of paragraph 35, wherein the therapeutic amount of the metalloporphyrin is from about 0.75 mg/kg to about 5 mg/kg of the infant's weight.
43. The method of paragraph 35, wherein the therapeutic amount of the metalloporphyrin is selected from 0.75 mg/kg, 1.5 mg/kg, 3.0 mg/kg and 4.5 mg/kg of the infant's weight.
44. The method of paragraph 35, wherein the therapeutic amount of the stannsoporfin is from about 0.75 mg kg to about 5 mg/kg of the infant's weight.
45. The method of paragraph 35, wherein the therapeutic amount of the stannsoporfin is selected from 0.75 mg/kg, 1.5 mg/kg, 3.0 mg/kg and 4.5 mg/kg of the infant's weight.
46. A method of treating hyperbilirubinemia and the symptoms thereof in an infant, the method comprising:
   administering a therapeutic amount of a metalloporphyrin to the infant; and
   administering a therapeutic amount of phototherapy to the infant
   wherein the hyperbilirubinemia or symptoms thereof is treated.
47. The method of paragraph 46, further comprising determining baseline total bilirubin levels.
48. The method of paragraph 47, wherein determining baseline total bilirubin levels is performed within 48 hours of birth.
49. The method of paragraph 46, further comprising identifying the presence of at least one risk factor prior to administering a therapeutic amount of the metalloporphyrin to the infant.
50. The method of paragraph 49, wherein the at least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.
51. The method of paragraph 46, further comprising identifying the presence of at least one exclusion factor prior to administering a therapeutic amount of the metalloporphyrin to the infant.
52. The method of paragraph 51, wherein the at least one exclusion factor is selected from a clinical suggestion of neonatal thyroid disease, current uncontrolled thyroid disease in the mother excluding maternal Hashimoto's, treatment or need for treatment in the infant with medications that may prolong the QT interval excluding eythromycin ointment for eye prophylaxis, a family history of Long QT syndrome, a family history of sudden infant death syndrome, known porphyrias, risk factors for porphyrias, a family history of porphyrias, a maternal history of systemic lupus erythematosus, maternal use of phenobarbital 30 days before, or after delivery, if breastfeeding, maternal current drug or alcohol abuse, maternal history of drug or alcohol abuse, an Apgar score less than or equal to 6 at age 5 minutes, congenital anomalies or infections, acidosis, sepsis, hepatitis; an excess risk of requiring surgery or exposure to operating room lights in the foreseeable future, cardiorespiratory distress defined as a respiratory rate >60 breaths per minute, a diagnosis of transient tachypnea of the newborn, abnormal auditory or ophthalmologic findings, clinically significant abnormalities on a screening laboratory evaluation, elevated direct or conjugated bilirubin (>1.0 mg/dL if the TSB is <5.0 mg/dL or >20% of the TSB if the TSB is >5.0 mg/dL), persistent hypoglycemia (blood glucose <40 mg/dL) despite standard- of-care treatment, liver diseases defined as ALT and/or AST greater than 2 times the upper limit of normal [ULN], abnormal renal function defined as creatinine and/or blood urea nitrogen greater than 2 times the ULN, any blood smear finding of structural red cell abnormalities, such as spherocytosis, not caused by isoimmune hemolysis, temperature instability defined as temperature consistently (3 consecutive times) greater than 36°C and/or greater than 37.5°C axillary, use of photosensitizing drugs or agents; dehydration, defined by hypernatremia, serum sodium greater than ULN, use of intravenous immunoglobulin (IVIG) or albumins, post-delivery treatment with medications that are known or suspected to displace bilirubin from albumin (e.g., ceftriaxone or sulfa-based antibiotics), serious morbid conditions including but not limited to pulmonary disease, cardiovascular disease), exposure to any investigational medications or devices after delivery, participation in a clinical trial and combinations thereof.
53. The method of paragraph 46, wherein administering a therapeutic amount of a metalloporphyrin and administering a therapeutic amount of phototherapy is performed where no exclusion factor is present.
54. The method of paragraph 46, wherein administering a therapeutic amount of a metalloporphyrin and administering a therapeutic amount of phototherapy is performed where at least one of a baseline total bilirubin level elevated above a predetermined threshold and at least one risk factor, or a combination thereof is present.
55. The method of paragraph 54, wherein the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 2 mg dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, and about 1 to about 3 mg/dL below the threshold for administration of phototherapy according to AAP nomogram corresponding to the infants age.
56. The method of paragraph 46, wherein the therapeutic amount of the metalloporphyrin is from about 0.75 mg/kg to about 5 mg/kg of the infant's weight.
57. The method of paragraph 46, wherein the therapeutic amount of the metalloporphyrin is selected from 0.75 mg/kg, 1.5 mg/kg, 3.0 mg/kg and 4.5 mg/kg of the infant's weight.
58. The method of paragraph 46, wherein the therapeutic amount of the stannsoporfin is from about 0.75 mg/kg to about 5 mg/kg of the infant's weight.
59. The method of paragraph 46, wherein the therapeutic amount of the stannsoporfin is selected from 0.75 mg/kg, 1.5 mg kg, 3.0 mg/kg and 4.5 mg/kg of the infant's weight.
60. The method of paragraph 46, wherein administering a therapeutic amount of a metalloporphyrin is performed in the infant is performed when the infants age is less than about 48 hours.
61. The method of paragraph 46, wherein administering a therapeutic amount of a metalloporphyrin is performed in the infant is performed when the infants age is less than about 20 days of age.
62. The method of paragraph 46, wherein administering a therapeutic amount of a metalloporphyrin is performed in the infant is performed when the infants age is less than about 30 days of age.
63. The method of paragraph 46, wherein administering a therapeutic amount of a metalloporphyrin and phototherapy is performed simultaneously.
64. The method of paragraph 46, wherein phototherapy is performed at a time selected from within about 12 hours of administration of therapeutic amount of a metalloporphyrin and within about 24 hours of administration of therapeutic amount of a metalloporphyrin.
65. The method of paragraph 46, further comprising conducting on the infant, a physical exam selected from, a dermatologic exam, an audiology exam, an ophthalmological exam, a neurological exam, a laboratory test, an electrocardiogram and a combination thereof.
66. A method of reducing the risk of hyperbilirubinemia and the symptoms thereof in an infant, the method comprising
   administering a therapeutic amount of a metalloporphyrin to the infant wherein the infant has at least one risk factor associated with hyperbilirubinemia.
67. The method of paragraph 66, wherein the infant has a total bilirubin level of less than about 3 mg/dL below the threshold for administration of phototherapy according to AAP nomogram corresponding to the infants age.
68. The method of paragraph 66, wherein administering a therapeutic amount of a metalloporphyrin to the infant comprises administering a single dose of a metalloporphyrin.
69. The method of paragraph 66, wherein the least one risk factor is selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.
70. The method of paragraph 66, wherein the risk factor is a total bilirubin level at or above a pre-determined threshold.
71. The method of paragraph 70, wherein the predetermined threshold is selected from about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg/dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for aclministration of phototherapy to an infant from about 12 to 48 hours of age per the AAP guidelines.
72. The method of paragraph 66, wherein administering a therapeutic amount of the metalloporphyrin to the infant results in at least one of a decrease in total bilirubin levels compared with total bilirubin levels prior to administering the metalloporphyrin and no detectable increase in total bilirubin levels compared with total bilirubin levels prior to administering the metalloporphyrin.
73. The method of paragraph 66, wherein the therapeutic amount of the metalloporphyrin is from about 0.75 mg/kg to about 5 mg/kg of infant's weight.
74. The method of paragraph 66, wherein the therapeutic amount of the metalloporphyrin is selected from 0.75 mg/kg, 1.5 mg/kg, 3.0 mg/kg and 4.5 mg/kg of infant's weight.
75. The method of paragraph 66, wherein the therapeutic amount of the stannsoporfin is from about 0.75 mg/kg to about 5 mg/kg of infant's weight.
76. The method of paragraph 66, wherein the therapeutic amount of the stannsoporfin is selected from 0.75 mg kg, 1.5 mg kg, 3.0 mg/kg and 4.5 mg kg of infant's weight.
77. A method of stabilizing bilirubin levels in an infant, the method comprising:
   obtaining a baseline total bilirubin level measurement; and
   administering a therapeutic amount of a metalloporphyrin to the infant wherein the infant has at least one of hyperbilirubinemia, bilirubin levels above a pre-determined threshold, rising bilirubin levels, and a combination thereof
   wherein bilirubin levels in the infant are stabilized.
78. The method of paragraph 77, wherein administering a therapeutic amount of a metalloporphyrin to the infant comprises administering a single dose of a metalloporphyrin.
79. The method of paragraph 77, wherein the infant has at least one risk factor selected from hemolytic disease, ABO blood type incompatibility, anti-C Rh incompatibility, anti-c Rh incompatibility, anti-D Rh incompatibility, anti-E Rh incompatibility, anti-e Rh incompatibility, G6PD deficiency and a combination thereof.
80. The method of paragraph 77, wherein the infant is of a gestational age from about 35 to about 43 weeks.
81. The method of paragraph 77, wherein the infant has a minimum birth weight of about 2500 g.
82. The method of paragraph 77, wherein the infant has a birth weight from about 1,700 g to about 4,000 g.
83. The method of paragraph 77, wherein stabilization of total bilirubin levels is achieved when at least two total bilirubin level measurements taken at pre-determined time points after administration of a single therapeutic amount of a metalloporphyrin indicate a total bilirubin level at or below the baseline total bilirubin level.
84. The method of paragraph 77, wherein the predetermined threshold is about 1-3 mg/dL below a threshold for administration of phototherapy to an infant up to about 12 hours of age per the AAP guidelines, about 1 mg/dL below a threshold for administration of phototherapy to an infant up to about 12 hours of age per the AAP guidelines, about 2 mg/dL below a threshold for administration of phototherapy to an infant up to about 12 hours of age per the AAP guidelines, is at the threshold for administration of phototherapy to an infant up to about 12 hours of age per the AAP guidelines, about 1-3mg/dL below a threshold for administration of phototherapy to an infant from about 12 to 48 hours of age per the AAP guidelines, about 1-3mg/dL below a threshold for administration of phototherapy to an infant from about 12 to 48 hours of age per the AAP guidelines, about 2 mg/dL below a threshold for administration of phototherapy to an infant from about 12 to 48 hours of age per the AAP guidelines, about 3 mg dL below a threshold for administration of phototherapy to an infant from about 12 to 48 hours of age per the AAP guidelines, at the threshold for administration of phototherapy to an infant from about 12 to 48 hours of age per the AAP guidelines, about 1 to about 3 mg/dL below the threshold for administration of phototherapy according to AAP nomogram corresponding to the infants age and at the threshold for administration of phototherapy to an infant from about 12 to 48 hours of age per the AAP guidelines.
85. The method of paragraph 77, wherein the therapeutic amount of the metalloporphyrin is from about 0.75 mg/kg to about 5 mg/kg.
86. The method of paragraph 77, wherein the therapeutic amount of the metalloporphyrin is selected from 0.75 mg/kg, 1.5 mg/kg, 3.0 mg/kg and 4.5 mg/kg.
87. The method of paragraph 77, wherein the therapeutic amount of the stannsoporfin is from about 0.75 mg/kg to about 5 mg/kg.
88. The method of paragraph 77, wherein the therapeutic amount of the stannsoporfin is selected from 0.75 mg/kg, 1.5 mg/kg, 3.0 mg/kg and 4.5 mg/kg.
89. A method for treating rising bilirubin levels comprising:
   establishing a baseline bilirubin level in a patient at risk for hyperbilirubinemia at a predetermined age;
   administering to the patient a therapeutic amount of stannsoporfin after the baseline is established.
90. The method of paragraph 89, wherein the predetermined age is about 6 hours, about 12 hours, or about 24 hours from birth.
91. The method of paragraph 89, wherein a baseline reading at the AAP nomogram threshold for administering phototherapy or up to 3.0mg/dL below the AAP nomogram threshold for administering phototherapy indicates treatment is required.
92. A method of treating hyperbilirubinemia comprising:
   administering a therapeutic amount of stannsoporfin to a patient in need thereof to achieve a Cmax of at least 5000ng/mL.
93. The method of paragraph 92, wherein the therapeutic amount of stannsoporfin is 1.5mg/kg and achieves a Cmax of about 6450 ng/mL.
94. The method of paragraph 92, wherein the therapeutic amount of stannsoporfin is 3.0 mg/kg and achieves a Cmax of about 11500 ng/mL.
95. The method of paragraph 92, wherein the therapeutic amount of stannsoporfin is 4.5 mg/kg and achieves a Cmax of about 20400 ng mL.
96. The method of paragraph 92, wherein Cmax is achieved at a Tmax of about 1.5 hours to about 2.5 hours.

## Claims

1. A therapeutic amount of stannsoporfin for use in the treatment of hyperbilirubinemia, **characterized in that** the therapeutic amount of stannsoporfin is administered to a patient in need thereof to achieve a Cmax of at least 5000ng/mL.

2. The therapeutic amount of stannsoporfin for use of claim 1, wherein the therapeutic amount of stannsoporfin is 1.5mg/kg and achieves a Cmax of about 6450 ng/mL.

3. The therapeutic amount of stannsoporfin for use of claim 1, wherein the therapeutic amount of stannsoporfin is 3.0 mg/kg and achieves a Cmax of about 11500 ng/mL.

4. The therapeutic amount of stannsoporfin for use of claim 1, wherein the therapeutic amount of stannsoporfin is 4.5 mg/kg and achieves a Cmax of about 20400 ng mL.

5. The therapeutic amount of stannsoporfin for use of claim 1, wherein Cmax is achieved at a Tmax of about 1.5 hours to about 2.5 hours.

6. The therapeutic amount of stannsoporfin for use of claim 1, wherein the patient is an infant.

7. The therapeutic amount of stannsoporfin for use of claim 6, wherein the infant is of a gestational age from about 35 to about 43 weeks.

8. The therapeutic amount of stannsoporfin for use of claim 6, wherein the infant has a minimum birth weight of about 2,500 g.

9. The therapeutic amount of stannsoporfin for use of claim 6, wherein the infant has a birth weight from about 1,700 g to about 4,000 g.

10. The therapeutic amount of stannsoporfin for use of claim 6, wherein the therapeutic amount of stannsoporfin is administered at a time selected from within about 6 hours of birth, within about 12 hours of birth, within about 24 hours of birth and within about 48 hours of birth.

11. The therapeutic amount of stannsoporfin for use of claim 6, wherein the stannsoporfin is administered by intramuscular injection.

12. The therapeutic amount of stannsoporfin for use of claim 6, wherein the therapeutic amount of stannsoporfin is administered when the infant's age is less than 20 days of age.

13. The therapeutic amount of stannsoporfin for use of claim 6, wherein the therapeutic amount of stannsoporfin is administered when the infant's age is less than 30 days of age.

14. The therapeutic amount of stannsoporfin for use of claim 6, wherein the therapeutic amount of stannsoporfin is from about 0.75 mg kg to about 5 mg/kg of the infant's weight.

15. The therapeutic amount of stannsoporfin for use of claim 1 wherein the patient is and infant, wherein the therapeutic amount of stannsoporfin is from about 0.75 mg kg to about 5 mg/kg of the infant's weight, and wherein the Cmax is achieved at a Tmax of about 1.5 hours to about 2.5 hours.
